# EUROPEAN PATENT SPECIFICATION

(11) **EP 2 661 444 B1**
(45) Date of publication and mention of the grant of the patent: **23.09.2015**
(21) Application number: 11799388.1
(22) Date of filing: 02.12.2011
(51) Int. Cl.: C07K 16/10, C07K 14/005

(54) **ALPHABODIES SPECIFICALLY BINDING TO CLASS-I VIRAL FUSION PROTEINS AND METHODS FOR PRODUCING THE SAME**
SPEZIFISCH AN KLASSE-I-VIRENFUSIONSPROTEINE BINDENDE ALPHAKÖRPER UND VERFAHREN ZU IHRER HERSTELLUNG
ALPHABODIES SE LIANT SPÉCIFIQUEMENT À DES PROTÉINES DE FUSION VIRALES DE CLASSE 1 ET PROCÉDÉS DE PRODUCTION ASSOCIÉS

(30) Priority: 06.01.2011 WO PCT/EP2011/050136
(43) Date of publication of application: 13.11.2013
(73) Proprietor: Complix SA, 1526 Luxembourg (LU)
(72) Inventor: DESMET, Johan, B-8500 Kortrijk (BE); LASTERS, Ignace, B-2018 Antwerpen (BE); MEERSSEMAN, Geert, B-1000 Brussel (BE); DEROO, Sabrina, F-57330 Roussy le Village (FR)
(74) Representative: Paemen, Liesbet R.J.
(86) International application number: PCT/EP2011/071634
(87) International publication number: WO 2012/093013

(56) References cited:
- EP-A1- 2 161 278
- WO-A1-2010/066740
- WO-A1-2011/003936
- M. ROOT ET AL.: "Protein design of an HIV-1 entry inhibitor", SCIENCE, vol. 291, 2 February 2001 (2001-02-02), pages 884-888, XP002170634, Washington, DC, U.S.A. cited in the application

## Description

### FIELD OF THE INVENTION

The present invention relates to the field of binding agents directed against class-I viral fusion proteins and methods for producing such binding agents as well as uses of such binding agents for prophylactic, therapeutic or diagnostic purposes.

### BACKGROUND

One of the essential steps in viral infection is the fusion between the virus membrane and the membrane of the host cell. Viral infection is mediated by viral glycoproteins, including viral attachment proteins and fusion-driving viral fusion proteins. Viral membrane fusion with the host cell can take place either at the plasma membrane or at an intracellular location (endosome) following virus uptake by endocytosis (Earp et al. Curr Topics Microbiol Immunol 2005, 285:25-66).

Antibody therapy using polyclonal and monoclonal antibodies (mAbs) has been effective in prophylaxis of varicella, hepatitis A, hepatitis B, rabies (Montano-Hirose et al. Vaccine 1993, 11: 1259-1266), and respiratory syncytial virus infections (Sawyer, Antiviral Res. 2000, 47: 57-77). In the past decade, two antibodies have been licensed for a viral indication, namely RSV-IG (i.e., RespiGam) and Palivizumab (i.e., Synagis®), both for prevention of respiratory syncytial virus infection. Cytogam^{®} is indicated for the prophylaxis of cytomegalovirus disease associated with transplantation of kidney, lung, liver, pancreas, and heart. Antibody-based therapy for human patients with influenza is up to now little explored. Nevertheless, it has been demonstrated that specific monoclonal antibodies can confer prophylactic and therapeutic protection against influenza in mice (Smirnov et al. Arch. Virol. 2000, 145: 1733-1741). Humanized mouse mAbs and equine F(ab')2 fragments specific for hemagglutinin H5 protein of the influenza virus have also been used for prophylaxis and therapy in a mouse model (Lu et al., Respir. Res. 2006, 7: 43).

Antibody fragments, such as F(ab')2 fragments, Fab fragments (Lamarre et al. J. Immunol. 1995, 154: 3975-3984), single-chain Fv fragments (Mason et al. Virology 1996, 224: 548) and variable domains derived from camelid species heavy chain antibodies (Sherwood et al. J. Infect. Dis. 2007, 196: S213-219; Goldman et al. Anal. Chem. 2006, 78: 8245-8255) have also proven to be successful in neutralizing a variety of enveloped viruses both in vitro and in vivo in animal models (predominantly in mice).

Nevertheless, the development of effective and potent antiviral drugs remains a major scientific challenge. Only for a minority of viral infections, there is at present an effective prophylactic and/or therapeutic compound available. In addition, the antiviral drugs that are currently on the market show numerous side-effects, such as nausea, vomiting, skin rashes, migraine, fatigue, trembling, and, more rarely, epileptic seizures. Also, the constant ability of viruses to mutate and adapt themselves to the environmental conditions, such as challenges by neutralizing antibodies or neutralizing therapeutic compounds, presents an enormous difficulty to the design of antiviral strategies that are effective over the long term.

Accordingly, there remains a serious need for new potent antiviral drugs for the treatment and prevention of infectious viral diseases as well as for alternative and improved antiviral drugs that are more efficient, preferably over the long term, in comparison with the existing antiviral agents that are currently on the market.

WO 2010/066740 and EP 2 161 278 describe Alphabody scaffolds as single-chain triple-stranded alpha-helical coiled coil scaffolds. Both applications describe the architecture and physico-chemical properties of such scaffolds. WO 2010/066740 provides single-chain Alphabody scaffolds which adopt a so-called antiparallel structure, i.e., an architecture wherein one of the three alpha-helices in a single-chain Alphabody is oriented antiparallel with respect to the other two alpha-helices; the three alpha-helices thus constitute an antiparallel coiled coil structure. In contrast, EP 2 161 278 A1 provides single-chain Alphabody scaffolds which adopt an all-parallel structure, i.e. an architecture wherein all three alpha-helices in a single-chain Alphabody together form a parallel coiled coil structure. However, it has not been disclosed how these Alphabody scaffolds can be manipulated to obtain Alphabodies specifically binding to targets of interest.

Several naturally occurring proteins and peptides have been described to form alpha-helical coiled-coils. For instance WO 2005/077103 describes HRN1 of SARS coronavirus S protein consisting of amino acid residues 882-1011 as forming a stable alpha-helical coiled-coil which associates in a tetrameric state. These proteins and peptides however differ significantly from Alphabodies in that they do not comprise three alpha-helices separated by flexible linkers in a single-chain molecule that folds into a coiled coil structure. Indeed, the SARS HRN1 may comprise heptad repeats but does not form a coild coil by itself but as a tetramer (i.e., association of four similar HRN1 peptides). Moreover, in the SARS HR-N sequences, the (predicted) heptad repeats are separated by regions wherein the repeat pattern gets out of register (or phase), which implies that the (also predicted) frameshift/hinge regions can not be considered 'linker' sequences, but rather form part of a single, extended alpha-helix.

Replicas of naturally occurring coiled coils have been made including polypeptides comprising five or all six 6 alpha-helical heptad repeat fragments of gp41 (Root et al. Science 291:886-888), with the aim of developing viral inhibitors. The fact that the described 5-helix construct was derived from exact copies of HIV-1 gp41 heptad repeat sequences (N40 and C38 representing HR1 and HR2, respectively) also necessarily implies that it serves only a single use (i.e., entry inhibition through binding of the C-peptide region of gp41). It cannot bind to other targets and there is no indication on how this could be ensured starting from the five helix structure. Moreover, recombinant synthesis of these polypeptides in E. coli was found to be complicated by the requirement of slow refolding from inclusion bodies using high concentrations of denaturants, reducing their potential as commercial inhibitors and rendering these constructs unsuited as scaffold molecules. Protein molecules of the size of 5-helix (more than 200 amino acid residues) are moreover in general quite intractable for usage as scaffold molecules. In addition, the presence of HR2-derived regions (C38 segments) in the 5-helix is also strictly required for the stability of the construct, because it is well-known that assemblies of gp41 N-peptides have a strong tendency to aggregate (Root et al. *ibid*). Thus, based on these observations, the use of coiled coil molecules as such or as scaffolds for the development of inhibitors of viral fusion proteins would appear to have many practical disadvantages.

### SUMMARY OF THE INVENTION

The present inventors have developed new methods which allow the generation of Alphabodies which specifically bind to a class-I viral fusion protein. It has been found that using the Alphabody scaffold, binders can be generated which bind to such viral fusion protein of interest with high affinity and specificity and which overcome one or more of the disadvantages of the prior art binders. Moreover it has been found that such binders have several advantages over the traditional (immunoglobulin and non-immunoglobulin) binding agents known in the art. Such advantages include, without limitation, the fact that they are compact and small in size (between 10 and 14 kDa, which is 10 times smaller than an antibody), they are extremely (thermo)stable (having a melting temperature of more than 100 °C), and are relatively insensitive to changes in pH and to proteolytic degradation. In addition, Alphabodies are highly soluble, they are highly engineerable (in the sense that multiple substitutions will generally not obliterate their correct and stable folding), and have a structure which is based on natural motifs but is designed via protein engineering techniques.

The Alphabody inhibitors of the present invention do not suffer from the inconveniences of naturally occurring coiled coils: they can be produced from the soluble fraction of E. coli, they have de novo designed sequences with no significant identity to natural proteins, they can be made to bind to a large variety of target molecules of interest, their basic fold is composed of exactly three alpha-helices (i.e., there are no 'C-type' alpha-helices associated with the coiled coil helices), and they are small and highly stable constructs which can be redesigned almost at will. Moreover, the Alphabody molecules of the present invention are stabilized by specific core amino acid residues, located at heptad a- and d-positions, which must be at least 50% isoleucine amino acid residues.

The present inventors have recognized that by using Alphabodies as a scaffold to obtain structural mimics of the fusion driving proteins, the disadvantages of the naturally occurring molecules can be overcome, while retaining the advantage of optimal binding affinity. Moreover, the present inventors have identified methods for obtaining such structural mimics of viral fusion proteins.

In one aspect, the present invention provides single-chain Alphabodies comprising an alpha-helical binding region, which region mediates binding to a first fusion-driving region of a class-I viral fusion protein and which structurally mimics a second fusion-driving region of that class-I viral fusion protein, wherein the first and second fusion-driving regions of the class-I viral fusion protein are regions which interact to drive the fusion between a virus displaying the class-I viral fusion protein and a target cell, wherein said class-I viral fusion protein is not the gp41 subunit of HIV-1 envelope glycoprotein.

In particular embodiments, the fusion-driving regions are chosen from the group consisting of 'heptad repeat 1' ('HR1'), 'N-terminal heptad repeat' ('HRN' or 'HR=N'), 'N-trimer region' ('N-trimer'), 'N-peptide region', 'coiled coil region', 'heptad repeat 2' ('HR2'), 'C-terminal heptad repeat' ('HRC' or 'HR-C') and 'C-peptide region'.

In further particular embodiments, the alpha-helical binding region, comprised in the Alphabodies of the invention, forms a structural mimic of the secondary structure of another fusion-driving region of the class-I viral fusion protein.

In certain particular embodiments, the alpha-helical binding region, comprised in the Alphabodies of the invention, is located at a solvent-oriented surface of one of the Alphabody alpha-helices and the alpha-helical binding region includes at least 9 amino acid residues located at heptad b-, c- and f-positions. In certain embodiments, at least 5 of the 9 amino acid residues that are located at said heptad b-, c- and f-positions are identical to amino acid residues appearing at structurally equivalent positions in the mimicked fusion-driving region of the class-I viral fusion protein.

In certain particular embodiments, the alpha-helical binding region, comprised in the Alphabodies of the invention, is located at the groove formed by or between two adjacent alpha-helices of the Alphabody, and the alpha-helical binding region includes at least 10 amino acid residues that are located at heptad b- and e-positions in one of the two adjacent alpha-helices and at heptad c- and g-positions in the other of the two adjacent alpha-helices. In particular embodiments, at least 5 of those 10 amino acid residues that are located at heptad b- and e-positions in one of the two adjacent alpha-helices and at heptad c- and g-positions in the other of the two adjacent alpha-helices, are identical to amino acid residues appearing at structurally equivalent positions in the mimicked fusion-driving region of the class-I viral fusion protein.

In particular embodiments, the invention provides single-chain Alphabodies comprising an alpha-helical binding region, which region mediates binding to a first fusion-driving region of a class-I viral fusion protein and which structurally mimics a second fusion-driving region of that class-I viral fusion protein, wherein the first and second fusion-driving regions of the class-I viral fusion protein are regions which interact to drive the fusion between a virus displaying the class-I viral fusion protein and a target cell, wherein the class-1 viral fusion protein is from a virus of the family of the Paramyxoviridae. In more particular embodiments, the virus is a virus such as but not limited to HRSV, TRT, PVM, NDV, Nipah, Hendra, Measles, hpiv3, Sendai, SV5 and Mumps.

In more particular embodiments, the class-1 viral fusion protein is a protein selected from the group of SEQ ID NO: 24 to 45. In further particular embodiments, the first fusion-driving region of the class-1 viral fusion protein has a sequence selected from the group of SEQ ID NO: 24 to 34 and the second fusion-driving region of the class-1 viral fusion driving protein has a sequence selected from the group consisting of SEQ ID NO: 35 to 45 (from the same virus). Alternatively, the first fusion-driving region of the class-1 viral fusion protein has a sequence selected from the group of SEQ ID NO: 35 to 45 and the second fusion-driving region of the class-1 viral fusion driving protein has a sequence selected from the group consisting of SEQ ID NO: 24 to 34 (from the same virus). In a further particular embodiment, the class-I viral fusion protein is not HIV gp41.

In further particular embodiments, the invention provides bispecific single-chain Alphabodies comprising two alpha-helical binding regions, wherein one alpha-helical binding region is located at a solvent-oriented surface of one of the Alphabody alpha-helices and includes at least 9 amino acid residues located at heptad b-, c- and f-positions, and wherein the other alpha-helical binding region is located at the groove formed by or between two adjacent alpha-helices of the Alphabody, and the alpha-helical binding region includes at least 10 amino acid residues that are located at heptad b- and e-positions in one of the two adjacent alpha-helices and at heptad c- and g-positions in the other of the two adjacent alpha-helices.

In a further aspect, the present invention provides methods for producing single-chain Alphabodies binding to a class-I viral fusion protein according to the invention, at least comprising the step of grafting amino acid residues that are selected from a membrane fusion-driving region of said class-I viral fusion protein onto an alpha-helical region of a single-chain Alphabody.

In particular embodiments, the methods for producing single-chain Alphabodies binding to a class-I viral fusion protein according to the invention at least comprise the steps of:
a) selecting fusion-driving region of a class-I viral fusion protein, wherein the selected region is chosen from the group consisting of 'heptad repeat 2' ('HR2'), 'C-terminal heptad repeat' ('HRC' or 'HR-C') or 'C-peptide region',
b) identifying in the selected fusion-driving region the amino acid residues interacting with a complementary fusion-driving region,
c) selecting an alpha-helical region located at a solvent-oriented surface of one of the Alphabody alpha-helices, this alpha-helical region forming a structural mimic of the secondary structure of said selected fusion-driving region, and identifying in this alpha-helical region the heptad b-, c- and f-positions,
d) matching the amino acid residues identified in step b) with the heptad b-, c- and f-positions identified in step c),
e) selecting at least 5 amino acid residues identified in step b) and transferring them to heptad b-, c- and f-positions of the alpha-helical region selected in step c) in accordance with the matching operation of step d),
f) producing the Alphabody comprising the amino acid residues that are transferred in step e).
The fusion-driving region of a class-I viral fusion protein selected in step (a) is typically an alpha-helical fusion-driving region, which facilitates selecting and matching of a region of the Alphabody with the fusion-driving region.

In further particular embodiments, the methods for producing single-chain Alphabodies binding to a viral fusion protein according to the invention at least comprise the steps of:
a) selecting a fusion-driving region of a class-I viral fusion protein, said selected region being chosen from the group consisting of 'heptad repeat 1' ('HR1'), 'N-terminal heptad repeat' ('HRN' or 'HR-N'), 'N-trimer region' ('N-trimer'), 'N-peptide region' or 'coiled coil region',
b) identifying in said selected fusion-driving region the amino acid residues interacting with a complementary fusion-driving region,
c) selecting an alpha-helical region located at a groove formed by two adjacent alpha-helices of the Alphabody, this alpha-helical region forming a structural mimic of the secondary structure of said selected fusion-driving region, and identifying in this alpha-helical region the heptad b- and e-positions in one of said adjacent alpha-helices and heptad c- and g-positions in the other of said adjacent alpha-helices,
d) matching the amino acid residues identified in step b) with the heptad b-, c-, e- and g-positions identified in step c),
e) selecting at least 5 amino acid residues identified in step b) and transferring them to heptad b-, c-, e- and g-positions of the alpha-helical region selected in step c) in accordance with the matching operation of step d),
f) producing the Alphabody comprising the amino acid residues that are transferred in step e).

In further particular embodiments, the methods of the invention comprise the production of bispecific single-chain Alphabodies comprising two alpha-helical binding regions, wherein one alpha-helical binding region is located at a solvent-oriented surface of one of the Alphabody alpha-helices and includes at least 9 amino acid residues located at heptad b-, c- and f-positions, and wherein the other alpha-helical binding region is located at the groove formed by or between two adjacent alpha-helices of the Alphabody, and the alpha-helical binding region includes at least 10 amino acid residues that are located at heptad b- and e-positions in one of the two adjacent alpha-helices and at heptad c- and g-positions in the other of the two adjacent alpha-helices. More particularly, in said methods, at least one of said alpha-helical binding regions is generated by the methods described above comprising selecting a fusion-driving region, identifying the amino acid residues therein interacting with a complementary fusion-driving region, selecting an alpha-helical region located at a groove or a solvent-oriented surface of an Alphabody, matching the amino acid residues of the fusion-driving region with amino acid residues in the Alphabody region and transferring these amino-acids to these regions and producing the corresponding Alphabody comprising the transferred amino-acids.

In particular embodiments of the methods of the invention for producing bi-specific antibodies, only one of the alpha-helical binding regions is obtained by the methods described above, and the other alpha-helical binding region is obtained by a random based screening method. More particularly, the methods of the invention may further comprise the identification of a second alpha-helical binding region, which comprises
a) producing a single-chain Alphabody library comprising at least 100 different-sequence single-chain Alphabody polypeptides, wherein said Alphabody polypeptides differ from each other in at least one of a defined set of 5 to 20 variegated amino acid residue positions, and wherein at least 70% of said variegated amino acid residue positions are located either:
   (i) at heptad e-positions in a first alpha-helix of the Alphabody polypeptides and at heptad g-positions in a second alpha-helix, and optionally at heptad b-positions in said first alpha-helix of the Alphabody polypeptides and/or at heptad c-positions in said second alpha-helix of the Alphabody polypeptides, or
   (ii) at heptad b-, c- and f-positions in one alpha-helix of the Alphabody polypeptides, and
b) selecting from said single-chain Alphabody library at least one single-chain Alphabody having detectable binding affinity for, or detectable in vitro activity on, said viral protein of interest.
Typically, an alpha-helical binding region obtained by random variegation of amino acid residues in specified positions of a solvent-oriented alpha-helix is combined with an alphahelical binding region in a groove formed by or between two adjacent alpha-helices of the Alphabody obtained by rational design as described herein above, or vice versa. In further particular embodiments, a first binding site is obtained by random variegation of amino acid residues in specified positions and screening for binding to a first epitope, and the resulting Alphabody is further modified to introduce a second alpha-helical binding region by specific transfer of amino acids corresponding to the amino acids of a complementary fusion driving region.

The methods of the invention can be used for the production of single-chain Alphabodies capable of inhibiting the fusion of class-I viral fusion proteins. Thus a further aspect of the invention relates to single-chain Alphabodies obtainable by the methods of the invention.

Yet a further aspect of the invention relates to the use of the Alphabodies described herein as medicaments and more particularly for the treatment of a viral infection.

Yet a further aspect of the invention relates to methods for the treatment of a patient suffering from a disease caused by a virus characterized in that it has a class I viral fusion protein, which method comprises administering to said patient, a therapeutic dosage of the Alphabody according to the invention.

### DETAILED DESCRIPTION OF THE INVENTION

As used herein, the singular forms 'a', 'an', and 'the' include both singular and plural referents unless the context clearly dictates otherwise.

The terms 'comprising', 'comprises' and 'comprised of as used herein are synonymous with 'including', 'includes' or 'containing', 'contains', and are inclusive or open-ended and do not exclude additional, non-recited members, elements or method steps.

The recitation of numerical ranges by endpoints includes all numbers and fractions subsumed within the respective ranges, as well as the recited endpoints.

The term 'about' as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier 'about' refers is itself also specifically, and preferably, disclosed.

As used herein, an 'Alphabody (of the invention)' or 'Alphabodies (of the invention)' can generally be defined as self-folded, single-chain, triple-stranded, predominantly alpha-helical, coiled coil amino acid sequences, polypeptides or proteins. The term 'single-chain' in 'single-chain Alphabody' is therefore redundant, but usually included to emphasize the composition of an Alphabody as a single polypeptide chain, as opposed to the many known occurrences of oligomeric (e.g., trimeric) peptidic coiled coils. More particularly, Alphabodies as used in the context of the present invention can be defined as amino acid sequences, polypeptides or proteins having the general formula HRS1-L1-HRS2-L2-HRS3, wherein
- each of HRS1, HRS2 and HRS3 is independently a heptad repeat sequence (HRS) consisting of 2 to 7 consecutive heptad repeat units, at least 50% of all heptad a- and d-positions are occupied by isoleucine residues, each HRS starts and ends with an aliphatic or aromatic amino acid residue located at either a heptad a- or d-position, and HRS1, HRS2 and HRS3 together form a triple-stranded, alpha-helical, coiled coil structure; and
- each of L1 and L2 are independently a linker fragment, covalently connecting HRS1 to HRS2 and HRS2 to HRS3, respectively, and consisting of at least 4 amino acid residues, preferably at least 50% of which are selected from the group proline, glycine, serine.

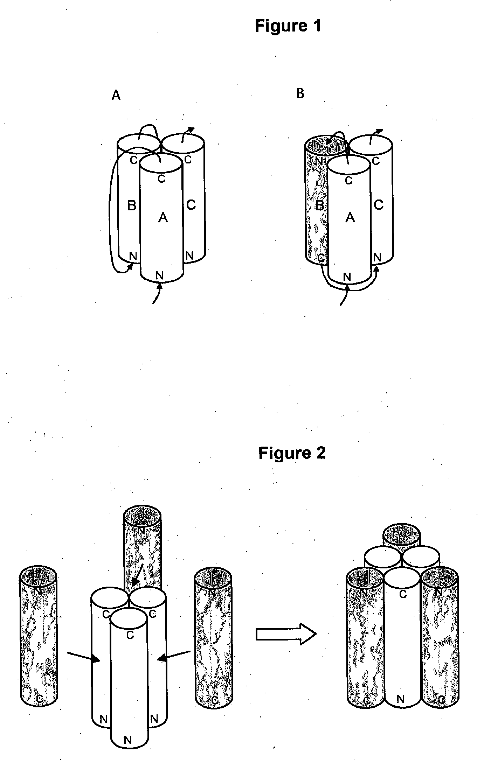

As used herein, a 'parallel Alphabody' shall have the meaning of an Alphabody (of the invention) wherein the alpha-helices of the triple-stranded, alpha-helical, coiled coil structure together form a parallel coiled coil structure, i.e., a coiled coil wherein all three alpha-helices are parallel (see also Figure 1A).

As used herein, an 'antiparallel Alphabody' shall have the meaning of an Alphabody (of the invention) wherein the alpha-helices of the triple-stranded, alpha-helical, coiled coil structure together form an antiparallel coiled coil structure, i.e., a coiled coil wherein two alpha-helices are parallel and the third alpha-helix is antiparallel with respect to these two helices (see also Figure 1 B).

As will become clear from the further description herein Alphabodies having the general formula HRS1-L1-HRS2-L2-HRS3 may in certain particular embodiments be covalently linked to further groups, moieties and/or residues, more particularly N- and/or C-terminal covalently linked. The present invention thus generally relates to Alphabody polypeptides comprising one or more Alphabodies according to the invention and/or other groups, moieties and/or residues linked thereto.

The terms 'heptad', 'heptad unit' or 'heptad repeat unit' are used interchangeably herein and shall herein have the meaning of a 7-residue (poly)peptide fragment that is repeated two or more times within each heptad repeat sequence of an Alphabody, polypeptide or composition of the invention and is represented as 'abcdefg' or 'defgabc', wherein the symbols 'a' to 'g' denote conventional heptad positions. Conventional heptad positions are assigned to specific amino acid residues within a heptad, a heptad unit, or a heptad repeat unit, present in an Alphabody, polypeptide or composition of the invention, for example, by using specialized software such as the COILS method of Lupas et al. (Lupas et al., Science 252:1162-1164 (1994)); http://www.russell.emblheidelberg.de/cgi-bin/coils-svr.pl). However, it is noted that the heptads or heptad units as present in the Alphabodies of the invention (or polypeptides and compositions of the invention comprising these Alphabodies) are not strictly limited to the above-cited representations (i.e., 'abcdefg' or 'defgabc') as will become clear from the further description herein and in their broadest sense constitute a 7-residue (poly)peptide fragment per se, comprising at least assignable heptad positions a and d.

The terms 'heptad a-positions', 'heptad b-positions', 'heptad c-positions', 'heptad d-positions', 'heptad e-positions', 'heptad f-positions' and 'heptad g-positions' refer respectively to the conventional 'a', 'b', 'c', 'd', 'e', 'f and 'g' amino acid positions in a heptad, heptad repeat or heptad repeat unit of an Alphabody, polypeptide or composition of the invention.

A 'heptad motif as used herein shall have the meaning of a 7-residue (poly)peptide pattern. A 'heptad motif' of the type 'abcdefg' can usually be represented as 'HPPHPPP', whereas a 'heptad motif of the type 'defgabc' can usually represented as 'HPPPHPP', wherein the symbol 'H' denotes an apolar or hydrophobic amino acid residue and the symbol 'P' denotes a polar or hydrophilic amino acid residue. However, it is noted that the heptad motifs as present in the Alphabodies of the invention (or polypeptides and compositions of the invention comprising these Alphabodies) are not strictly limited to the above-cited representations (i.e., 'abcdefg', 'HPPHPPP', 'defgabc' and 'HPPPHPP') as will become clear from the further description herein.

A 'heptad repeat sequence' ('HRS') as used herein shall have the meaning of an amino acid sequence or sequence fragment consisting of n consecutive heptads, where n is a number equal to or greater than 2.

In the context of the single-chain structure of the Alphabodies (as defined herein) the terms 'linker', 'linker fragment' or 'linker sequence' are used interchangeably herein and refer to an amino acid sequence fragment that is part of the contiguous amino acid sequence of a single-chain (monomeric) Alphabody, and covalently interconnects the HRS sequences of that Alphabody.

In the context of the present invention, a 'coiled coil' or 'coiled coil structure' shall be used interchangeably herein and will be clear to the person skilled in the art based on the common general knowledge and the description and further references cited herein. Particular reference in this regard is made to review papers concerning coiled coil structures (such as for example, Cohen and Parry, Proteins 7:1-15 (1990); Kohn and Hodges, Trends Biotechnol. 16:379-389 (1998); Schneider et al., Fold. Des. 8, 3:R29-R40 (1998); Harbury et al., Science 282:1462-1467 (1998); Mason and Arndt, Chem. BioChem. 5:170-176 (2004); Lupas and Gruber, Adv. Protein Chem. 70:37-78 (2005); Woolfson, Adv. Protein Chem. 70:79-112 (2005); Parry et al., J. Struct. Biol. 163:258-269 (2008); McFarlane et al., Eur. J. Pharmacol. 625:101-107 (2009)).

An 'alpha-helical part of an Alphabody' shall herein have the meaning of that part of an Alphabody which has an alpha-helical secondary structure. Furthermore, any part of the full part of an Alphabody having an alpha-helical secondary structure is also considered an alpha-helical part of an Alphabody. More particularly, in the context of a binding site, where one or more amino acids located in an alpha-helical part of the Alphabody contribute to the binding site, the binding site is considered to be formed by an alpha-helical part of the Alphabody.

A 'solvent-oriented' or 'solvent-exposed' region of an alpha-helix of an Alphabody shall herein have the meaning of that part on an Alphabody which is directly exposed or which comes directly into contact with the solvent, environment, surroundings or milieu in which it is present. Furthermore, any part of the full part of an Alphabody which is directly exposed or which comes directly into contact with the solvent is also considered a solvent-oriented or solvent-exposed region of an Alphabody. More particularly, in the context of a binding site, where one or more amino acids located in a solvent-oriented part of the Alphabody contribute to the binding site, the binding site is considered to be formed by a solvent-oriented part of the Alphabody.

The term 'groove of an Alphabody' shall herein have the meaning of that part on an Alphabody which corresponds to the concave, groove-like local shape, which is formed by any pair of spatially adjacent alpha-helices within an Alphabody.

As used herein, amino acid residues will be indicated either by their full name or according to the standard three-letter or one-letter amino acid code.

As used herein, the term 'homology' denotes at least primary structure similarity between two macromolecules, particularly between two polypeptides or polynucleotides, from same or different taxons, wherein said similarity is due to shared ancestry. Preferably, homologous polypeptides will also display similarity in secondary or tertiary structure. Hence, the term 'homologues' denotes so-related macromolecules having said primary and optionally, for proteinaceous macromolecules, secondary or tertiary structure similarity. For comparing two or more nucleotide sequences, the '(percentage of) sequence identity' between a first nucleotide sequence and a second nucleotide sequence may be calculated using methods known by the person skilled in the art, e.g. by dividing the number of nucleotides in the first nucleotide sequence that are identical to the nucleotides at the corresponding positions in the second nucleotide sequence by the total number of nucleotides in the first nucleotide sequence and multiplying by 100% or by using a known computer algorithm for sequence alignment such as NCBI Blast. In determining the degree of sequence identity between two Alphabodies, the skilled person may take into account so-called 'conservative' amino acid substitutions, which can generally be described as amino acid substitutions in which an amino acid residue is replaced with another amino acid residue of similar chemical structure and which has little or essentially no influence on the function, activity or other biological properties of the polypeptide. Possible conservative amino acid substitutions will be clear to the person skilled in the art. Two or more Alphabodies, or two or more nucleic acid sequences are said to be 'exactly the same' if they have 100% sequence identity over their entire length.

An Alphabody, polypeptide or composition of the invention is said to 'specifically bind to' a particular target when that Alphabody, polypeptide or composition of the invention has affinity for, specificity for and/or is specifically directed against that target (or against at least one part or fragment thereof).

The 'specificity' of an Alphabody, polypeptide or composition of the invention as used herein can be determined based on affinity and/or avidity. The 'affinity' of an Alphabody, polypeptide or composition of the invention is represented by the equilibrium constant for the dissociation of the Alphabody, polypeptide or composition and the target protein of interest to which it binds. The lower the KD value, the stronger the binding strength between the Alphabody, polypeptide or composition and the target protein of interest to which it binds. Alternatively, the affinity can also be expressed in terms of the affinity constant (KA), which corresponds to 1/KD. The binding affinity of an Alphabody, polypeptide or composition of the invention can be determined in a manner known to the skilled person, depending on the specific target protein of interest.

It is generally known in the art that the KD can be expressed as the ratio of the dissociation rate constant of a complex, denoted as kOff (expressed in seconds⁻¹ or s⁻¹), to the rate constant of its association, denoted kOn (expressed in molar⁻¹ seconds⁻¹ or M⁻¹ s⁻¹). A KD value greater than about 1 millimolar is generally considered to indicate non-binding or non-specific binding.

The 'avidity' of an Alphabody, polypeptide or composition of the invention is the measure of the strength of binding between an Alphabody, polypeptide or composition of the invention and the pertinent target protein of interest. Avidity is related to both the affinity between a binding site on the target protein of interest and a binding site on the Alphabody, polypeptide or composition of the invention and the number of pertinent binding sites present on the Alphabody, polypeptide or composition of the invention. Binding affinities, kOff and kOn rates may be determined by means of methods known to the person skilled in the art. These methods include, but are not limited to RIA (radioimmunoassays), ELISA (enzyme-linked immuno-sorbent assays), 'sandwich' immunoassays, immunoradiometric assays, gel diffusion precipitation reactions, immunodiffusion assays, Western blots, precipitation reactions, agglutination assays (e.g., gel agglutination assays, hemagglutination assays, etc.), complement fixation assays, immunofluorescence assays, immunoelectrophoresis assays, isothermal titration calorimetry, surface plasmon resonance, fluorescence-activated cell sorting analysis, etc.

An Alphabody, polypeptide or composition of the invention is said to be 'specific for a first target protein of interest as opposed to a second target protein of interest' when it binds to the first target protein of interest with an affinity that is at least 5 times, such as at least 10 times, such as at least 100 times, and preferably at least 1000 times higher than the affinity with which that Alphabody, polypeptide or composition of the invention binds to the second target protein of interest. Accordingly, in certain embodiments, when an Alphabody, polypeptide or composition is said to be 'specific for' a first target protein of interest as opposed to a second target protein of interest, it may specifically bind to (as defined herein) the first target protein of interest, but not to the second target protein of interest. The same applies when reference is made to specificity for specific protein domains or subregions thereof.

An Alphabody, polypeptide or composition of the invention is said to 'have detectable binding affinity for' a protein of interest, when it binds to that protein of interest (more particularly to a domain or subregion thereof) with an affinity higher than the detection limit of any of the methods known to the person skilled in the art, i.e., methods including but not limited to RIA (radioimmunoassays), ELISA (enzyme-linked immuno-sorbent assays), 'sandwich' immunoassays, immunoradiometric assays, gel diffusion precipitation reactions, immunodiffusion assays, Western blots, precipitation reactions, agglutination assays (e.g., gel agglutination assays, hemagglutination assays, etc.), complement fixation assays, immunofluorescence assays, immunoelectrophoresis assays, isothermal titration calorimetry, surface plasmon resonance, fluorescence-activated cell sorting analysis, etc.

The 'half-life' of an Alphabody, polypeptide or compound of the invention can generally be defined as the time that is needed for the in vivo serum or plasma concentration of the Alphabody, polypeptide or compound to be reduced by 50%. The in vivo half-life of an Alphabody, compound or polypeptide of the invention can be determined in any manner known to the person skilled in the art, such as by pharmacokinetic analysis. As will be clear to the skilled person, the half-life can be expressed using parameters such as the t1/2-alpha, t1/2-beta and the area under the curve (AUC). An increased half-life in vivo is generally characterized by an increase in one or more and preferably in all three of the parameters t1/2-alpha, t1/2-beta and the area under the curve (AUC).

As used herein, the terms 'inhibiting', 'reducing' and/or 'preventing' may refer to (the use of) an Alphabody, polypeptide or composition according to the invention that specifically binds to a target protein of interest (in particular to a target protein domain or subregion thereof), and inhibits, reduces and/or prevents the interaction between that target protein and its natural binding partner or between two different subregions, subdomains, domains or parts of that target protein. The terms 'inhibiting', 'reducing' and/or 'preventing' may also refer to (the use of) an Alphabody, polypeptide or composition according to the invention that specifically binds to a target protein and inhibits, reduces and/or prevents a biological activity of that target protein of interest, as measured using a suitable in vitro, cellular or in vivo assay. Accordingly, 'inhibiting', 'reducing' and/or 'preventing' may also refer to (the use of) an Alphabody, polypeptide or composition according to the invention that specifically binds to a target protein of interest and inhibits, reduces and/or prevents one or more biological or physiological mechanisms, effects, responses, functions pathways or activities in which the target protein of interest is involved. Such an action of the Alphabody, polypeptide or composition according to the invention as an antagonist, in the broadest possible sense, may be determined in any suitable manner and/or using any suitable (in vitro and usually cellular or in vivo) assay known in the art, depending on the type of inhibition, reduction and/or prevention of the said one or more biological or physiological mechanisms, effects, responses, functional pathways or activities in which the said target protein is involved. Non-limiting examples of such types of functional effects include (i) the (possibly indirect) prevention of attachment to cellular receptors on specific, dedicated target cells, (ii) the (possibly indirect) prevention of interaction with the glycocalyx of target cells, through blockage of conformational changes that are required for membrane fusion, (viii) the arrest of a viral fusion protein in a conformational or mechanistic state that is intermediate to the native and postfusion states, (viiv) the irreversible functional deactivation of a viral fusion protein prior to attachment to a target cell, and wherein said deactivation is further characterized by the inability of said viral fusion protein to recover membrane fusion activity even after removal of the antiviral Alphabody, polypeptide or composition according to the invention.

The said 'inhibiting', 'reducing' and/or 'preventing' activity of an Alphabody, polypeptide or composition of the invention may be reversible or irreversible.

An Alphabody, polypeptide, composition or nucleic acid sequence of the invention is herein considered to be '(in) essentially isolated (form)' when it has been extracted or purified from the host cell and/or medium in which it was produced.

In respect of the Alphabodies, polypeptides and (pharmaceutical) compositions, the terms 'binding region', 'binding site' or 'interaction site' present on the Alphabodies, polypeptides or pharmaceutical compositions shall herein have the meaning of a particular site, part, domain or stretch of amino acid residues present on the Alphabodies, polypeptides or pharmaceutical compositions that is responsible for binding to a target molecule. Such binding region essentially consists of specific amino acid residues from the Alphabody which are in contact with the target molecule, in particular, viral fusion protein.

An Alphabody, polypeptide or composition of the invention is said to show 'cross-reactivity' for two different target proteins of interest if it is specific for (as defined herein) both of these different target proteins of interest.

The term 'monovalent' as used herein, refers to the fact that the Alphabody contains one binding site directed against or specifically binding to a site, determinant, part, domain or stretch of amino acid residues of the target of interest.

In cases where two or more binding sites of an Alphabody are directed against or specifically bind to the same site, determinant, part, domain or stretch of amino acid residues of the target of interest, the Alphabody is said to be 'bivalent' (in the case of two binding sites on the Alphabody) or multivalent (in the case of more than two binding sites on the Alphabody), such as for example trivalent.

The term 'bi-specific' when referring to an Alphabody implies that either a) two or more of the binding sites of an Alphabody are directed against or specifically bind to the same target of interest but not to the same (i.e., to a different) site, determinant, part, domain or stretch of amino acid residues of that target, or b) two or more binding sites of an Alphabody are directed against or specifically bind to different target molecules of interest. The term 'multispecific' is used in the case that more than two binding sites are present on the Alphabody.

Accordingly, a 'bispecific Alphabody' or a 'multi-specific Alphabody' as used herein, shall have the meaning of a single-chain Alphabody of the formula (N-)HRS1-L1-HRS2-L2-HRS3(-C) comprising respectively two or at least two binding sites, wherein these two or more binding sites have a different binding specificity. Thus, an Alphabody is herein considered 'bispecific' or 'multispecific' if respectively two or more than two different binding regions exist in the same, monomeric, single-domain Alphabody.

As used herein, the term 'prevention and/or treatment' comprises preventing and/or treating a certain disease and/or disorder, preventing the onset of a certain disease and/or disorder, slowing down or reversing the progress of a certain disease and/or disorder, preventing or slowing down the onset of one or more symptoms associated with a certain disease and/or disorder, reducing and/or alleviating one or more symptoms associated with a certain disease and/or disorder, reducing the severity and/or the duration of a certain disease and/or disorder, and generally any prophylactic or therapeutic effect of the Alphabodies or polypeptides of the invention that is beneficial to the subject or patient being treated.

A 'class-I viral fusion protein' shall herein have the meaning of a fusion glycoprotein from an enveloped virus belonging to the families of Orthomyxoviridae, Paramyxoviridae, Retroviridae, Filoviridae or Coronaviridae. For the sake of clarity, the notion 'fusion protein' relates to a viral glycoprotein that aids in driving the fusion process between the membranes of a virus and a target cell. A fusion protein is herein also denoted as an 'F-protein'.

A 'fusion-driving region' shall herein have the meaning of any region from a class-I viral fusion protein contributing to the formation and thermodynamic stability of the postfusion state of that class-I viral fusion protein.

A 'target cell' shall herein have the meaning of any cell that is susceptible to fusion with a virus or susceptible to entry or infection by a virus. Alternatively, a target cell can also be any cell that is susceptible to fusion with another cell which displays class-I viral F-proteins at its surface.

A 'binding region' on or comprised in an Alphabody of the invention shall herein have the meaning of that area on an Alphabody that is responsible for binding to a target molecule, i.e. a class-I viral fusion protein. Such binding region essentially consists of specific residues from the Alphabody which are in contact or interact with (certain regions or areas of) the target molecule.

The term 'structural mimic' shall herein have the meaning of 'structurally similar'. In the context of the present invention the term 'structural mimic' does not include a replica or a slightly modified replica. More particularly, the Alphabodies of the present invention comprise less than 20% sequence homology with the naturally occurring viral fusion protein region they mimic. In particular embodiments, the correspondence with the natural molecule is less than 50%.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The present inventors have identified methods of producing specific binders to class-I viral fusion proteins, making use of the structural similarity between (particular subregions within) these fusion proteins and (subregions within) Alphabodies. Accordingly, the inventors have identified methods of making Alphabodies which specifically bind to membrane fusion-driving regions within class-I viral fusion proteins (also referred to herein as 'Alphabodies of the invention').

In addition, it has been found that these Alphabodies of the invention can bind to class-I viral fusion proteins with affinities at least comparable to the affinities of traditional binding agents directed against the same class-I viral fusion proteins. Moreover, target-binding Alphabodies maintain the advantages identified for Alphabody scaffolds, such as the Alphabody scaffolds provided in WO 2010/066740 and EP 2 161 278. Alphabodies not only have a unique structure but also have several advantages over the traditional (immunoglobulin and non-immunoglobulin) scaffolds known in the art. These advantages include, but are not limited to, the fact that they are compact and small in size (between 10 and 14 kDa, which is 10 times smaller than an antibody), they are extremely thermostable (i.e., they generally have a melting temperature of more than 100 °C), they can be made resistant to different proteases, they are highly engineerable (in the sense that multiple substitutions will generally not obliterate their correct and stable folding), and have a structure which is based on natural motifs which have been redesigned via protein engineering techniques.

The Alphabodies according to the present invention that bind to class-I viral fusion proteins are amino acid sequences, polypeptides or proteins having the general formula HRS1-L1-HRS2-L2-HRS3. Such Alphabodies are optionally covalently linked to additional N- and C-terminal groups, residues or moieties resulting in the formula N-HRS1-L1-HRS2-L2-HRS3-C. The optional N and C extensions can be, for example, a tag for detection or purification (e.g., a His-tag) or another protein or protein domain, in which case the full construct is denoted a fusion protein (yet not to be confused with a viral fusion protein as defined herein). For the sake of clarity, the optional extensions N and C are herein considered not to form part of a single-chain Alphabody structure, which is defined by the general formula 'HRS1-L1-HRS2-L2-HRS3'. General reference is made herein to Alphabody polypeptides which can consist of an Alphabody or comprise one or more Alphabodies, optionally having groups, residues or moieties linked thereto.

As indicated above, a heptad repeat of an Alphabody is generally represented as 'abcdefg' or 'defgabc', wherein the symbols 'a' to 'g' denote conventional heptad positions. The 'a-positions' and 'd-positions' in each heptad unit of an Alphabody of the invention are amino acid residue positions of the coiled coil structure where the solvent-shielded (i.e., buried) core residues are located. The 'e-positions' and 'g-positions' in each heptad unit of an Alphabody of the invention are amino acid residue positions of the coiled coil structure where the amino acid residues which are partially solvent-exposed are located. In a triple-stranded coiled coil, these 'e-positions' and 'g-positions' are located in the groove formed between two spatially adjacent alpha-helices, and the corresponding amino acid residues are commonly denoted the 'groove residues'. The 'b-positions', 'c-positions' and 'f-positions' in each heptad unit of an Alphabody of the invention are the most solvent-exposed positions in a coiled coil structure.

It is noted that in the prior art, a heptad may be referred to as 'heptad repeat' because the 7-residue fragment is usually repeated a number of times in a true coiled coil amino acid sequence.

A heptad motif (as defined herein) of the type 'abcdefg' is typically represented as 'HPPHPPP', whereas a 'heptad motif of the type 'defgabc' is typically represented as 'HPPPHPP', wherein the symbol 'H' denotes an apolar or hydrophobic amino acid residue and the symbol 'P' denotes a polar or hydrophilic amino acid residue. Typical hydrophobic residues located at a- or d-positions include aliphatic (e.g., leucine, isoleucine, valine, methionine) or aromatic (e.g., phenylalanine) amino acid residues. Heptads within coiled coil sequences do not always comply with the ideal pattern of hydrophobic and polar residues, as polar residues are occasionally located at 'H' positions and hydrophobic residues at 'P' positions. Thus, the patterns 'HPPHPPP' and 'HPPPHPP' are to be considered as ideal patterns or characteristic reference motifs. Occasionally, the characteristic heptad motif is represented as 'HPPHCPC' or 'HxxHCxC' wherein 'H' and 'P' have the same meaning as above, 'C' denotes a charged residue (lysine, arginine, glutamic acid or aspartic acid) and 'x' denotes any (unspecified) natural amino acid residue. Since a heptad can equally well start at a d-position, the latter motifs can also be written as 'HCPCHPP' or 'HCxCHxx'. It is noted that single-chain Alphabodies are intrinsically so stable that they do not require the aid of ionic interactions between charged ('C') residues at heptad e- and g-positions.

A heptad repeat sequence (HRS) (as defined herein) is typically represented by (abcdefg)ₙ or (defgabc)ₙ in notations referring to conventional heptad positions, or by (HPPHPPP)ₙ or (HPPPHPP)ₙ in notations referring to the heptad motifs, with the proviso that not all amino acid residues in a HRS should strictly follow the ideal pattern of hydrophobic and polar residues. In order to identify heptad repeat sequences, and/or their boundaries, including heptad repeat sequences comprising amino acids or amino acid sequences that deviate from the consensus motif, and if only amino acid sequence information is at hand, then the COILS method of Lupas et al. (Science 1991, 252:1162-1164) is a suitable method for the determination or prediction of heptad repeat sequences and their boundaries, as well as for the assignment of heptad positions. Furthermore, the heptad repeat sequences can be resolved based on knowledge at a higher level than the primary structure (i.e., the amino acid sequence). Indeed, heptad repeat sequences can be identified and delineated on the basis of secondary structural information (i.e., alpha-helicity) or on the basis of tertiary structural (i.e., protein folding) information. A typical characteristic of a putative HRS is an alpha-helical structure. Another (strong) criterion is the implication of a sequence or fragment in a coiled coil structure. Any sequence or fragment that is known to form a regular coiled coil structure, i.e., without stutters or stammers as described in Brown et al. Proteins 1996, 26:134-145), is herein considered a HRS. Also and more particularly, the identification of HRS fragments can be based on high-resolution 3-D structural information (X-ray or NMR structures). Finally, but not limited hereto, and unless clear evidence of the contrary exists, or unless otherwise mentioned, the boundaries to any HRS fragment may be defined as the first (respectively last) a- or d-position at which a standard hydrophobic amino acid residue (selected from the group valine, isoleucine, leucine, methionine, phenylalanine, tyrosine or tryptophan) is located.

The linkers within a single-chain structure of the Alphabodies (as defined herein) interconnect the HRS sequences, and more particularly the first to the second HRS, and the second to the third HRS in an Alphabody. Connections between HRS fragments via disulfide bridges or chemical cross-linking or, in general, through any means of inter-chain linkage, are explicitly excluded from the definition of a linker fragment (at least, in the context of an Alphabody) because such would be in contradiction with the definition of a single-chain Alphabody. A linker fragment in an Alphabody is preferably flexible in conformation to ensure relaxed (unhindered) association of the three heptad repeat sequences as an alpha-helical coiled coil structure. Further in the context of an Alphabody, 'L1' shall denote the linker fragment one, i.e., the linker between HRS1 and HRS2, whereas 'L2' shall denote the linker fragment two, i.e., the linker between HRS2 and HRS3.

The 'coiled coil' structure of an Alphabody can be considered as being an assembly of alpha-helical heptad repeat sequences wherein the alpha-helical heptad repeat sequences are as defined supra; furthermore,
- the said alpha-helical heptad repeat sequences are wound (wrapped around each other) with a left-handed supertwist (supercoiling);
- the core residues at a- and d-positions form the core of the assembly, wherein they pack against each other in a knobs-into-holes manner as defined in the Socket algorithm (Walshaw and Woolfson, J. Mol. Biol. 2001, 307:1427-1450 and reiterated in Lupas and Gruber, Adv. Protein Chem. 2005, 70:37-78);
- the core residues are packed in regular core packing layers, where the layers are defined as in Schneider et al. (Schneider et al., Fold. Des. 1998, 3:R29-R40).

The coiled coil structure of the Alphabodies of the present invention is not to be confused with ordinary three-helix bundles. Criteria to distinguish between a true coiled coil and non-coiled coil helical bundles are provided in Desmet et al. WO 2010/066740 A1 and Schneider et al. (Schneider et al., Fold. Des. 1998, 3:R29-R40); such criteria essentially relate to the presence or absence of structural symmetry in the packing of core residues for coiled coils and helix bundles, respectively. Also the presence or absence of left-handed supercoiling for coiled coils and helix bundles, respectively, provides a useful criterion to distinguish between both types of folding.

While aforegoing criteria in principle apply to 2-stranded, 3-stranded, 4-stranded and even more-stranded coiled coils, the Alphabodies of the present invention are restricted to 3-stranded coiled coils. The coiled coil region in an Alphabody can be organized with all alpha-helices in parallel orientation (corresponding to a 'parallel Alphabody' as described in EP2161278 by Applicant Complix NV) or with one of the three alpha-helices being antiparallel to the two other (corresponding to an 'antiparallel Alphabody' as described in WO 2010/066740 by Applicant Complix NV).

The alpha-helical part of an Alphabody (as defined herein) will usually grossly coincide with the heptad repeat sequences although differences can exist near the boundaries. For example, a sequence fragment with a clear heptad motif can be non-helical due to the presence of one or more helix-distorting residues (e.g., glycine or proline). Reversely, part of a linker fragment can be alpha-helical despite the fact that it is located outside a heptad repeat region. Further, any part of one or more alpha-helical heptad repeat sequences is also considered an alpha-helical part of a single-chain Alphabody.

The solvent-oriented region of (the alpha-helices of) an Alphabody (as defined herein) is an important Alphabody region. In view of the configuration of the alpha-helices in an Alphabody, wherein the residues at heptad a- and d-positions form the core, the solvent-oriented region is largely formed by b-, c- and f-residues. There are three such regions per single-chain Alphabody, i.e., one in each alpha-helix. Any part of such solvent-oriented region is also considered a solvent-oriented region. For example, a subregion composed of the b-, c- and f-residues from three consecutive heptads in an Alphabody alpha-helix will also form a solvent-oriented surface region.

Residues implicated in the formation of (the surface of) a groove between two adjacent alpha-helices in an Alphabody are generally located at heptad e- and g-positions, but some of the more exposed b- and c-positions as well as some of the largely buried core a- and d-positions may also contribute a the binding site located in a groove surface; such will essentially depend on the size of the amino acid side chains placed at these positions. Where the groove is formed by spatially adjacent alpha-helices running parallel, then the groove is formed by b- and e-residues from a first helix and by c- and g-residues of a second helix. If the said spatially adjacent alpha-helices are antiparallel, then there exist two possibilities. In a first possibility, both halves of the groove are formed by b- and e-residues (i.e., by b- and e- residues from both the first and second helix). In the second possibility, both halves of the groove are formed by c- and g-residues (i.e., by c- and g-residues from the first and second helix). The three types of possible grooves are herein denoted by their primary groove-forming (e- and g-) residues: if the helices are parallel, then the groove is referred to as an 'e/g-groove'; if the helices are antiparallel, then the groove is referred to as either an 'e/e-groove' or a 'g/g-groove'. Parallel Alphabodies (i.e., wherein all three helixes run in parallel) have three e/g-grooves, whereas antiparallel Alphabodies (i.e., comprising one antiparallel and two parallel helixes) have one e/g-groove, one e/e-groove and one g/g-groove. Any part of an Alphabody groove is also referred to herein as a groove region.

As a main object, the present invention provides methods for providing Alphabodies that specifically bind to a class-I viral fusion protein of interest and Alphabodies having detectable binding affinity for, or detectable in vitro activity on, a class-I viral fusion protein of interest, which are obtainable by the methods according to the invention (also referred to herein as 'Alphabodies of the invention'). The invention also provides polypeptides and compositions comprising the class-I viral fusion protein-binding Alphabodies of the invention (referred to herein as 'polypeptides of the invention' and '(pharmaceutical) compositions of the invention', respectively) and the use thereof for prophylactic, therapeutic or diagnostic purposes or as research tools.

In particular, the present invention provides in a first aspect single-chain Alphabodies comprising an alpha-helical binding region, which region mediates binding to a first fusion-driving region of a class-I viral fusion protein, wherein said class-I viral fusion protein is not the gp41 subunit of HIV-1 envelope glycoprotein.

Accordingly, the Alphabodies of the invention have at least one binding region, which is located at, within or on one or more alpha-helices of the Alphabodies (referred to herein as an alpha-helical binding region), wherein this binding region is responsible for binding to a class-I viral fusion protein. Such binding region of an Alphabody of the invention essentially consists of specific residues from the Alphabody which are in contact with the class-I viral fusion protein.

In certain particular embodiments, the binding region, comprised in the Alphabodies of the invention, is located at a solvent-oriented surface of one of the Alphabody alpha-helices. Thus, in these embodiments, the alpha-helical binding region for binding to a class-I viral protein will be directly exposed or will be in direct contact with the solvent, environment, surroundings or milieu in which it is present. In these particular embodiments, the alpha-helical binding region of the Alphabodies of the invention can include several amino acid residues located at heptad b-, c- and f-positions present in a solvent-oriented surface of one of the Alphabody alpha-helices. Accordingly, the alpha-helical binding region, may be a subregion comprising some or all of the b-, c- and f-residues present in one Alphabody alpha-helix.

In certain particular embodiments, at least 9 amino acid residues of the alpha-helical binding region of the Alphabodies of the invention are located at heptad b-, c- and f-positions. Again, these heptad b-, c- and f-positions may be present in one Alphabody alpha-helix. In further particular embodiments, at least 5 of these at least 9 amino acid residues of the alpha-helical binding region of the Alphabodies that are located at heptad b-, c- and f-positions, are identical to the amino acid residues appearing at structurally equivalent positions in a fusion-driving region of the class-I viral fusion protein.

In other particular embodiments, the alpha-helical binding region, comprised in the Alphabodies of the invention, is located at the groove formed by or between two adjacent alpha-helices of the Alphabody. Since the residues present at such a groove between two adjacent alpha-helices in an Alphabody are generally located at heptad e- and g-positions, but some of the more exposed b- and c-positions may also contribute to the binding site located in a groove surface, the alpha-helical binding region may in these particular embodiments include several amino acid residues that are located at heptad band e-positions in one of the two adjacent alpha-helices and at heptad c- and g-positions in the other of the two adjacent alpha-helices. Indeed, where the groove is formed by spatially adjacent alpha-helices running parallel, then the groove is formed by b- and e-residues from a first helix and by c- and g-residues of a second helix.

In these embodiments, the alpha-helical binding region, may be a subregion comprising some or all of the b-, e-, c- and g-residues present in an Alphabody groove formed by two adjacent alpha-helices.

In particular embodiments, the alpha-helical binding region includes at least 10 amino acid residues that are located at heptad b- and e-positions in one of the two adjacent alpha-helices and at heptad c- and g-positions in the other of the two adjacent alpha-helices.

Class-I viral fusion proteins are known to the skilled person and include fusion glycoproteins present or displayed on the surface of enveloped viruses belonging to the families of Orthomyxoviridae, Paramyxoviridae, Retroviridae, Filoviridae or Coronaviridae. For the sake of clarity, the notion 'fusion protein' as in 'viral fusion protein' relates to a viral glycoprotein that aids in driving the fusion process between the membranes of a virus and a target cell, and not to a fusion protein comprising a protein fused to another protein, unless explicitly stated otherwise. A (class-I viral) fusion protein is herein also referred to as an 'F-protein'. Class-I viral F-proteins being displayed at the viral surface are mostly trimers of cleavage-activated heterodimers. Alternatively, an F-protein is a trimeric cleavage-activated glycoprotein which forms a complex with a receptor binding protein (as in the case of Paramyxoviridae). A non-limiting list of examples of class-I viral F-proteins includes influenza virus haemagglutinin (HA), simian virus 5 F1 protein, ebola virus Gp2 protein, HIV-1 envelope glycoprotein (Env), SARS corona virus S1/S2 protein, F protein of respiratory syncytial virus, the HEF protein of influenza C virus, the F protein of Simian parainfluenza virus, the F protein of Human parainfluenza virus, the F protein of Newcastle disease virus, the F2 protein of measles, the F2 protein of Sendai virus, the TM protein of Moloney murine leukemia virus, the gp41 protein of HIV-1, the gp41 protein of Simian immunodeficiency virus, the gp21 protein of Human T-cell leukemia virus 1, the TM protein of Human syncytin-2, the TM protein of Visna virus, the S2 protein of Mouse hepatitis virus, the E2 protein of SARS corona virus, the E protein of Tick-borne encephalitis virus, the E2 protein of Dengue 2 and 3 virus, the E protein of Yellow Fever virus, the E protein of West Nile virus, the E1 protein of Semliki forest virus, the E1 protein of Sindbis virus, the G protein of Rabies virus, the G protein of Vesicular stomatitis virus and the gB protein of Herpes simplex virus (Kielian and Rey Nat Rev Microbiol 2006, 4:67-76).

In particular embodiments, the class-I viral fusion protein to which the Alphabodies of the invention bind is not the gp41 protein of HIV, i.e., the gp41 subunit of HIV-1 envelope glycoprotein (Env).

A target cell as used herein may be any cell that is susceptible to fusion with a virus or susceptible to entry or infection by a virus. Also, a target cell can also be any cell that is susceptible to fusion with another cell which displays class-I viral F-proteins at its surface. Target cells may, in particular embodiments, be mammalian or human cells.

In particular, the present invention provides Alphabodies comprising an alpha-helical binding region, which region mediates binding to a first fusion-driving region of a class-I viral fusion protein, which is not the gp41 subunit of HIV-1 envelope glycoprotein, and which structurally mimics a second fusion-driving region of that class-I viral fusion protein, wherein the first and second fusion-driving regions of the class-I viral fusion protein are regions which, in the viral fusion process, interact to drive the fusion between a virus displaying the class-I viral fusion protein and a target cell.

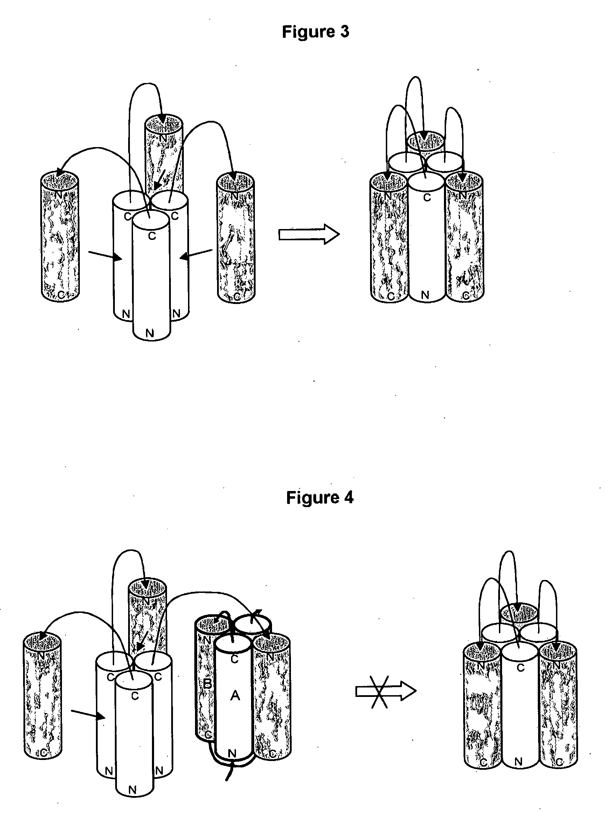

The first and second fusion-driving regions of a class-I viral fusion protein can in principle be any regions present in a class-I viral fusion protein, as long as those first and second fusion-driving regions interact with each other during the viral fusion process and thus contribute to the thermodynamic stability of the postfusion state of the fusion protein. A common characteristic of all class-I F-proteins is that the core of their respective postfusion structures is composed of a central triple-stranded coiled coil region with outer C-terminal antiparallel layers, i.e., structural elements bound to the surface of the central coiled coil structure (Schibli and Weissenhorn Mol Membr Biol 2004, 21:361-371). These outer elements are mostly alpha-helical (in which case a 6-helix bundle structure is formed) or adopt extended conformations, or adopt mixed helical and extended conformations. The formation of a 6-helix bundle by alpha-helical fusion-driving regions of a class-I viral fusion protein is illustrated in Figure 2. The alpha-helical elements constituting the central coiled coil are located in the sequence N-terminally to the elements forming the antiparallel outer layers, as illustrated in Figure 3. The coiled coil and outer elements are kept separated from each other in a metastable native state. Conversion from the metastable prefusion to the highly thermostable postfusion state occurs as a result of a triggering event, which can be a decrease in pH upon endosomal uptake (as in the case of influenza HA) or binding to one or more specific cellular receptors (e.g., as in the case of HIV-1 Env). This conversion is thermodynamically driven by the association of the outer layer elements with the central coiled coil elements (Figure 3); this process provides the necessary free energy for the fusion of viral and target cell membranes. Thus, the fusion-driving regions in a class-I viral F-protein are, on the one hand, the N-terminally located coiled coil forming fragments and, on the other hand, the C-terminally located outer layer fragments. N-terminal fusion-driving regions are in the public domain also referred to as 'heptad repeat 1' ('HR1'), 'N-terminal heptad repeat' ('HRN' or 'HR-N'), 'N-trimer region' ('N-trimer'), 'N-peptide region' or 'coiled coil region'. C-terminal fusion-driving regions are also referred to as 'heptad repeat 2' ('HR2'), 'C-terminal heptad repeat' ('HRC' or 'HR-C') or 'C-peptide region'.

Accordingly, in particular embodiments, the first and second fusion-driving regions of a class-I viral fusion protein to which the alpha-helical binding regions of the Alphabodies of the invention bind and which are structurally mimicked by the alpha-helical binding regions of the Alphabodies of the invention, respectively, may be chosen from the group of viral fusion driving regions consisting of 'heptad repeat 1' ('HR1'), 'N-terminal heptad repeat' ('HRN' or 'HR-N'), 'N-trimer region' ('N-trimer'), 'N-peptide region', 'coiled coil region', 'heptad repeat-2' ('HR2'), 'C-terminal heptad repeat' ('HRC' or 'HR-C') and 'C-peptide region', as long as the first and second fusion-driving regions interact with each other in the viral fusion process.

In particular, the Alphabodies of the invention comprise an alpha-helical binding region, which binds to a first fusion-driving region of a class-I viral fusion protein, which is not the gp41 subunit of HIV-1 envelope glycoprotein, and structurally mimics a second fusion-driving region of the class-I viral fusion protein, which second fusion-driving region interacts with the first fusion-driving region of the class-I viral fusion protein during the fusion process of a virus with a target cell under normal, i.e. biological and natural circumstances. This interaction between the second and the first fusion-driving region of the class-I viral fusion protein occurs when the class-I viral protein is exposed on the surface of a viral particle during the process of viral infection and in the absence of the Alphabodies of the invention or any other compound that may interfere with the viral fusion or entry process.

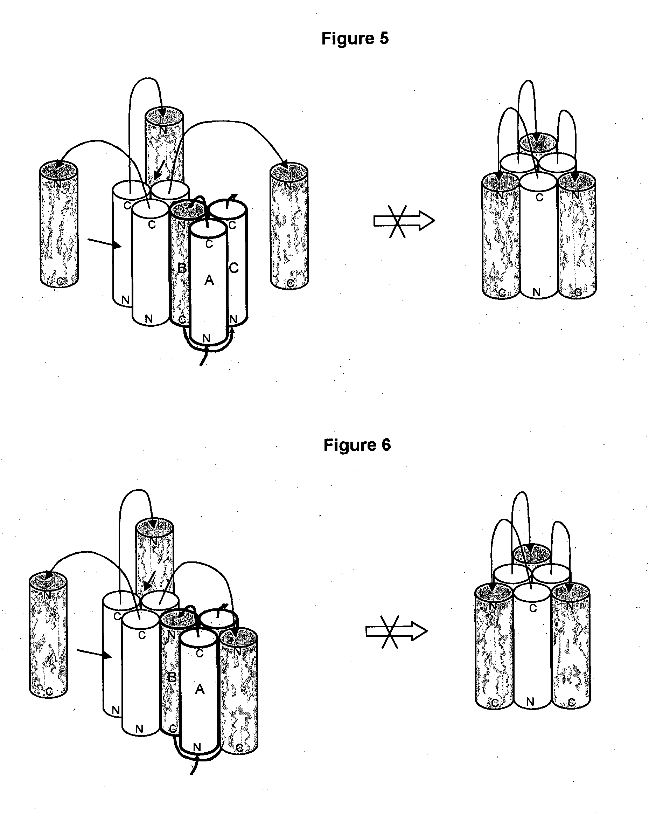

The alpha-helical binding region of the Alphabodies of the invention binds to a first fusion-driving region and structurally mimics, i.e. is structurally similar to, a second fusion-driving region of the class-I viral fusion protein. Thus, the alpha-helical binding region of the Alphabodies of the invention is characterized by a significant degree of structural and functional similarity with the second fusion-driving region of the class-I viral fusion protein to which the Alphabodies bind; such structural similarity can be a similarity in secondary structure (e.g., alpha-helicity) or tertiary structure (e.g., specific amino acid side chains in a specific conformation). The functional similarity is ensured by the binding to the first fusion-driving region of the viral fusion protein. The combination of the binding to the first fusion-driving region of the class-I viral fusion protein and the mimicry of the second fusion-driving region of the viral protein allows the Alphabody to stably replace the second fusion-driving region and disrupt the fusion mechanism. The latter is illustrated in Figures 4 and 5.

In particular embodiments, Alphabodies structurally mimicking a viral F-protein region are provided. An Alphabody binding region structurally mimicking a viral F-protein region, has at least partly the same secondary structure or a similar secondary structure compared to the viral F-protein region. Thus, in particular embodiments, the alpha-helical blinding region, comprised in the Alphabodies of the invention, forms a structural mimic of the secondary structure of the another (herein referred to as the "second", only with reference to the interaction with "the first") fusion-driving region of the class-I viral fusion protein.

In particular embodiments, wherein the alpha-helical binding region of an Alphabody of the invention, which structurally mimics a viral fusion-driving region, is located at a solvent-oriented surface of one of the Alphabody alpha-helices (as illustrated in Figure 5), at least 9 amino acid residues of the alpha-helical binding region are located at heptad b-, c- and f-positions, which are present in one Alphabody alpha-helix. In further particular embodiments, at least 5 of these at least 9 amino acid residues of the alpha-helical binding region that are located at heptad b-, c- and f-positions, are identical to the amino acid residues appearing at structurally equivalent positions in the mimicked fusion-driving region of the class-I viral fusion protein. Such structurally equivalent positions between the alpha-helical region of the Alphabody and the mimicked fusion-driving region of the class-I viral fusion protein to which the Alphabody binds can be identified, for example, by superimposing both structures and by identifying the residues in the mimicked fusion-driving region that overlap with the b-, c- and f-positions of the Alphabody binding region.

In other particular embodiments, wherein the alpha-helical binding region of an Alphabody of the invention, which structurally mimics a viral fusion-driving region, is located at or within the groove between two adjacent alpha-helices of the Alphabody (as illustrated in Figure 4), at least 10 amino acid residues of the alpha-helical binding region are located at heptad b- and e-positions in one of the two adjacent alpha-helices and at heptad c- and g-positions in the other of the two adjacent alpha-helices. In further particular embodiments, at least 5 of those 10 amino acid residues are identical to amino acid residues appearing at structurally equivalent positions in the mimicked fusion-driving region of the class-I viral fusion protein. Again, these structurally equivalent positions between the alpha-helical region of the Alphabody and the mimicked fusion-driving region of the class-I viral fusion protein to which the Alphabody binds can be identified, for example, by superimposing both structures and by identifying the residues in the mimicked fusion-driving region that overlap with the b-, e-, and/or c- and f-positions of the Alphabody binding region.

In further particular embodiments, the invention provides bispecific single-chain Alphabodies comprising two alpha-helical binding regions. The latter is illustrated in Figure 6. One advantage of bispecific binding may be a higher binding affinity or a more potent antiviral effect. Another advantage of bifunctional binding may be a lower propensity for eliciting resistance mutations. Accordingly, certain embodiments of the present invention may be in agreement with the need for new antiviral drugs that are less susceptible to viral resistance.

Such bispecific Alphabodies thus comprise two antiviral functional binding regions or binding sites, wherein these binding sites have a different binding specificity (Figure 6). Thus, a (monomeric, single-domain) Alphabody is considered to be bispecific if it comprises two different binding regions with a different binding specificity. Then, for example, a first binding region may be formed by a surface-exposed region on an Alphabody alpha-helix and have a binding specificity for an N-peptide region in a class-I viral fusion protein, whereas a second binding region may be formed by an Alphabody groove region and have a binding specificity for a C-peptide region. Alternatively, a first Alphabody binding region may have a binding specificity for an N-terminally located subregion of an N-peptide region in a class-I viral fusion protein , whereas a second binding region may have a binding specificity for a subregion that is located more C-terminally in the same N-peptide region. Alternatively, a first binding region may have a binding specificity for an N-peptide region of a first class-I viral fusion protein, whereas a second binding region may have a binding specificity for an N-peptide region of a second class-I viral fusion protein; the first and second class-I viral fusion proteins may be displayed, for example, by different viral strains. A multispecific Alphabody is herein considered an Alphabody with two or more binding specificities located in the same monomeric single-domain Alphabody.

Thus, in particular embodiments, a bispecific Alphabody may comprise one alpha-helical binding region that is located at a solvent-oriented surface of one of the Alphabody alpha-helices and includes at least 9 amino acid residues located at heptad b-, c- and f-positions, and another (i.e., second) alpha-helical binding region that is located at a groove formed by or between two adjacent alpha-helices of the same Alphabody, and includes at least 10 amino acid residues that are located at heptad band e-positions in one of the two adjacent alpha-helices and at heptad c- and g-positions in the other of the two adjacent alpha-helices.

In a further aspect, the present invention provides methods for producing single-chain Alphabodies binding to a class-I viral fusion protein according to the invention. The methods of the present invention are typically aimed at generating Alphabodies, polypeptides and compositions which can bind to one or more of the above-listed or - mentioned class-I viral fusion proteins.

The Alphabodies of the present invention typically bind their epitopes of the viral fusion proteins after they have become accessible as a result of triggering by cellular receptors or by a pH drop. However, in particular embodiments, the Alphabodies of the invention may also bind to the class-I viral fusion protein in its native, usually oligomeric form, on condition that the targeted fusion-driving subregion is at least temporarily accessible. Also, the Alphabodies of the invention may bind to the viral fusion protein of interest in isolated, soluble form. Further, the Alphabodies of the invention may bind to the viral fusion protein of interest as precursor or as mature protein. Further, the Alphabodies of the invention may bind the viral fusion protein of interest in its native conformational state (if the targeted fusion-driving subregion is at least temporarily accessible), in a mechanistic intermediate state (for example, in a fusion-activated or prefusion intermediate state wherein the targeted fusion-driving subregion becomes more fully accessible), or in a postfusion state (again, if the targeted fusion-driving subregion is still at least temporarily accessible). Normally however, the postfusion state will represent the end of the binding window (i.e. when binding is no longer possible) Further, the Alphabodies of the invention may bind the viral fusion protein of interest in a free, unliganded state, or in a receptor-or ligand-bound state. In further particular embodiments, the Alphabodies of the invention may bind to a domain of a viral fusion protein, which is responsible for membrane fusion. Further, the Alphabodies of the invention may bind to a fragment of a full viral fusion protein, such as, but not limited to a domain or a subregion. Such fragment may be obtained, for example, by recombinant or chemical protein synthesis. In particular embodiments, the viral fusion protein is not HIV-1 envelope glycoprotein (HIV-1 Env). In further particular embodiments, the target protein is not HIV-1 gp41. In further particular embodiments, the target protein is not any of subregions HR1 or HR2 of HIV-1 gp41.

In particular embodiments, the Alphabodies, and/or polypeptides of the invention bind to the class-I viral fusion protein of interest with a dissociation constant (KD) of less than about 1 micromolar (1 µM), and preferably less than about 1 nanomolar (1 nM) [i.e., with an association constant (KA) of about 1,000,000 per molar (10⁶ M⁻¹, 1E6 /M) or more, and preferably about 1,000,000,000 per molar (10⁹ M⁻¹, 1 E9 /M) or more]. In particular embodiments, an Alphabody, polypeptide or composition of the invention will bind to the target protein of interest with a kOff ranging between 0.1 and 0.0001 s⁻¹ and/or a kOn ranging between 1,000 and 1,000,000 M⁻¹ s⁻¹.

The ability of the Alphabodies and/or polypeptides of the invention to bind to a viral fusion protein with a particular affinity make them suitable for use in a number of applications, including screening, detection, diagnostic and therapeutic applications as will be described more in detail herein below.

In particular embodiments, the Alphabodies produced by the methods of the present invention are capable of inhibiting, reducing and/or preventing the activity of a viral fusion protein of interest, or, inhibiting, reducing and/or preventing one or more biological or physiological mechanisms, effects, responses, functions pathways or activities in which the viral target protein of interest is involved, such as by at least 10%, but preferably at least 20%, for example by at least 50%, at least 60%, at least 70%, at least 80%, at least 90%, at least 95% or more, as measured using a suitable in vitro, cellular or in vivo assay, compared to the activity of the viral fusion protein of interest in the same assay under the same conditions but without using the Alphabody, polypeptide or composition of the invention.

Thus, in particular embodiments, the Alphabodies, polypeptides and compositions obtainable by the methods of the invention can reduce or inhibit the biological activity of a viral fusion protein, compared to the biological activity of that viral fusion protein in the absence of such molecules of the invention, and this by at least 10%, but preferably at least 20%, for example by at least 50%, at least 60%, at least 70%, at least 80%, at least 90%, at least 95% or more, as determined by a suitable assay known in the art. The binding of the Alphabodies to a viral fusion protein may be such that it still allows the viral fusion protein to bind to its cellular receptor(s), but prevents, reduces or inhibits viral membrane fusion or viral entry into a target cell or viral infection by at least 10%, but preferably at least 20%, for example by at least 50%, at least 60%, at least 70%, at least 80%, at least 90%, at least 95% or more, as determined by a suitable assay known in the art.

Accordingly, in particular embodiments, the Alphabodies, polypeptides and compositions of the present invention can be used for the prevention and treatment of viral diseases which are characterized by viral-mediated biological pathway(s) in which a viral protein, such as a viral fusion protein of interest, or a subdomain or subfragment thereof, is involved.

The present invention provides methods for obtaining the target-specific Alphabodies according to the invention, and more particularly for obtaining Alphabodies specifically binding to or directed against class-I viral fusion proteins. These methods involve or are based on the concept of rational-based design or, in the context of the present invention, 'design by grafting', and rely in part on a certain structural similarity between an Alphabody and particular subregions within a class-I viral fusion protein. More in particular, the methods of the present invention rely in part on the similarity in alpha-helical structure between the Alphabodies of the present invention and particular alpha-helical subregions of class-I viral fusion proteins.

It has been shown that class-I viral fusion proteins drive the process of membrane fusion (between the viral and target cell membranes) by conformational transitions of their surface-displayed fusion proteins wherein, first, a so-called fusion peptide is inserted in the membrane of a target cell (thereby physically linking the virus to the target cell) and, second, by the transition toward an energetically stable postfusion state (thereby exerting a pulling force on the mutual membranes). The postfusion state of class-I viral fusion proteins is characterized by, on the one hand, a trimeric form of a first fusion-driving element, this trimeric form adopting a parallel triple-stranded alpha-helical coiled coil structure and, on the other hand, a second fusion-driving element which tightly binds to the grooves of the central coiled coil structure (three times, one such second element is bound in each of the grooves). It has also been demonstrated that the formation of the postfusion state can be prevented (and, hence, membrane fusion and viral entry can be inhibited) by contacting viruses with peptides that are directly derived from the fusion-driving regions of the class-I viral fusion proteins themselves. In other words, it has been shown that such peptides can bind to their complementary region in the viral fusion protein (either as a free peptide to the viral coiled coil, or as a peptidic coiled coil to the viral coiled coil-binding region). When these peptides succeed to bind, they sterically block the formation of the postfusion state of the viral fusion proteins, thereby thwarting the fusion mechanism and, hence membrane fusion and viral entry. Thus, it has been shown that peptides mimicking fusion-driving regions of class-I viral fusion proteins can be successful inhibitors of viral entry.

The Alphabodies obtained by the methods of the present invention essentially consist of a (single-chain) triple-stranded alpha-helical coiled coil structure. Alphabody structures at least comprise one groove between parallel alpha-helices: there is one such groove in antiparallel Alphabodies and three such grooves in parallel Alphabodies (although two of the latter grooves may be partially obstructed by the presence of the linker fragments). Thus, an Alphabody groove may, in principle, be utilized as a structural mimic of a viral coiled coil groove. In addition, the surface-oriented sides of Alphabody helices are in principle available for binding to a viral coiled coil groove, and can therefore, in principle, form structural mimics of groove-binding helices.

Thus, with respect to its constitution, an Alphabody is essentially also a trimeric coiled coil, although there are major differences between an Alphabody type coiled coil and a class-I viral fusion protein coiled coil:
(i) an Alphabody is a (covalently linked) single-chain protein molecule whereas an N-trimer of a class-I viral fusion protein is a complex of three individual, non-covalently associated heptad repeat fragments;
(ii) the core of an Alphabody consists of a majority (i.e., at least 50%) of isoleucines, while the core of an N-trimer of a class-I viral fusion protein is relatively heterogeneous and is never composed of more than 50% isoleucine residues;
(iii) an Alphabody can exist as a parallel or antiparallel coiled coil, whereas an N-trimer of a class-I viral fusion protein is invariably a parallel coiled coil;
(iv) Alphabody helices mimicking a groove-binding helix (such as an HR2 helix) of a class-I viral fusion protein have the wrong curvature: since the Alphabody helices are part of a coiled coil domain, they bend 'inwards' (i.e., toward the coiled coil center) whereas they should have the opposite curvature to mimic a viral groove-binding alpha-helical region.
(v) an Alphabody is not composed of, or derived from, a naturally occurring amino acid sequence but it is a non-natural sequence that is optimized to adopt a stable fold.

Nonetheless, the present inventors have found that in a parallel or antiparallel Alphabody there is at least one pair of parallel helices which are potentially suitable for redesign with the aim of mimicking an N-terminal fusion-driving region of a class-I viral fusion protein. Analogously, the present inventors have found that in a parallel or antiparallel Alphabody there is at least one solvent-oriented alpha-helical surface which is potentially suitable for redesign with the aim of mimicking a C-terminal fusion-driving region of a class-I viral fusion protein.

One such method of redesign is known in the art as grafting. The technique of grafting comprises the transfer of specific amino acid residues that are selected from a reference structure onto a target structure that is to be (re)designed. Such redesign by grafting aims at mimicking the binding (and optionally also functional) properties of the reference structure by the redesigned target structure. In the context of the present invention, the reference structure is an alpha-helical membrane-driving region from a class-I viral fusion protein, whereas the redesigned structure is an Alphabody, and more in particular, an alpha-helical region within an Alphabody.

Accordingly, by performing the methods of the invention, Alphabodies are produced which structurally mimic a membrane fusion-driving region of a class-I viral fusion protein. Such fusion driving region can be either a coiled coil-forming N-terminal fusion-driving region (also known as HR1, HRN, HR-N, N-trimer region, N-trimer, N-peptide region or coiled coil region) or a coiled coil-binding C-terminal fusion-driving region (also known as HR2, HRC, HR-C or C-peptide region). The present inventors have now found that by structurally mimicking one of the two such types of membrane fusion-driving regions of a class-I viral fusion protein, an Alphabody of the invention is able to target, i.e., interact with or bind to, the other type of membrane fusion-driving region (in the same viral fusion protein), thereby preventing the natural interaction between these two types of membrane-fusion driving regions and thus preventing the formation of the postfusion state (such as a six-helix bundle state) of the viral fusion protein.

Grafting is a non-obvious approach because of the multitude of simultaneously 'transplanted' amino acid side chains. Moreover, grafting of amino acid residues from class-I fusion protein subregions onto Alphabodies is also non-obvious because of the various structural differences between such fusion protein subregions and Alphabodies, as indicated above.

Accordingly, the inventors have contemplated the redesign of an Alphabody binding region to mimic a fusion-driving region of a class-I viral fusion protein by way of selecting and grafting specific residues onto the Alphabody.

Thus, a first step in the methods for producing single-chain Alphabodies binding to a viral fusion protein according to the invention comprises selecting a fusion-driving region of a class-I viral fusion protein which can be used for "grafting" onto the Alphabody according to the invention.

A selected fusion-driving region of a class-I viral fusion protein can in principle be any region from a class-I viral fusion protein which contributes to the formation and thermodynamic stability of the postfusion state of said fusion protein. The fusion-driving regions in a class-I viral F-protein are, on the one hand, the N-terminally located coiled coil-forming fragments and, on the other hand, the C-terminally located outer layer fragments. N-terminal fusion-driving regions are also referred to as 'heptad repeat 1' ('HR1'), 'N-terminal heptad repeat' ('HRN' or 'HR-N'), 'N-trimer region' ('N-trimer'), 'N-peptide region' or 'coiled coil region'. C-terminal fusion-driving regions are also referred to as 'heptad repeat 2' ('HR2'), 'C-terminal heptad repeat' ('HRC' or 'HR-C') or 'C-peptide region'.

For the production of Alphabodies, where the grafted alpha-helical binding region is to be located at a solvent-oriented surface of one of the Alphabody alpha-helices, the selection of a membrane fusion-driving region more specifically comprises the selection of a C-terminal fusion driving region, such as but not limited to 'heptad repeat 2' ('HR2'), 'C-terminal heptad repeat' ('HRC' or 'HR-C') or 'C-peptide region'.

For the production of Alphabodies, where the grafted alpha-helical binding region is to be located at or within a groove formed between two adjacent alpha-helices of the Alphabody, the selection of a membrane fusion-driving region more specifically comprises the selection of an N-terminal fusion driving region, such as but not limited to 'heptad repeat 1' ('HR1'), 'N-terminal heptad repeat' ('HRN' or 'HR-N'), 'N-trimer region' ('N-trimer'), 'N-peptide region' or 'coiled coil region'.

The second step in the methods of the invention for the production of class-I viral fusion protein-binding Alphabodies using the rational-based design comprises identifying in the selected fusion-driving region those amino acid residues that are involved in the interaction with another, i.e. complementary, fusion-driving region in the same fusion protein. Indeed, the present inventors have found that by mimicking the amino acids that are involved in the interaction between two membrane fusion driving regions, an Alphabody can be produced which specifically interferes with such interaction and, hence with the membrane fusion mechanism.

After having selected a fusion-driving region of a class I viral protein and having identified the amino acid residues residing therein that are involved in the interaction with another fusion-driving region, the methods of the invention for the rational-based design of a class I viral protein binding Alphabody comprise the further step of selecting in an Alphabody an alpha-helical region which may potentially form a structural mimic of the selected viral fusion-driving region. Such a selection is based on the fact that the alpha-helical region is to form a structural mimic of at least the secondary structure of the selected fusion-driving region. In these embodiments, wherein the Alphabody to be produced is an Alphabody having a alpha-helical binding region located at a solvent-oriented surface, the methods further comprise the step of identifying in this alpha-helical region the heptad b-, c- and f-positions. In other particular embodiments, wherein the Alphabody to be produced is an Alphabody having an alpha-helical binding region located at or within a groove formed between two adjacent alpha-helices of the Alphabody, the methods further comprise the step of identifying in this alpha-helical region the heptad b- and e- and/or c- and f-positions. These positions are located as defined herein and can be retrieved by the skilled person on the basis of the description herein and suitable methods known in the art.

Indeed, where the Alphabody to be produced is an Alphabody having a rationally designed alpha-helical binding region located at a solvent-oriented surface, a number of amino acid residues of the alpha-helical binding region are located at heptad b-, c- and f-positions, which may be present in consecutive heptads in one Alphabody alpha-helix.

These amino acid residues of the alpha-helical binding region that are located at heptad b-, c- and f-positions, are to be linked, identified or matched with the amino acid residues appearing at structurally equivalent positions in the mimicked fusion-driving region of the class-I viral fusion protein. Such structurally equivalent positions between the alpha-helical region of the Alphabody and the mimicked fusion-driving region of the class-I viral fusion protein can be identified, for example, by structurally superimposing both structures and identifying the residues in the mimicked fusion-driving region that overlap or coincide with the b-, c- and f-positions of the Alphabody binding region.

Similarly, where the alpha-helical binding region of the Alphabody obtained through rational design is located at or within the groove between two adjacent alphabody helices of the Alphabody, a number of amino acid residues of the alpha-helical binding region are located at heptad b- and e-positions in one of the two adjacent alpha-helices and at heptad c- and g-positions in the other of the two adjacent alpha-helices.

These amino acid residues of the alpha-helical binding region that are located at heptad b- and e-positions and at heptad c- and g-positions, are to be linked, identified or matched with the amino acid residues appearing at structurally equivalent positions in the mimicked fusion-driving region of the class-I viral fusion protein. Again, these structurally equivalent positions between the alpha-helical region of the Alphabody and the mimicked fusion-driving region of the class-I viral fusion protein can be identified by superimposing both structures and identifying the residues in the mimicked fusion-driving region that overlap or coincide with the b-, e-, c- and f-positions of the Alphabody binding region.

Accordingly, a further step in the methods for the rational-based approach for the production of the Alphabodies of the invention comprises matching the identified amino acid residues within the fusion-driving region of the class-I viral protein with the heptad b-, c- and f-positions or with the heptad b-, e-, c- and g-positions identified in the alpha-helical binding region of the Alphabody.

Subsequently, at least 5 of the identified amino acid residues within the fusion-driving region of the class-I viral protein are chosen and introduced, transferred or grafted onto heptad b-, c- and f-positions in one alpha-helix of the Alphabody (in order to produce an Alphabody having a solvent-exposed binding region) or onto b- and e-positions in one of two adjacent alpha-helices and at heptad c- and g-positions in the other of two adjacent alpha-helices (in order to produce an Alphabody having a groove-located binding region) based on the information obtained in the preceding matching step.

In a further step of the methods of the invention for the production of class-I viral fusion protein-binding Alphabodies using a rational-based approach, the Alphabodies comprising the amino acid residues that have been introduced, grafted or transferred onto the particular heptad positions in the selected alpha-helical binding region are produced.

For instance, the Alphabodies produced by the methods of the present invention can be synthesized using recombinant or chemical synthesis methods known in the art. Also, the Alphabodies produced by the methods of the present invention can be produced by genetic engineering techniques. Thus, methods for synthesizing an Alphabody produced by the methods of the present invention may comprise transforming or infecting a host cell with a nucleic acid or a vector encoding an Alphabody sequence having detectable binding affinity for, or detectable in vitro activity on, a viral fusion protein of interest. Accordingly, the Alphabody sequences having detectable binding affinity for, or detectable in vitro activity on, a viral fusion protein of interest can be made by recombinant DNA methods. DNA encoding the Alphabodies can be readily synthesized using conventional procedures. Once prepared, the DNA can be introduced into expression vectors, which can then be transformed or transfected into host cells such as E. coli or any suitable expression system, in order to obtain the expression of Alphabodies in the recombinant host cells and/or in the medium in which these recombinant host cells reside.

Transformation or transfection of nucleic acids or vectors into host cells may be accomplished by a variety of means known to the person skilled in the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

Suitable host cells for the expression of the desired Alphabodies may be any eukaryotic or prokaryotic cell (e.g., bacterial cells such as E. coli, yeast cells, mammalian cells, avian cells, amphibian cells, plant cells, fish cells, and insect cells), whether located in vitro or in vivo. For example, host cells may be located in a transgenic animal.

According to particular embodiments, the methods of the invention comprise the production of bispecific single-chain Alphabodies comprising two alpha-helical binding regions, wherein one alpha-helical binding region is located at a solvent-oriented surface of one of the Alphabody alpha-helices and includes at least 9 amino acid residues located at heptad b-, c- and f-positions, and wherein the other alpha-helical binding region is located at the groove formed by or between two adjacent alpha-helices of the Alphabody, and the alpha-helical binding region includes at least 10 amino acid residues that are located at heptad b- and e-positions in one of the two adjacent alpha-helices and at heptad c- and g-positions in the other of the two adjacent alpha-helices.

More particularly, in said methods, at least one of said alpha-helical binding regions is generated by the methods described above comprising selecting a fusion-driving region, identifying the amino acid residues therein interacting with a complementary fusion-driving region, selecting an alpha-helical region located at a groove or a solvent-oriented surface of an Alphabody, matching the amino acid residues of the fusion-driving region with amino acid residues in the Alphabody region and transferring these amino-acids to these regions and producing the corresponding Alphabody comprising the transferred amino-acids.

In particular embodiments of the methods of the invention for producing bi-specific antibodies, only one of the alpha-helical binding regions is obtained by the methods described above, and the other alpha-helical binding region is obtained by a random-based screening method. Random-based screening methods for developing Alphabodies against viral targets are described in co-pending application WO2012/092971. More particularly, the methods of the invention may further comprise the provision of a second alpha-helical binding region to a fusion-driving protein by a method which comprises the steps of:
a) producing a single-chain Alphabody library comprising at least 100 different-sequence single-chain Alphabody polypeptides, wherein said Alphabody polypeptides differ from each other in at least one of a defined set of 5 to 20 variegated amino acid residue positions, and wherein at least 70% of said variegated amino acid residue positions are located either:
   (i) at heptad e-positions in a first alpha-helix of the Alphabody polypeptides and at heptad g-positions in a second alpha-helix, and optionally at heptad b-positions in said first alpha-helix of the Alphabody polypeptides and/or at heptad c-positions in said second alpha-helix of the Alphabody polypeptides, or
   (ii) at heptad b-, c- and f-positions in one alpha-helix of the Alphabody polypeptides
b) selecting from said single-chain Alphabody library at least one single-chain Alphabody having detectable binding affinity for said fusion driving protein.
Typically, an alpha-helical binding region against a first epitope of a fusion driving protein obtained by random variegation of amino acid residues in specified positions of a solvent-oriented alpha-helix is combined with an alpha helical binding region against a second epitope of a fusion-driving protein in a groove formed by or between two adjacent alpha-helices of the Alphabody obtained by rational design as described herein above, or vice versa.

In particular embodiments, the methods of the invention comprise providing an Alphabody with a first binding site against an epitope of a fusion driving protein by random variegation of amino acid residues in specified positions and screening for binding to said epitope, and the resulting Alphabody is further modified to introduce a second alpha-helical binding region directed against another epitope of a fusion driving protein by specific transfer of amino acids corresponding to the amino acids of a complementary fusion driving region interacting therewith as described above.

An Alphabody selected based on binding with the viral fusion protein of interest can thus be used as a basis for a rational-based approach, to introduce another binding region to a fusion protein. More particularly, it is envisaged that where the binding region obtained by the random library approach is directed against a first fusion protein region, a further binding region on the Alphabody directed against part of a complementary fusion protein of said first fusion protein region can be introduced by rational design.

The Alphabodies obtained by the methods of the present invention may further be optimized by on one or more modifications of the amino acids, which can affect stability of the Alphabody in isolation and/or in complex with a target molecule.

As further described herein, the total number of amino acid residues in an Alphabody of the invention can be in the range of about 50 to about 210, depending mainly on the number of heptads per heptad repeat sequence and the length of the flexible linkers interconnecting the heptad repeat sequences. Parts, fragments, analogs or derivatives of an Alphabody, polypeptide or composition of the invention are not particularly limited as to their length and/or size, as long as such parts, fragments, analogs or derivatives still have the biological function of an Alphabody, polypeptide or composition of the invention from which they are derived and can still be used for the envisaged (pharmacological) purposes.

In a further aspect, the present invention provides Alphabody polypeptides that comprise or essentially consist of at least one Alphabody of the present invention that specifically binds to a class I viral fusion protein (also referred to herein as polypeptides of the invention). The polypeptides of the invention may comprise at least one Alphabody of the present invention and optionally one or more further groups, moieties, residues optionally linked via one or more linkers.

Accordingly, a polypeptide of the invention may optionally contain one or more further groups, moieties or residues for binding to other targets or target proteins of interest. It should be clear that such further groups, residues, moieties and/or binding sites may or may not provide further functionality to the Alphabodies of the invention (and/or to the polypeptide or composition in which it is present) and may or may not modify the properties of the Alphabody of the invention. Such groups, residues, moieties or binding units may also for example be chemical groups which can be biologically and/or pharmacologically active.

These groups, moieties or residues are, in particular embodiments, linked N- or C-terminally to the Alphabody. In particular embodiments however, one or more groups, moieties or residues are linked to the body of the Alphabody, e.g. via coupling to a cysteine in an alpha-helix.

In particular embodiments, the polypeptides of the present invention comprise Alphabodies that have been chemically modified. For example, such a modification may involve the introduction or linkage of one or more functional groups, residues or moieties into or onto the Alphabody of the invention. These groups, residues or moieties may confer one or more desired properties or functionalities to the Alphabody of the invention. Examples of such functional groups will be clear to the skilled person and include, without limitation, a purification tag, a detection tag, a fluorescent tag, a glycan moiety, a PEG moiety.

The introduction or linkage of functional groups to an Alphabody of the invention may also have the effect of an increase in the half-life, the solubility and/or the stability of the Alphabody of the invention, or it may have the effect of a reduction of the toxicity of the Alphabody of the invention, or it may have the effect of the elimination or attenuation of any undesirable side effects of the Alphabody of the invention, and/or it may have the effect of other advantageous properties.

In particular embodiments, the Alphabody polypeptides of the present invention comprise Alphabodies that have been modified to specifically increase the half-life thereof, for example, by means of PEGylation, by means of the addition of a group or protein or protein domain which binds to or which is a serum protein (such as serum albumin) or, in general, by linkage of the Alphabody to a moiety that increases the half-life of the Alphabody of the invention. Typically, the polypeptides of the invention with increased half-life have a half-life that is at least twice, such as at least three times, such as at least five times, for example at least ten times or more than ten times greater than the half-life of the corresponding Alphabody of the invention lacking the said chemical modification.

A particular modification of the Alphabody polypeptides of the invention may comprise the introduction of one or more detectable labels or other signal-generating groups or moieties, depending on the intended use of the labeled Alphabody.

Yet a further particular modification may involve the introduction of a chelating group, for example to chelate one or more metals or metallic cations.

A particular modification may comprise the introduction of a functional group that is one part of a specific binding pair, such as the biotin-(strept)avidin binding pair.

For some applications, in particular for those applications in which it is intended to kill a viral particle or cell that expresses the target which the Alphabodies of the invention specifically bind to, or to reduce or slow the growth and/or proliferation of such a viral particle or cell, the Alphabodies of the invention may also be linked to a toxin or to a toxic residue or moiety.

Other potential chemical and enzymatic modifications will be clear to the skilled person.

In particular embodiments, the one or more groups, residues, moieties are linked to the Alphabody via one or more suitable linkers or spacers.

In further particular embodiments, the Alphabody polypeptides of the invention comprise two or more target-specific Alphabodies. In such particular embodiments, the two or more target-specific Alphabodies may be linked (coupled, concatenated, interconnected, fused) to each other either in a direct or in an indirect way. In embodiments wherein the two or more Alphabodies are directly linked to each other, they are linked without the aid of a spacer or linker fragment or moiety. Alternatively, in embodiments wherein the two or more Alphabodies are indirectly linked to each other, they are linked via a suitable spacer or linker fragment or linker moiety.

In embodiments wherein two or more Alphabodies are directly linked, they may be produced as single-chain fusion constructs (i.e., as single-chain protein constructs wherein two or more Alphabody sequences directly follow each other in a single, contiguous amino acid sequence). Alternatively, direct linkage of Alphabodies may also be accomplished via cysteines forming a disulfide bridge between two Alphabodies (i.e., under suitable conditions, such as oxidizing conditions, two Alphabodies comprising each a free cysteine may react with each other to form a dimer wherein the constituting momomers are covalently linked through a disulfide bridge).

Alternatively, in embodiments wherein two or more Alphabodies are indirectly linked, they may be linked to each other via a suitable spacer or linker fragment or linker moiety. In such embodiments, they may also be produced as single-chain fusion constructs (i.e., as single-chain protein constructs wherein two or more Alphabody sequences follow each other in a single, contiguous amino acid sequence, but wherein the Alphabodies remain separated by the presence of a suitably chosen amino acid sequence fragment acting as a spacer fragment). Alternatively, indirect linkage of Alphabodies may also be accomplished via amino acid side groups or via the Alphabody N- or C-termini. For example, under suitably chosen conditions, two Alphabodies comprising each a free cysteine may react with a homo-bifunctional chemical compound, yielding an Alphabody dimer wherein the constituting Alphabodies are covalently cross-linked through the said homo-bifunctional compound. Analogously, one or more Alphabodies may be cross-linked through any combination of reactive side groups or termini and suitably chosen homo- or heterobifunctional chemical compounds for cross-linking of proteins.

In particular embodiments of linked Alphabodies, the two or more linked Alphabodies can have the same amino acid sequence or different amino acid sequences. The two or more linked Alphabodies can also have the same binding specificity or a different binding specificity. The two or more linked Alphabodies can also have the same binding affinity or a different binding affinity.

Suitable spacers or linkers for use in the coupling of different Alphabodies of the invention will be clear to the skilled person and may generally be any linker or spacer used in the art to link peptides and/or proteins. In particular, such a linker or spacer is suitable for constructing proteins or polypeptides that are intended for pharmaceutical use.

Some particularly suitable linkers or spacers for coupling of Alphabodies in a single-chain amino acid sequence include for example, but are not limited to, polypeptide linkers such as glycine linkers, serine linkers, mixed glycine/serine linkers, glycine- and serine-rich linkers or linkers composed of largely polar polypeptide fragments. Some particularly suitable linkers or spacers for coupling of Alphabodies by chemical cross-linking include for example, but are not limited to, homo-bifunctional chemical cross-linking compounds such as glutaraldehyde, imidoesters such as dimethyl adipimidate (DMA), dimethyl suberimidate (DMS) and dimethyl pimelimidate (DMP) or N-hydroxysuccinimide (NHS) esters such as dithiobis(succinimidylpropionate) (DSP) and dithiobis(sulfosuccinimidylpropionate) (DTSSP). Examples of hetero-bifunctional reagents for cross-linking include, but are not limited to, cross-linkers with one amine-reactive end and a sulfhydryl-reactive moiety at the other end, or with a NHS ester at one end and an SH-reactive group (e.g., a maleimide or pyridyl disulfide) at the other end.

A polypeptide linker or spacer for usage in single-chain concatenated Alphabody constructs may be any suitable (e.g., glycine-rich) amino acid sequence having a length between 1 and 50 amino acids, such as between 1 and 30, and in particular between 1 and 10 amino acid residues. It should be clear that the length, the degree of flexibility and/or other properties of the spacer(s) may have some influence on the properties of the final polypeptide of the invention, including but not limited to the affinity, specificity or avidity for a viral fusion protein of interest, or for one or more other target proteins of interest. It should be clear that when two or more spacers are used in the polypeptides of the invention, these spacers may be the same or different. In the context and disclosure of the present invention, the person skilled in the art will be able to determine the optimal spacers for the purpose of coupling Alphabodies of the invention without any undue experimental burden.

The linked Alphabody polypeptides of the invention can generally be prepared by a method which comprises at least one step of suitably linking the one or more Alphabodies of the invention to the one or more further groups, residues, moieties and/or other Alphabodies of the invention, optionally via the one or more suitable linkers, so as to provide a polypeptide of the invention.

Also, the polypeptides of the present invention can be produced by methods at least comprising the steps of: (i) expressing, in a suitable host cell or expression system, the polypeptide of the invention, and (ii) isolating and/or purifying the polypeptide of the invention. Techniques for performing the above steps are known to the person skilled in the art.

The present invention also encompasses parts, fragments, analogs, mutants, variants, and/or derivatives of the Alphabodies and polypeptides of the invention and/or polypeptides comprising or essentially consisting of one or more of such parts, fragments, analogs, mutants, variants, and/or derivatives. In particular embodiments, these parts, fragments, analogs, mutants, variants, and/or derivatives are capable of binding to a class I viral fusion protein of interest. Most particularly the binding affinity of the part, fragment, analog, mutant, variant, and/or derivative of the Alphabodies and polypeptides bind to the class I viral fusion protein of interest with a binding affinity which is comparable or increased compared to the Alphabody from which it is derived. In particular embodiments, the parts, fragments, analogs, mutants, variants, and/or derivatives of the Alphabodies and polypeptides are suitable for the prophylactic, therapeutic and/or diagnostic purposes envisaged herein. Such parts, fragments, analogs, mutants, variants, and/or derivatives according to the invention are still capable of specifically binding to a viral fusion protein.

It should be noted that the Alphabodies, polypeptides or compositions of the invention (or of the nucleotide sequences of the invention used to express them) are not naturally occurring proteins (or nucleotide sequences). Furthermore, the present invention is also not limited as to the way that the Alphabodies, polypeptides or nucleotide sequences of the invention have been generated or obtained. Thus, the Alphabodies of the invention may be synthetic or semi-synthetic amino acid sequences, polypeptides or proteins.

The Alphabodies, polypeptides and compositions provided by the invention can be in essentially isolated form (as defined herein), or alternatively can form part of a polypeptide or composition of the invention, which may comprise or essentially consist of at least one Alphabody of the invention and which may optionally further comprise one or more other groups, moieties or residues (all optionally linked via one or more suitable linkers or spacers).

It will be appreciated based on the disclosure herein that for prophylactic, therapeutic and/or diagnostic applications, the Alphabodies, polypeptides and compositions of the invention will in principle be directed against or specifically bind to class-I viral fusion proteins of human viruses. However, where the Alphabodies, polypeptides and compositions of the invention are intended for veterinary purposes, they will be directed against or specifically bind to viral fusion proteins from viruses which are able to infect and reproduce themselves in the particular (such as mammalian) species be treated, or they will be at least cross-reactive with viral fusion proteins from viruses which are able to infect and reproduce themselves in the particular (such as mammalian) species be treated. Accordingly, Alphabodies, polypeptides and compositions that specifically bind to viral fusion proteins from viruses which are able to infect and reproduce themselves in one subject species may or may not show cross-reactivity with viral fusion proteins from viruses which are able to infect and reproduce themselves in one or more other subject species. Of course it is envisaged that, in the context of the development of Alphabodies for use in humans or animals, Alphabodies may be developed which bind to viral fusion proteins from a virus which is able to infect and reproduce itself in another species than that which is to be treated for use in research and laboratory testing.

It is also expected that the Alphabodies and polypeptides of the invention may bind to some naturally occurring or synthetic analogs, variants, mutants, alleles, parts and fragments of viral fusion proteins. More particularly, it is expected that the Alphabodies and polypeptides of the invention will bind to at least those analogs, variants, mutants, alleles, parts and fragments of a viral fusion protein that (still) contain the binding site, part or domain of the (natural/wild-type) viral fusion protein and/or the viral fusion protein to which those Alphabodies and polypeptides bind.

In particular embodiments the Alphabodies, polypeptides and compositions that specifically bind to a class-1 viral fusion protein of interest do not show cross-reactivity with a naturally occurring protein other than a class-1 viral fusion protein, most particularly other than the target protein of interest.

In yet a further aspect, the invention provides nucleic acid sequences encoding single-chain Alphabodies binding to class I viral fusion proteins (also referred to herein as 'nucleic acid sequences of the invention') as well as vectors and host cells comprising such nucleic acid sequences.

In a further aspect, the present invention provides nucleic acid sequences encoding the Alphabodies or the polypeptides of the invention (or suitable fragments thereof). These nucleic acid sequences are also referred to herein as nucleic acid sequences of the invention and can also be in the form of a vector or a genetic construct or polynucleotide. The nucleic acid sequences of the invention may be synthetic or semi-synthetic sequences, nucleotide sequences that have been prepared by PCR using overlapping primers, or nucleotide sequences that have been prepared using techniques for DNA synthesis known per se.

The genetic constructs of the invention may be DNA or RNA, and are preferably double-stranded DNA. The genetic constructs of the invention may also be in a form suitable for transformation of the intended host cell or host organism or in a form suitable for integration into the genomic DNA of the intended host cell or in a form suitable for independent replication, maintenance and/or inheritance in the intended host organism. For instance, the genetic constructs of the invention may be in the form of a vector, such as for example a plasmid, cosmid, YAC, a viral vector or transposon. In particular, the vector may be an expression vector, i.e., a vector that can provide for expression in vitro and/or in vivo (e.g., in a suitable host cell, host organism and/or expression system).

In a further aspect, the invention provides vectors comprising nucleic acids encoding single-chain Alphabodies, which are obtainable by the methods according to the invention.

In yet a further aspect, the present invention provides host cells comprising nucleic acids encoding single-chain Alphabodies of the invention or vectors comprising these nucleic acids. Accordingly, a particular embodiment of the invention is a host cell transfected or transformed with a vector comprising the nucleic acid sequence encoding the Alphabodies of the invention and which is capable of expressing the Alphabodies. Suitable examples of hosts or host cells for expression of the Alphabodies or polypeptides of the invention will be clear to the skilled person and include any suitable eukaryotic or prokaryotic cell (e.g., bacterial cells such as E. coli, yeast cells, mammalian cells, avian cells, amphibian cells, plant cells, fish cells, and insect cells), whether located in vitro or in vivo.

A further aspect of the invention relates to the use of the Alphabodies and polypeptides of the present invention to detect a class-I viral fusion protein of interest in vitro or in vivo.

In particular embodiments, the Alphabodies and polypeptides of the present invention comprise a label or other signal-generating moiety. Suitable labels and techniques for attaching labels on Alphabodies are known in the art. These include, but are not limited to, fluorescent labels, phosphorescent labels, chemiluminescent labels, bioluminescent labels, radio-isotopes, metals, metal chelates, metallic cations, chromophores and enzymes.

Such labeled Alphabodies and polypeptides of the invention may for example be used for in vitro, in vivo or in situ assays (including immunoassays known per se such as ELISA, RIA, EIA and other 'sandwich assays', etc.) as well as in vivo diagnostic and imaging purposes, depending on the choice of the specific label.

In further particular embodiments, the invention relates to the use of the target-specific Alphabodies and polypeptides of the invention for drug delivery. More particularly, it can be envisaged that the Alphabodies of the present invention, as a result of their specific binding to a class-I viral fusion protein, can be designed to deliver, upon binding to their viral protein target (typically naturally located at the surface of a virus), an antiviral compound.

A further aspect of the invention relates to the use of the Alphabodies, polypeptides and pharmaceutical compositions of the invention for inhibition, reduction and/or prevention of a biological activity of a class-I viral fusion protein, as can be measured using a suitable in vitro, cellular or in vivo assay. The Alphabodies, polypeptides and pharmaceutical compositions of the present invention can also be used to inhibit, reduce and/or prevent one or more biological or physiological mechanisms, effects, responses, functions pathways or activities in which such viral fusion protein is involved. Such an action of the Alphabody, polypeptide or composition according to the invention as an antagonist, in the broadest possible sense, may be determined in any suitable manner and/or using any suitable (in vitro and usually cellular or in vivo) assay known in the art, depending on the type of inhibition, reduction and/or prevention of the said one or more biological or physiological mechanisms, effects, responses, functional pathways or activities in which the said viral fusion protein is involved. Non-limiting examples of such types of functional effects include (i) the (indirect) prevention of attachment of the virus to cellular receptors on specific, dedicated target cells, (ii) the (indirect) prevention of interaction with the glycocalyx of target cells, (iii) the arrest of a viral fusion protein in a conformational or mechanistic state that is intermediate to the native and postfusion states, (iv) the irreversible functional deactivation of a viral fusion protein prior to attachment to a target cell, and wherein said deactivation is further characterized by the inability of said viral fusion protein to recover membrane fusion activity even after removal of the antiviral Alphabody, polypeptide or composition according to the invention.

In view of the ability of the Alphabodies, polypeptides and compositions of the invention to inhibit viral protein functions in vivo, the present invention also envisages pharmaceutical compositions. Thus, in yet a further aspect, the present invention provides pharmaceutical compositions comprising one or more Alphabodies, polypeptides and/or nucleic acid sequences according to the invention and optionally at least one pharmaceutically acceptable carrier (also referred to herein as pharmaceutical compositions of the invention). According to certain particular embodiments, the pharmaceutical compositions of the invention may further optionally comprise at least one other pharmaceutically active compound.

The pharmaceutical compositions of the present invention can be used in the diagnosis, prevention and/or treatment of diseases and disorders associated with viral diseases, more particularly with viral infection, viral entry or viral fusion being mediated by a viral fusion protein.

In particular, the present invention provides pharmaceutical compositions comprising Alphabodies and polypeptides of the invention that are suitable for prophylactic, therapeutic and/or diagnostic use in a warm-blooded animal, and in particular in a mammal, and more in particular in a human being.

The present invention also provides pharmaceutical compositions comprising Alphabodies and polypeptides of the invention that can be used for veterinary purposes in the prevention and/or treatment of one or more diseases, disorders or conditions associated with and/or mediated by a viral fusion protein.

Generally, for pharmaceutical use, the polypeptides of the invention may be formulated as a pharmaceutical preparation or compositions comprising at least one Alphabody or polypeptide of the invention and at least one pharmaceutically acceptable carrier, diluent or excipient and/or adjuvant, and optionally one or more further pharmaceutically active polypeptides and/or compounds. Such a formulation may be suitable for oral, parenteral, topical administration or for administration by inhalation. Thus, the Alphabodies, or polypeptides of the invention and/or the compositions comprising the same can for example be administered orally, intraperitoneally, intravenously, subcutaneously, intramuscularly, transdermally, topically, by means of a suppository, by inhalation, again depending on the specific pharmaceutical formulation or composition to be used. The clinician will be able to select a suitable route of administration and a suitable pharmaceutical formulation or composition to be used in such administration.

The pharmaceutical compositions may also contain suitable binders, disintegrating agents, sweetening agents or flavoring agents. Tablets, pills, or capsules may be coated for instance with gelatin, wax or sugar and the like. In addition, the Alphabodies and polypeptides of the invention may be incorporated into sustained-release preparations and devices.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. Antibacterial and antifungal agents and the like can optionally be added.

Useful dosages of the Alphabodies and polypeptides of the invention can be determined by comparing their in vitro activity, and in vivo activity in animal models. Methods for the extrapolation of effective dosages in mice, and other animals, to humans are known to the skilled person.

The amount of the Alphabodies and polypeptides of the invention required for used in prophylaxis and/or treatment may vary not only with the particular Alphabody or polypeptide selected but also with the route of administration, the nature of the condition being treated and the age and condition of the patient and will be ultimately at the discretion of the attendant physician or clinician.

The Alphabodies or polypeptides of the invention and/or the compositions comprising the same are administered according to a regimen of treatment that is suitable for preventing and/or treating the disease or disorder to be prevented or treated. The clinician will generally be able to determine a suitable treatment regimen. Generally, the treatment regimen will comprise the administration of one or more Alphabodies and/or polypeptides of the invention, or of one or more compositions comprising the same, in one or more pharmaceutically effective amounts or doses.

The desired dose may conveniently be presented in a single dose or as divided doses (which can again be sub-dosed) administered at appropriate intervals. An administration regimen could include long-term (i.e., at least two weeks, and for example several months or years) or daily treatment.

The Alphabodies and polypeptides of the present invention will be administered in an amount which will be determined by the medical practitioner based inter alia on the severity of the condition and the patient to be treated. Typically, for each disease indication an optimal dosage will be determined specifying the amount to be administered per kg body weight per day, either continuously (e.g. by infusion), as a single daily dose or as multiple divided doses during the day. The clinician will generally be able to determine a suitable daily dose, depending on the factors mentioned herein. It will also be clear that in specific cases, the clinician may choose to deviate from these amounts, for example on the basis of the factors cited above and his expert judgment.

In particular embodiments, the Alphabodies and polypeptides of the invention may be used in combination with other pharmaceutically active compounds or principles that are or can be used for the prevention and/or treatment of the diseases and disorders cited herein, as a result of which a synergistic effect may or may not be obtained. Examples of such compounds and principles, as well as routes, methods and pharmaceutical formulations or compositions for administering them will be clear to the clinician.

According to a further aspect, the present invention provides the use of Alphabodies or polypeptides of the invention that specifically bind to a class-I viral fusion protein as a medicament or for the preparation of a medicament for the prevention and/or treatment of a viral fusion protein-mediated disease and/or disorder in which said viral fusion protein is involved. Accordingly, the invention provides Alphabodies, polypeptides and pharmaceutical compositions specifically binding to a viral fusion protein for use in the prevention and/or treatment of a virus-mediated disease and/or disorder in which said viral fusion protein is involved. In particular embodiments, the present invention also provides methods for the prevention and/or treatment of a viral fusion protein-mediated disease and/or disorder, comprising administering to a subject in need thereof, a pharmaceutically active amount of one or more Alphabodies, polypeptides and/or pharmaceutical compositions of the invention. In particular, the pharmaceutically active amount may be an amount that is sufficient (to create a level of the Alphabody or polypeptide in circulation) to inhibit, prevent or decrease the biological or physiological mechanisms, effects, responses, functional pathways or activities in which viral fusion proteins are involved.

The subject or patient to be treated with the Alphabodies or polypeptides of the invention may be any warm-blooded animal, but is in particular a mammal, and more in particular a human suffering from, or at risk of, diseases and disorders in which the viral fusion protein to which the Alphabodies or polypeptides of the invention specifically bind to are involved.

'Viral diseases (and disorders)' or 'virus-mediated diseases' as used in the context of the present invention can be defined as diseases and disorders that are caused by one or more viruses. In particular embodiments, viral diseases are diseases that can be prevented and/or treated by suitably administering to a subject in need thereof (i.e., having the disease or disorder or at least one symptom thereof and/or at risk of attracting or developing the disease or disorder) an Alphabody, polypeptide or composition of the invention. More particularly, these viral diseases are diseases and disorders in which the biological activity of (a) viral fusion protein(s) is/are involved.

Examples of such viral diseases will be clear to the skilled person based on the disclosure herein, and for example include the following diseases and disorders (caused by the following viruses): AIDS (caused by HIV), AIDS Related Complex (caused by HIV), Aseptic meningitis (caused by HSV-2), Bronchiolitis (caused by e.g. RSV), Common cold (caused by e.g. RSV or Parainfluenza virus), Conjunctivitis (caused by e.g. Herpes simplex virus), Croup (caused by e.g. parainfluenza viruses 1 to 3), Dengue fever (caused by dengue virus), Eastern equine encephalitis (caused by EEE virus), Ebola hemorrhagic fever (caused by Ebola Virus), encephalitis and chronic pneumonitis in sheep (caused by Visna virus), encephalitis (caused by Semliki Forest virus), Gingivostomatitis (caused by HSV-I), Genital herpes (caused by HSV-2), Herpes labialis (caused by HSV-I), neonatal herpes (caused by HSV-2), Genital HSV (caused by Herpes simplex virus), Influenza (Flu) (caused by influenza viruses A, B and C), Japanese encephalitis virus (caused by JEE virus), Keratoconjunctivitis (caused by HSV-I), Lassa fever, Leukemia and lymphoma (caused by e.g. Human T cell leukemia virus or Moloney murine leukemia virus), Lower respiratory tract infections (caused by e.g. RSV or Sendai virus), Measles (caused by rubeola virus), Marburg hemorrhagic fever (caused by Marburg virus), Molluscum contagiosum (caused by Molluscum), Mononucleosis-like syndrome (caused by CMV), mumps (caused by mumps virus), Newcastle disease (caused by avian paramoxyvirus 1), Norovirus, Orf (caused by Orf virus), Pharyngitis (caused by e.g. RSV, Influenza virus, Parainfluenza virus and Epstein-Barr virus), Pneumonia (viral) (caused by e.g. RSV or CMV), Progressive multifocal leukencephalopathy, Rabies (caused by Rabies virus), Roseola (caused by HHV-6), Rubella (caused by rubivirus), SARS (caused by a human coronavirus), Shingles (caused by Varicella zoster virus), Smallpox (caused by Variola virus), St. Louis encephalitis (caused by SLE virus), Strep Throat (caused by e.g. RSV, Influenza viruses, Parainfluenza virus, Epstein-Barr virus), Sindbis fever (Sindbis virus), Temporal lobe encephalitis (caused by HSV-I), Urethritis (caused by Herpes simplex virus), Vesicular stomatitis (caused by vesicular stomatitis virus), Viral encephalitis, Viral gastroenteritis, Viral meningitis, Viral pneumonia, Western equine encephalitis (caused by WEE virus), West Nile disease, Yellow fever (caused by Yellow Fever virus), and Zoster (caused by Varicella zoster virus).

In particular embodiments, the Alphabodies of the invention are used in the treatment of a disease caused by a virus comprising a class I viral fusion protein, more particularly by a virus which enters a target cell by way of the activity of a class-I viral fusion protein.

Examples of such viruses include but are not limited to influenza virus, simian virus, ebola virus, HIV-1, SARS corona virus, respiratory syncytial virus, influenza C virus, Simian parainfluenza virus, Human parainfluenza virus, Newcastle disease virus, measles, Sendai virus, Moloney murine leukemia virus, Human T-cell leukemia virus 1, Human syncytin-2, Visna virus, Mouse hepatitis virus, SARS corona virus, Tick-borne encephalitis virus, Dengue 2 and 3 virus, Yellow Fever virus, West Nile virus, Semliki forest virus, Sindbis virus, Rabies virus, Vesicular stomatitis virus and Herpes simplex virus. In a most particular embodiment, the virus is of the family of the Paramixoviridae and the Alphabodies of the invention are used to treat a patient suffering from a disease caused by a virus of the family of the Paramixoviridae.

Thus, in further particular embodiments, the Alphabodies of the present invention are used to treat and prevent progression of diseases selected from the group consisting of AIDS (caused by HIV), AIDS Related Complex (caused by HIV), Aseptic meningitis (caused by HSV-2), Bronchiolitis (caused by e.g. RSV), Common cold (caused by e.g. RSV or Parainfluenza virus), Conjunctivitis (caused by e.g. Herpes simplex virus), Croup (caused by e.g. parainfluenza viruses 1 to 3), Dengue fever (caused by dengue virus), Eastern equine encephalitis (caused by EEE virus), Ebola hemorrhagic fever (caused by Ebola virus), encephalitis and chronic pneumonitis in sheep (caused by Visna virus), encephalitis (caused by Semliki Forest virus), Gingivostomatitis (caused by HSV-I), Genital herpes (caused by HSV-2), Herpes labialis (caused by HSV-I), neonatal herpes (caused by HSV-2), Genital HSV (caused by Herpes simplex virus), Influenza (Flu) (caused by influenza viruses A, B and C), Keratoconjunctivitis (caused by HSV-I), Lassa fever, Leukemia and lymphoma (caused by e.g. Human T cell leukemia virus or Moloney murine leukemia virus), Lower respiratory tract infections (caused by e.g. RSV Measles (caused by rubeola virus), Newcastle disease (caused by avian paramoxyvirus 1), Pharyngitis (caused by e.g. RSV, Influenza virus, Parainfluenza virus and Epstein-Barr virus), Pneumonia (viral) (caused by e.g. RSV), Progressive multifocal leukencephalopathy, Rabies (caused by Rabies virus), SARS (caused by a human coronavirus), , Strep Throat (caused by e.g. RSV, Influenza viruses, Parainfluenza virus, Epstein-Barr virus), Sindbis fever (Sindbis virus), Temporal lobe encephalitis (caused by HSV-I), Urethritis (caused by Herpes simplex virus), Vesicular stomatitis (caused by vesicular stomatitis virus), West Nile disease, and Yellow fever (caused by Yellow Fever virus.

In particular embodiments, the Alpahbodies of the invention are used to inhibit an infection by one or more of the viruses described herein.

In particular embodiments, the Alphabodies of the invention are used for eliciting an immune response against a virus comprising a class I viral fusion protein, more particularly by a virus which enters a target cell by way of the activity of a class-I viral fusion protein.

In further particular embodiments, the Alphabodies of the invention are used as vaccines for the prevention of one or more of the diseases described herein, more particularly one or more diseases caused by a virus comprising a class I viral fusion protein, more particularly by a virus which enters a target cell by way of the activity of a class-I viral fusion protein.

The efficacy of the Alphabodies and polypeptides of the invention, and of compositions comprising the same, can be tested using any suitable in vitro assay, cell-based assay, in vivo assay and/or animal model known per se, or any combination thereof, depending on the specific disease or disorder involved. Suitable assays and animal models will be clear to the skilled person, and for example include those listed in McMahon et al., Curr. Opin. Infect. Dis. 2009, 22:574-582, as well as the assays and animal models used in the experimental part below and in the prior art cited herein. Depending on the viral fusion protein(s) involved, the skilled person will generally be able to select a suitable in vitro assay, cellular assay or animal model to test the Alphabodies and polypeptides of the invention for their capacity to affect the activity of these viral fusion proteins, and/or the biological mechanisms in which these are involved; and for their therapeutic and/or prophylactic effect in respect of one or more diseases and disorders that are associated with a viral fusion protein.

### BRIEF DESCRIPTION OF THE DRAWINGS

The invention is herein illustrated by means of the following non-limiting **Examples and Figures,** in which the **FIGURES** show:
**Figure 1**: Parallel and antiparallel Alphabodies. Panel A, parallel Alphabody; panel B, antiparallel Alphabody. Parallel helices are represented by white cylinders, whereas the helix that is antiparallel to the two others in panel B is depicted as a gray cylinder. Curved arrows connecting different helices represent linker fragments. The tiny non-connecting arrows represent N- and C-terminal extensions to the Alphabody. Helices are labeled A, B, C according to their appearance in the Alphabody, which is composed of a single-chain amino acid sequence.
**Figure 2**: Formation of a 6-helix bundle composed of N-terminal and C-terminal fusion driving regions of a class-I viral fusion protein. White cylinders represent N-terminal fusion driving regions forming a trimeric parallel coiled coil (N-trimer); gray cylinders represent C-terminal fusion-driving regions binding to the grooves in between pairs of N-terminal fusion driving region-helices. Labels 'N' and 'C' denote the N- and C-terminal ends of the helices, respectively. Thus, C-terminal helices are bound in antiparallel orientation relative to the N-terminal helices. The result of the binding is a 6-helix bundle, as depicted to the right of the white arrow.
**Figure 3**: Formation of a 6-helix bundle composed of N-terminal and C-terminal fusion-driving regions from a class-I viral fusion protein, interconnected by a loop region. Shading and labeling is as in Figure 2. Curved arrows represent connecting segments between N-terminal fusion-driving regions (white cylinders) and C-terminal fusion-driving regions (gray cylinders) in class I viral fusion proteins prior to formation of the 6-helix bundle (to the left of the white arrow) and after formation of the 6-helix bundle (at the right). The curved arrows representing connecting segments are not indicative of the size or structure of these segments and can be relatively short hairpin loops or large, structured subunits. The mutual orientation of N-terminal and C-terminal helices in native or receptor-activated spikes is not necessarily the same as depicted in the left panel; the latter intends to illustrate that N-terminal and C-terminal fragments are covalently interconnected yet separated in space.
**Figure 4**: Binding of an Alphabody to a fusion driving region of a class I viral fusion protein. Shading and labeling is as in Figures 1 and 2. The Alphabody is represented by bold cylinders. This figure illustrates that an Alphabody bound to a C-terminal fusion-driving region of a class-I viral fusion protein prevents the latter from binding to the N-trimer and thereby precludes formation of a 6-helix bundle. The possibility that two or three C-terminal fusion-driving regions in a class-I viral protein are bound by an equal number of Alphabodies is not shown in the figure, but also not excluded by it.
**Figure 5**: Binding of an Alphabody to N-terminal fusion-driving region of a class-I viral fusion protein. Shading and labeling is as in Figure 4. This figure illustrates that an Alphabody bound to an N-terminal fusion-driving region of a class-I viral fusion protein prevents the latter from binding to a C-terminal fusion-driving region of that class-I viral fusion protein and thereby precludes formation of a 6-helix bundle. The possibility that two or three Alphabodies bind to an equal number of N-trimer grooves of a class-I viral fusion protein is not shown in the figure, but also not excluded by it.
**Figure 6**: Simultaneous binding of an Alphabody to both an N-terminal fusion-driving region and a C-terminal fusion-driving region of a class-I viral fusion protein. Shading and labeling is as in Figure 4. This figure illustrates that an Alphabody bound simultaneously to an N-trimer and to a C-terminal fusion-driving region of a class-I viral fusion protein prevents the latter from binding to the N-trimer and thereby precludes formation of a 6-helix bundle. This figure thus illustrates a bifunctional or a bispecific Alphabody. The possibility that two or three Alphabodies bind in a similar way to a class-I viral fusion protein is not shown in the figure, but also not excluded by it.

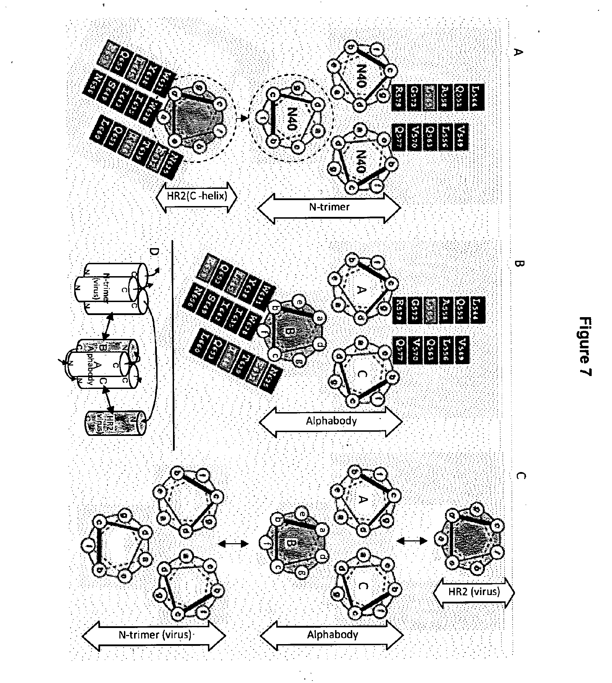

**Figure 7**: Structural aspects related to residue grafting. Panel A shows a representation of three N-terminal and one C-terminal fusion-driving regions of a class-I viral fusion protein. In this example, the N-trimer formed by three N-terminal fusion-driving regions is taken to be the N40 sequence as published in Root et al. Science 2001, 291: 884-888.
The HR2 (i.e., C-terminal fusion-driving region) sequence is taken to be a C38 sequence (ibid). Helices are looked upon with the N-trimer Z-axis pointing backward (N-terminus in front) and with the HR2 helical axis pointing forward (N-terminus at the back). Thus, the encircled helices are antiparallel to each other. The small arrow between the circles suggests a superposition of an HR2 (C-)helix on top of an N-helix, without considering the mismatch in direction. In practice this would correspond to mapping of HR2 positions c, f, b, e, a, d, g onto N-helix positions d, a, e, b, f, c, g, respectively. Replacing the N-helix by the fitted C-helix would give a construct similar to the one depicted in panel B.
However, an immediate and serious problem of this way of recombining N- and C-peptide fragments is the inevitable disturbance of the core packing. Key to the present invention is that such problem does not exist when using antiparallel Alphabodies. It is sufficient to graft the HR2 positions labeled d, a and e in panel A onto positions labeled b, f and c in panel B, respectively, to obtain an Alphabody construct with a well-packed isoleucine-core and which displays both an HR2-binding groove and a helix carrying the groove-binding HR2 residues. Panels C and D illustrate how such construct could capture the respective regions in a viral gp41 molecule; the views in panels C and D are along and perpendicular to the helical axes, respectively (all helices are idealized and the supercoiling is ignored). The double arrows symbolize non-covalent interactions (binding). The long arrow connecting an N-helix with an HR2 helix from the virus represents the hairpin loop. Panel D clearly illustrates that helix B in the Alphabody (with the grafted HR2 residues) and a pair of helices from the viral N-trimer (forming the binding site) are antiparallel. Likewise, the groove-forming helices A and C of the Alphabody and the viral HR2 helix are also antiparallel. This figure thus explains the rationale that lies at the basis of the construction of a bispecific Alphabody with the potential to simultaneously target the N-trimer and an HR2 fragment in HIV-1 Env spikes.

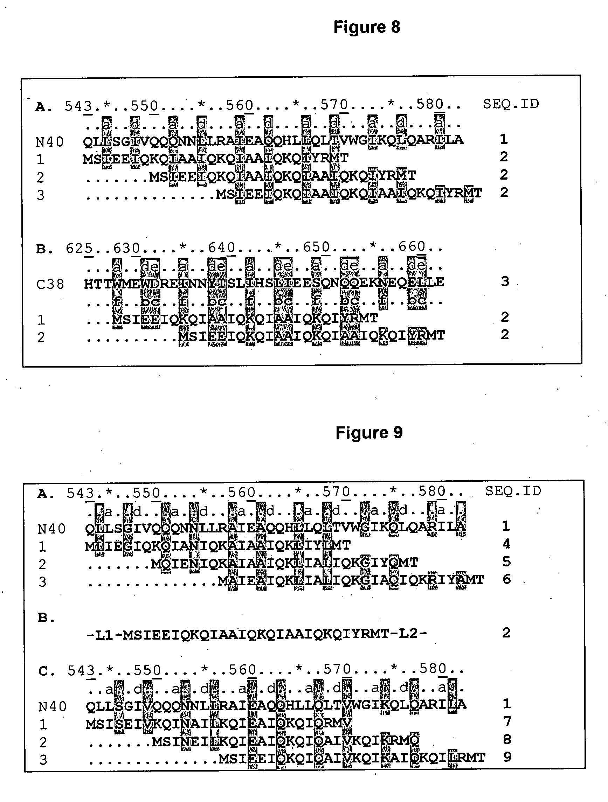

**Figure 8****:** Sequence alignments of an Alphabody with HIV gp41 HR1 and HR2 sequences. A, alignment of gp41 HR1 sequence denoted 'N40' (residues 543 to 582 of gp160 HXB2, also provided as SEQ ID NO: 1) with the sequence of selected Alphabody denoted 'scAB013' (only 1 helix thereof; sequence also provided as SEQ ID NO: 2) in three different frames. Heptad a/d-positions are shaded in gray. B, alignment of HR2 sequence denoted 'C38' (residues 625 to 662 of gp160 HXB2, also provided as SEQ ID NO: 3) with the sequence of selected Alphabody scAB013 (1 helix) in two different frames, such that C38 a-, d- and e-positions map onto the Alphabody f-, b- and c-positions, respectively. In this way, the HR2 (C38) contact residues (shaded in gray), when grafted onto the Alphabody, would face away from the center of the Alphabody.
**Figure 9****:** Initial selection of groove amino acids. A, selection of residues to be grafted at g- and c-positions of an Alphabody A-helix (shaded in gray) in the three registers that are possible for Alphabody/HR1 alignment. The resulting sequences labeled 1-3 are also provided as SEQ ID NO: 4, SEQ ID NO: 5 and SEQ ID NO: 6, respectively. B, amino acid sequence of the non-mutated Alphabody B-helix (SEQ ID NO: 2), supplemented with appropriate flanking linkers L1 and L2. C, selection of residues to be grafted at e-and b-positions of an Alphabody C-helix (shaded in gray) in the three possible heptad registers. The resulting sequences labeled 1-3 are also provided as SEQ ID NO: 7, SEQ ID NO: 8 and SEQ ID NO: 9, respectively.

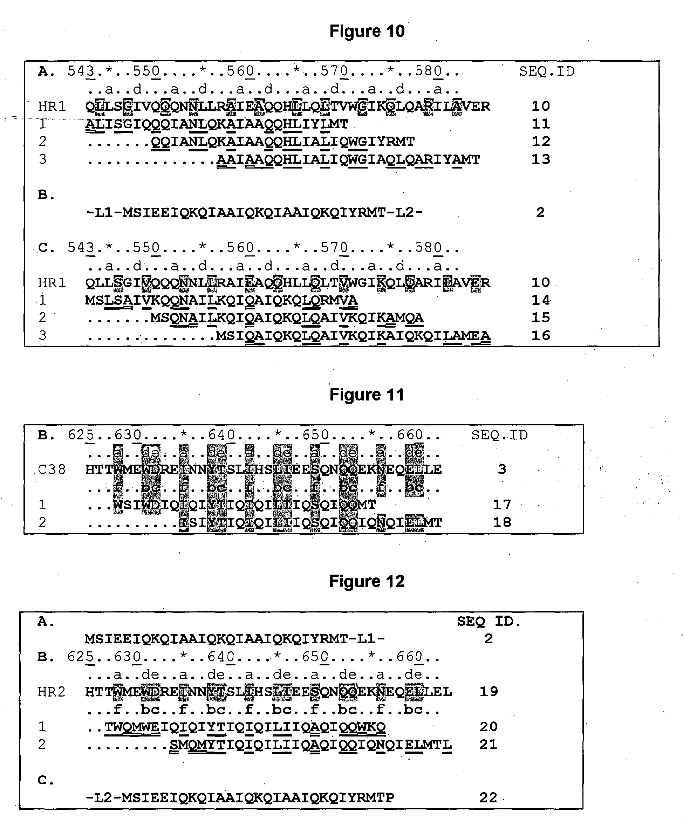

**Figure 10****:** Structurally optimized groove sequences. A, alignment of HR1 sequence labeled 'HR1' (residues 543 to 585 of gp160 HXB2, also provided as SEQ ID NO: 10) with the amino acid sequences of the structurally optimized A-helix in the three registers that are possible for Alphabody/HR1 alignment. The resulting sequences labeled 1-3 are also provided as SEQ ID NO: 11, SEQ ID NO: 12 and SEQ ID NO: 13, respectively. B, amino acid sequence of the non-mutated Alphabody B-helix (SEQ ID NO: 2), supplemented with appropriate flanking linkers L1 and L2. C, alignment of the HR1 sequence (SEQ ID NO: 10) with the amino acid sequences of the structurally optimized C-helix in the three possible alignment registers. The resulting sequences labeled 1-3 are also provided as SEQ ID NO: 14, SEQ ID NO: 15 and SEQ ID NO: 16, respectively. Residues appearing in the reference Alphabody scAB013 are not underlined. Singly underlined residues are grafted from HR1 amino acids. Doubly underlined residues are mutations selected on basis of structural considerations.
**Figure 11****:** Initial grafting of HR2 residues. B, amino acid sequence of Alphabody B-helix with grafted HR2 residues (shaded in gray) in two possible registers for alignment to HR2 peptide C38 (SEQ ID NO: 3). The resulting sequences labeled 1 and 2 are also provided as SEQ ID NO: 17 and SEQ ID NO: 18, respectively. The label 'B.' only serves to indicate that grafting is to be performed in the Alphabody B-helix.
**Figure 12****:** Structurally optimized B-helix sequences. A, amino acid sequence of the non-mutated Alphabody A-helix (SEQ ID NO: 2), supplemented with an appropriate flanking linker L1. B, alignment of the HR2 sequence (SEQ ID NO: 19) with the structurally optimized B-helix in the two registers that are possible for Alphabody/HR2 alignment. The resulting sequences labeled 1 and 2 are also provided as SEQ ID NO: 20 and SEQ ID NO: 21, respectively. C, amino acid sequence of the non-mutated Alphabody C-helix (SEQ ID NO: 22), preceded with an appropriate flanking linker L2. Residues appearing in the reference Alphabody scAB013 are not underlined. Singly underlined residues are grafted from HR2 amino acids. Doubly underlined residues are mutations selected on basis of structural considerations.

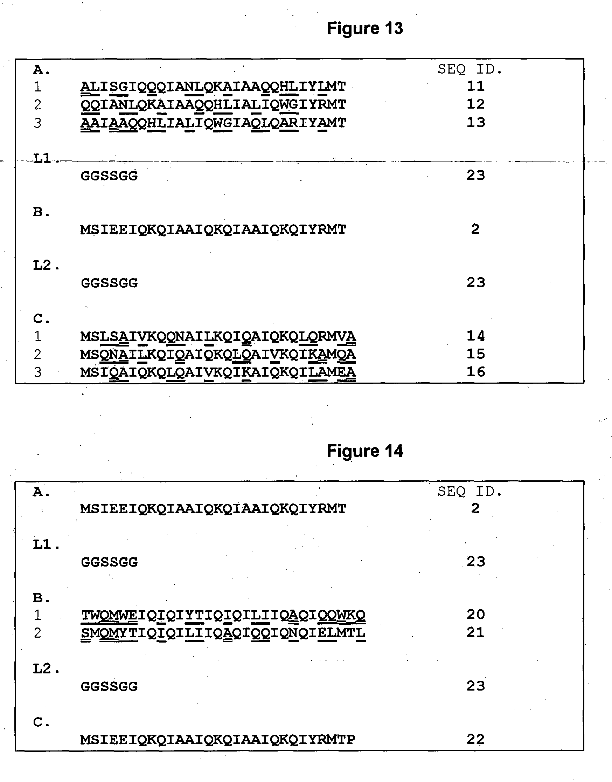

**Figure 13****:** Final Alphabody constructs with N-trimer-like binding grooves. A, 3 amino acid sequences (labels 1-3 correspond to SEQ ID NO: 11, SEQ ID NO: 12 and SEQ ID NO: 13, respectively) to be incorporated as the A-helix in an Alphabody; L1, linker 1 sequence (SEQ ID NO: 23); B, amino acid sequence (SEQ ID NO: 2) to be incorporated as the B-helix in an Alphabody, irrespective of A- and C-helix sequences; L2, linker 2 sequence (SEQ ID NO: 23); C, 3 amino acid sequences (labels 1-3 correspond to SEQ ID NO: 14, SEQ ID NO: 15 and SEQ ID NO: 16, respectively) to be incorporated as the C-helix in an Alphabody. Residues are underlined according to the same conventions as in Figure 10. The sequences are to be concatenated in an Alphabody in the order Ai-L1-B-L2-Ci, where i refers to any of the indices preceding the sequences under A and C. Thus, this figures represents three different constructs that are designed to target different sub-regions of HR2 in HIV-1 gp41.
**Figure 14****:** Final Alphabody constructs with HR2-like interface residues. A, amino acid sequence (SEQ ID NO: 2) to be incorporated as the A-helix in an Alphabody, irrespective of the B-helix sequence; L1, linker 1 sequence (SEQ ID NO: 23); B, 2 amino acid sequences (labels 1 and 2 correspond to SEQ ID NO: 20 and SEQ ID NO: 21, respectively) to be incorporated as the B-helix in an Alphabody; L2, linker 2 sequence (SEQ ID NO: 23); C, amino acid sequence (SEQ ID NO: 22) to be incorporated as the C-helix in an Alphabody, irrespective of the B-helix sequence; Residues are underlined according to the same conventions as in Figure 12. The sequences are to be concatenated in an Alphabody in the order A-L1-Bi-L2-C, where i refers to any of the indices preceding the sequences under B. Thus, this figures represents two different constructs that are designed to target different sub-regions of the N-trimer in HIV-1 gp41.

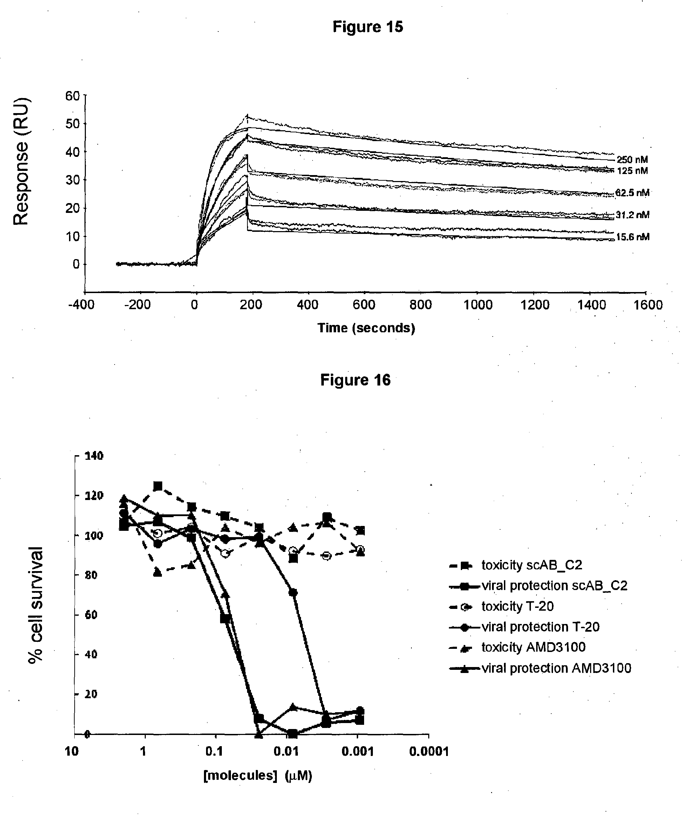

**Figure 15****:** Binding kinetics of scAB013_C2 to immobilized biotinylated HIV-1 N51. SPR sensorgrams were recorded at concentrations of scAB013_C2 ranging from 7.8 nM to 4000 nM. Sensorgrams at concentrations of 15.6, 31.2, 62.5, 125 and 250 nM (shown in the figure) were taken into account for the kinetic analysis. Curves correspond to sensorgrams subtracted with the signal recorded on irrelevant peptide and bulk corrected by subtraction of the sensorgram recorded with 0 nM of Alphabody. The sensorgrams showing scatter represent the experimental data whereas those without scatter are the mathematically fitted data.
**Figure 16****:** HIV inhibitory capacity of scAB013_C2 using MT4-X4 cells and the laboratory adapted HIV strain HXB2. scAB013_C2, denoted 'scAB_C2' in the figure, was tested in three-fold dilutions starting at 2 microM (square symbol, solid line). The toxicity of scAB_C2 was tested in the same concentration range (dashed line). T-20 (circle symbol) and AMD3100 (triangle symbol) were used as controls in the assay.

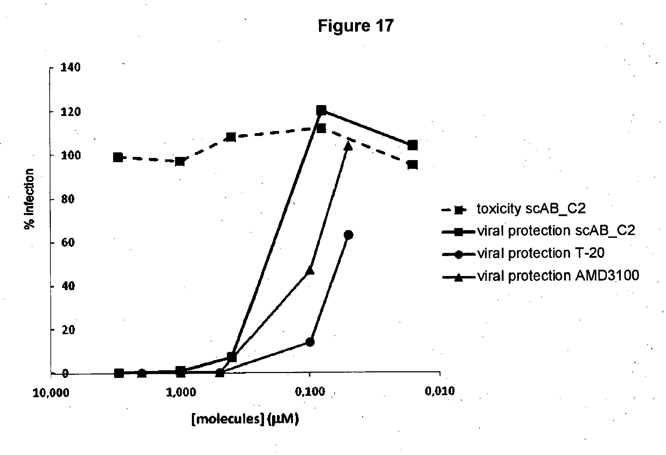

**Figure 17****:** HIV inhibitory capacity of scAB013_C2 using PBMC cells and the laboratory adapted HIV strain HXB2. The inhibitory capacity of scAB013_C2 (denoted 'scAB_C2' in the figure; square symbols, solid line) was measured by monitoring the production of p24 protein. The concentration of viral p24 after 7 days of infection of PBMC with virus in absence of inhibitory molecules was set as 100% infection. In the presence of inhibitory molecules, less p24 was measured and the % infection dropped. T-20 (circle symbol) and AMD3100 (triangle symbol) were used as controls in the assay. The toxicity of the scAB_C2 was measured by monitoring the cell viability using MTT (dashed line) and is to be read as % of cell survival instead of % infection.

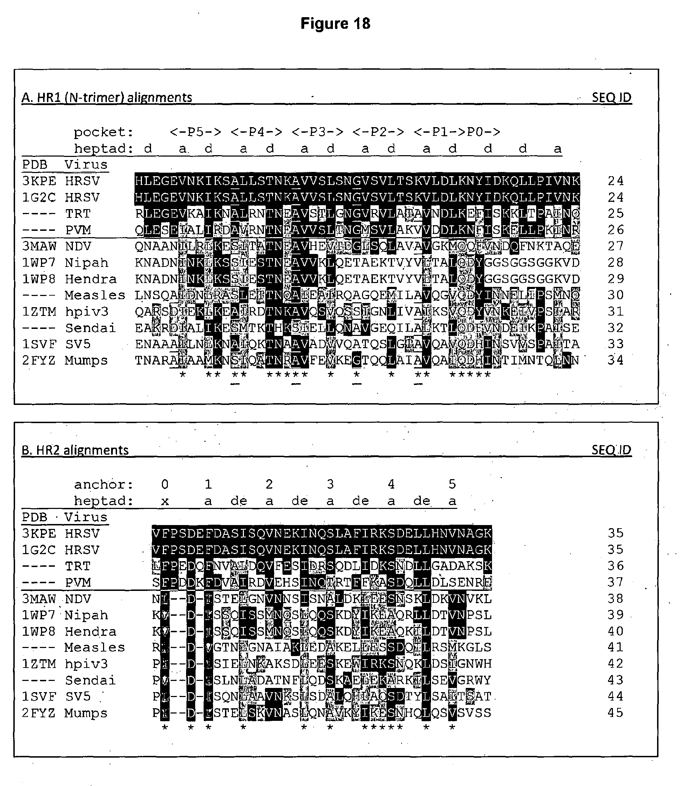

**Figure 18****:** Alignment of selected paramyxoviridae sequences. The selection was based on BLAST hits using the HRSV HR1 sequence (labeled '3KPE') as input, appended with sequences in Table I of Smith et al. Protein Eng 2002, 15:365-371. Pockets P0-P5 or pocket-filling residues ('anchors') 0-5 and heptad positions (shown at the top of each panel) were assigned by visual inspection of the HRSV core 3-D structure (PDB entry 3KPE). Anchors labeled 0-5 in panel B occupy pockets P0-P5 in panel A. The anchor residues labeled 'x' pack in a clear pocket (P0) but they are not in an a-position in pneumovirinae. HR2 d- and e-positions are not contained in real pockets but point in opposite, lateral directions. Asterisks (*) at the bottom indicate strong conservation (≥ 8/11). Black or gray shading is used to indicate identical or similar amino acids, respectively. Small residues at g-positions in HR1 are underlined. Dark gray shading is used for aliphatic pocket residues 0 and 1 in panel B. 'PDB' denotes 'Protein Data Bank entry code'. Virus names are as follows: HRSV, human respiratory syncytial virus; TRT turkey rhinotracheitis virus; PVM, pneumonia virus of mice; NDV, Newcastle disease virus; Nipah, Nipah virus; Hendra, Hendra virus; Measles, measles virus; hpiv3, human parainfluenza virus 3; Sendai, Sendai virus; SV5, simian parainfluenza virus 5; Mumps, mumps virus. The HR1 sequences in panel A for HRSV, TRT, PVM, NDV, Nipah, Hendra, Measles, hpiv3, Sendai, SV5 and Mumps are also provided as SEQ ID No: 24 to SEQ ID No: 34, respectively. The HR2 sequences in panel B for the same viruses are also provided as, respectively, SEQ ID No: 35 to SEQ ID No: 45.

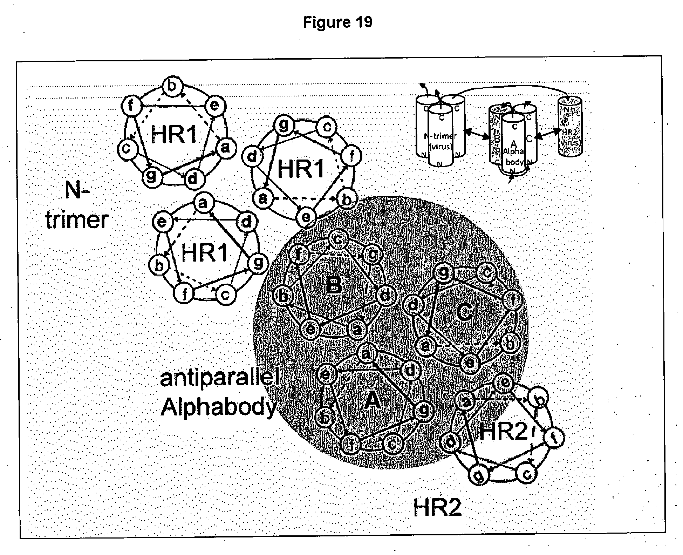

**Figure 19****:** HRSV-F grafting strategy. The HRSV HR1 N-trimer helices are shown at the top left with a view on the C-terminus (a-position at the back). The viral HR2 helix is shown at the bottom with a view on its N-terminus (a-position in front). The shaded area indicates a bispecific antiparallel Alphabody where helices A and C are parallel and B antiparallel. The Alphabody is bound with its B-helix sticking to the viral N-trimer groove and, at the opposite side, with the viral HR2 fragment binding to its A/C-groove. Thus, in order to create two monofunctional or one bifunctional mimic, the a/d/e-residues from HR2 are in principle to be transferred to the b/c/f-positions in the Alphabody B-helix. Reversely, the HR1 c/g-residues are to be grafted onto the same positions in the Alphabody A-helix and b/e-residues onto Alphabody C-helix positions. In addition, certain core residues might be considered a well. The inset at the top-right shows a side view projection of a bifunctional Alphabody binding simultaneously to HR1 and HR2 from the same viral fusion protein.

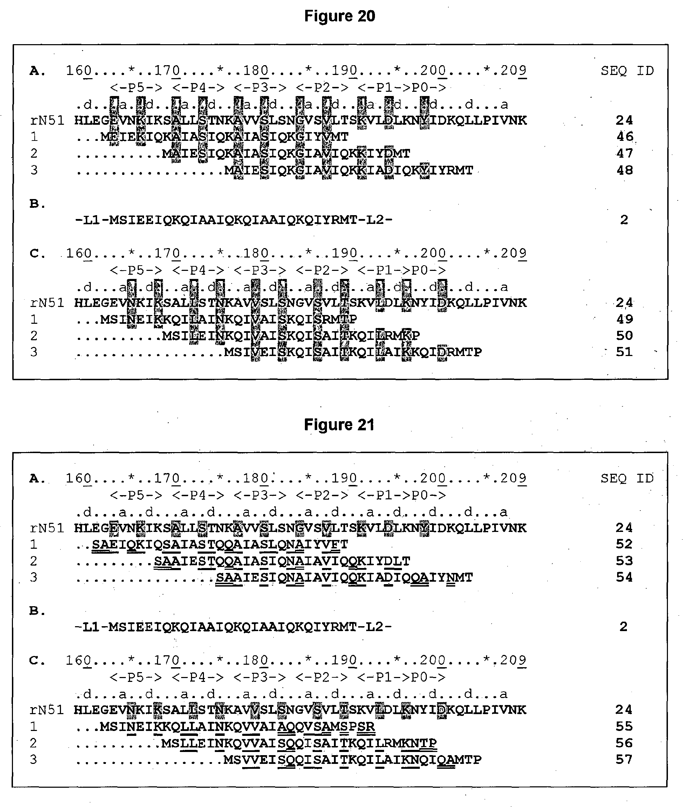

**Figure 20****:** Alignments and initial grafting of HRSV-F HR1. Alignments are shown of HRSV HR1 sequence denoted 'rN51' (residues 159 to 209 of HRSV-F, also provided as SEQ ID NO: 24) with the sequence of selected Alphabody denoted 'scAB013' in three different frames. A, selection of residues to be grafted at g- and c-positions of an Alphabody A-helix (shaded in gray) in the three possible registers. The resulting sequences labeled 1-3 are also provided as SEQ ID NO: 46, SEQ ID NO: 47 and SEQ ID NO: 48, respectively. B, amino acid sequence of the non-mutated Alphabody B-helix (SEQ ID NO: 2), supplemented with appropriate flanking linkers L1 and L2. C, selection of residues to be grafted at e- and b-positions of an Alphabody C-helix (shaded in gray) in the three possible heptad registers. The resulting sequences labeled 1-3 are also provided as SEQ ID NO: 49, SEQ ID NO: 50 and SEQ ID NO: 51, respectively.
**Figure 21****:** Structurally optimized HRSV HR1-grafted sequences. A, alignment of HR1 sequence labeled 'rN51' (as defined in Figure 20) with the amino acid sequences of the structurally optimized A-helix in the three registers that are possible for Alphabody/HR1 alignment. The resulting sequences labeled 1-3 are also provided as SEQ ID NO: 52, SEQ ID NO: 53 and SEQ ID NO: 54, respectively. B, amino acid sequence of the non-mutated Alphabody B-helix (SEQ ID NO: 2), supplemented with appropriate flanking linkers L1 and L2. C, alignment of the rN51 sequence with the amino acid sequences of the structurally optimized C-helix in the three possible alignment registers. The resulting sequences labeled 1-3 are also provided as SEQ ID NO: 55, SEQ ID NO: 56 and SEQ ID NO: 57, respectively. Residues appearing in the reference Alphabody scAB013 are not underlined. Singly underlined residues are grafted from HR1 amino acids. Doubly underlined residues are mutations selected on basis of structural considerations.

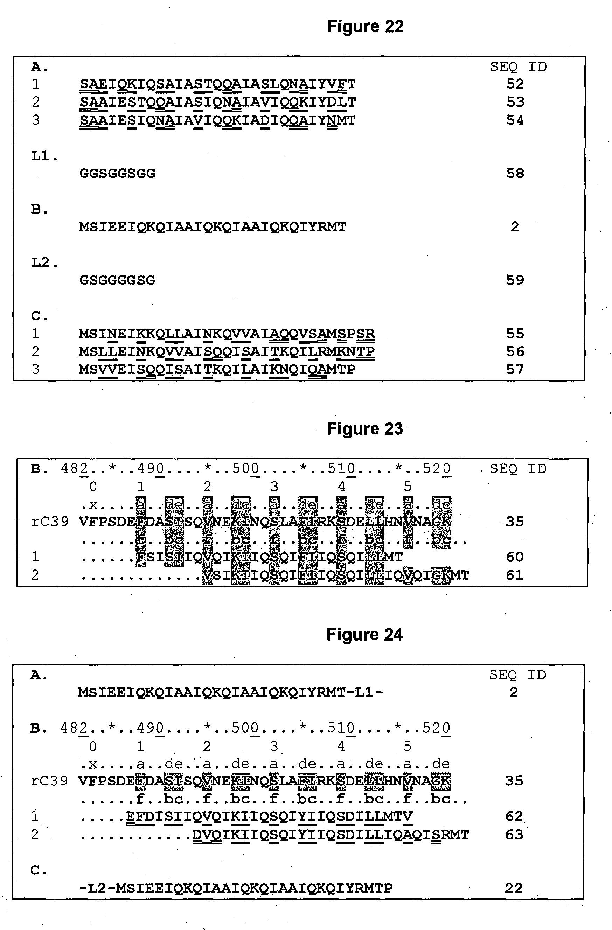

**Figure 22****:** Final Alphabody constructs with HRSV N-trimer-like binding grooves. A, 3 amino acid sequences (labels 1-3 correspond to SEQ ID NO: 52, SEQ ID NO: 53 and SEQ ID NO: 54, respectively) to be incorporated as the A-helix in an Alphabody; L1, linker 1 sequence (SEQ ID NO: 58); B, amino acid sequence (SEQ ID NO: 2) to be incorporated as the B-helix in an Alphabody, irrespective of A- and C-helix sequences; L2, linker 2 sequence (SEQ ID NO: 59); C, 3 amino acid sequences (labels 1-3 correspond to SEQ ID NO: 55, SEQ ID NO: 56 and SEQ ID NO: 57, respectively) to be incorporated as the C-helix in an Alphabody. Residues are underlined according to the same conventions as in Figure 21. The sequences are to be concatenated in an Alphabody in the order Ai-L1-B-L2-Ci, where i refers to any of the indices preceding the sequences under A and C. Thus, this figures represents three different constructs that are designed to target different sub-regions of HR2 in HRSV-F.
**Figure 23****:** Alignments and initial grafting of HRSV-F HR2. Alignments are shown of HRSV HR2 sequence denoted 'rC39' (residues 482 to 520 of HRSV-F, also provided as SEQ ID NO: 35) with the sequence of selected Alphabody denoted 'scAB013' in two different frames. B, amino acid sequence of Alphabody B-helix with grafted HR2 residues (shaded in gray) in the two possible registers. The resulting sequences labeled 1 and 2 are also provided as SEQ ID NO: 60 and SEQ ID NO: 61, respectively. The label 'B.' only serves to indicate that grafting is to be performed in the Alphabody B-helix. **Figure 24****:** Structurally optimized HRSV HR2-grafted sequences. A, amino acid sequence of the non-mutated Alphabody A-helix (SEQ ID NO: 2), supplemented with an appropriate flanking linker L1. B, alignment of the rC39 (as defined in Figure 23) with the structurally optimized B-helix in the two possible registers. The resulting sequences labeled 1 and 2 are also provided as SEQ ID NO: 62 and SEQ ID NO: 63, respectively. C, amino acid sequence of the non-mutated Alphabody C-helix (SEQ ID NO: 22), preceded with an appropriate flanking linker L2. Residues appearing in the reference Alphabody scAB013 are not underlined. Singly underlined residues are grafted from HR2 amino acids. Doubly underlined residues are mutations selected on basis of structural considerations.

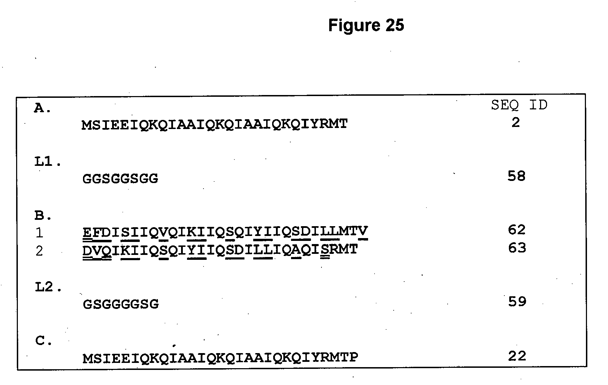

**Figure 25****:** Final Alphabody constructs with HRSV HR2-like interface residues. A, amino acid sequence (SEQ ID NO: 2) to be incorporated as the A-helix in an Alphabody, irrespective of the B-helix sequence; L1, linker 1 sequence (SEQ ID NO: 58); B, 2 amino acid sequences (labels 1 and 2 correspond to SEQ ID NO: 62 and SEQ ID NO: 63, respectively) to be incorporated as the B-helix in an Alphabody; L2, linker 2 sequence (SEQ ID NO: 59); C, amino acid sequence (SEQ ID NO: 22) to be incorporated as the C-helix in an Alphabody, irrespective of the B-helix sequence; Residues are underlined according to the same conventions as in Figure 24. The sequences are to be concatenated in an Alphabody in the order A-L1-Bi-L2-C, where i refers to any of the indices preceding the sequences under B. Thus, this figures represents two different constructs that are designed to target different sub-regions of the N-trimer in HRSV-F.

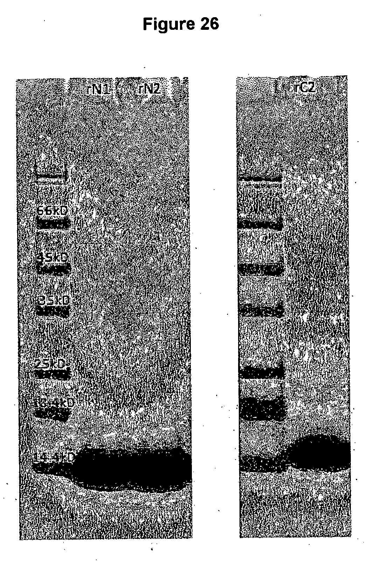

**Figure 26****:** SDS PAGE of the constructs scAB_RsvN1, scAB_RsvN2 and scAB_RsvC2 expressed in E. coli after size exclusion chromatography.

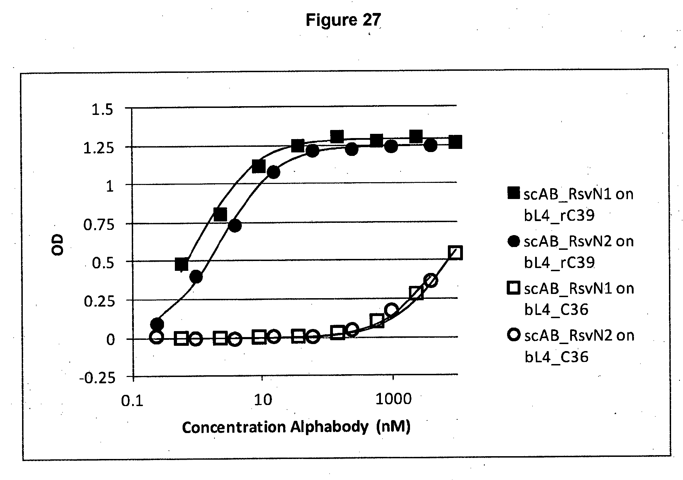

**Figure 27****:** Results of ELISA experiments on scAB_RsvN1 and scAB_RsvN2 to a HRSV-F HR2-derived biotinylated rC39 peptide and to the HIV-1 gp41 HR2-derived biotinylated control peptide C36.

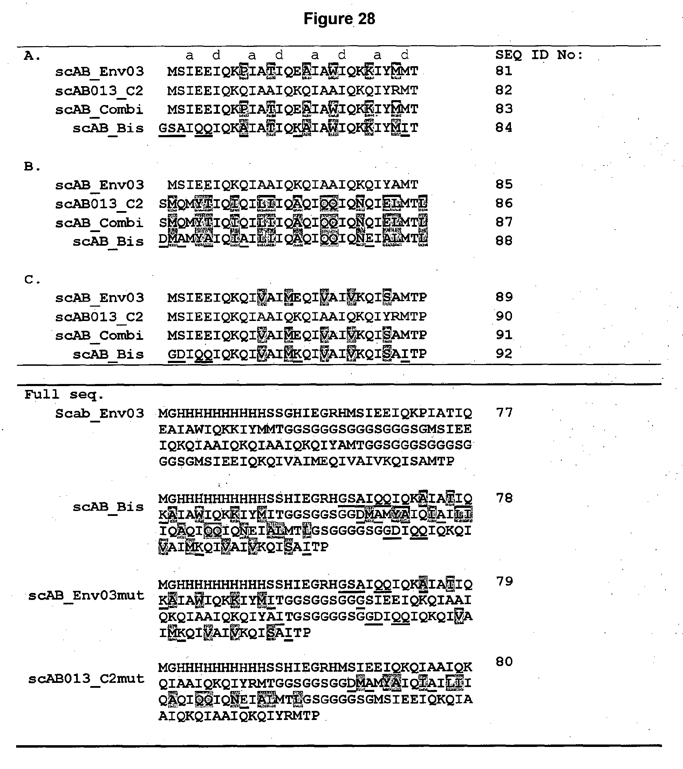

**Figure 28****:** Alignment of the A-, B- and C-helices of the starting templates scAB_Env03, scAB013_C2, the combination sequence named 'scAB_Combi', composed of the A- and C-helices of scAB_Env03 and the B-helix of scAB013_C2, and the optimized construct 'scAB_Bis', as well as the full sequences of the scAB_Env03 and scAB_Bis constructs, plus the control constructs 'scAB_Env03mut' and 'scAB013 C2mut' wherein the binding residues from either the A/C-groove or from the B-helix were 'reset' to those in the parental Alphabodies.

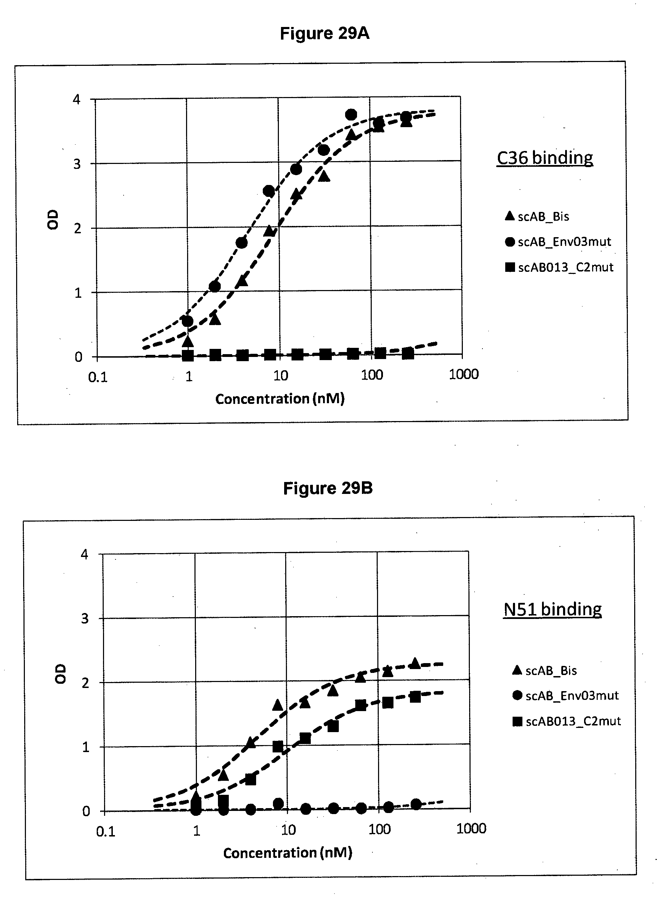

**Figure 29****:** Results of ELISA experiments on bispecific scAB_Bis and on monospecific control constructs scAb_Env03mut and scAB013_C2mut, to HIV-1 gp41-derived target peptides. Figure 29A shows the binding profiles to HR2-derived biotinylated peptide C36 and Figure 29B shows the binding profiles to HR1-derived biotinylated N51.

### EXAMPLE 1. HIV-1 gp41 N-trimer groove-grafted Alphabodies.

The aim of the present example is to demonstrate a practically feasible method to generate a gp41 N-trimer-mimicking Alphabody.

Applicants have analyzed the crystallographically determined structure of the 5-helix bundle in complex with the Fab antigen-binding domain D5 (Root et al. Science 2001, 291:884-888; PDB structure 2CMR). Figure 7, panel A, shows a schematic representation of the N-trimer part of the 5-helix bundle.

According to the authors (Root et al., ibid), the N-trimer groove residues that constitute the interface with HR2 are located at heptad e- and g-positions. However, according to our own structural analysis, at least the b- and c-residues should be taken into account as well. Therefore, when grafting gp41 groove residues onto structurally equivalent positions in an Alphabody, the set amino acid residues located at b-, c-, e- and g-positions are to be considered, unlike what is suggested in figure 4 of Root et al. (ibid), where only the e- and g-residues are depicted as interface residues.

In the legend to Figure 7, some structural aspects relating to the grafting of specific amino acid residues from the N-trimer groove onto an Alphabody are explained.

In Figure 8A, sequence alignments of Alphabody denoted 'scAB013' with HR1 sequence denoted 'N40' are provided in three different frames. scAB013 is a specific Alphabody that has been selected by the present applicants because of its high thermostability. scAB013 is defined in terms of its amino acid sequence as SEQ ID No: 64. In structural terms, the first Alphabody helix ('helix A', 'heptad repeat sequence 1') is connected to the second helix ('helix B', 'heptad repeat sequence 2') by a linker sequence ('L1') and the second Alphabody helix is connected to the third helix ('helix C', 'heptad repeat sequence 3') by a linker sequence ('L2'). This means that, irrespective of the orientation of helix B with respect to the mutually parallel helices A and C (thus irrespective of whether the Alphabody is parallel or antiparallel), helices A and C form a pair of parallel helices that are similar in structure and orientation to any pair of helices in an N-trimer.

The alignments in Figure 8A form the basis of the grafting procedure. In view of the structural similarity between Alphabody and N-trimer grooves, N-trimer positions c and g are to be grafted on an Alphabody A-helix and positions b and e are to be grafted on the C-helix. This gives rise to the initial (non-optimized) Alphabody sequences with grafted groove residues, as depicted in Figure 9.

As will be appreciated by persons skilled in the art, straightforward 'copy-pasting' of interface residues from one structure onto another will usually not lead to a full transfer of functionality; in other words, binding affinity will usually be lost or at least significantly diminished. Therefore, all amino acid residues that were grafted on a sequence basis as shown in Figure 9 were effectively placed on a 3-D model of the scAB013 Alphabody by mutating the latter with standard torsion angles. Next, each mutated residue was examined in its structural context. In case this analysis casted doubt on the structural compatibility, then alternative substitutions were considered. The latter are shown in Figure 10 as double-underlined residues. As is seen there, most of the uncertain residues were mutated into alanines which were considered generally safer.

The final, structurally optimized Alphabody constructs with grafted N-trimer-like binding grooves are shown in Figure 13. The linker sequences selected to connect helices A to B (L1) and helices B to C (L2) were chosen to be each having the 6-residue amino acid sequence 'glycine-glycine-serine-serine-glycine-glycine'. The combined sequences are provided as SEQ ID No: 65 (denoted 'scAB013_N1', Table 1), SEQ ID No: 66 ('scAB013_N2', Table 1) and SEQ ID No: 67 ('scAB013_N3', Table 1).

### EXAMPLE 2. gp41 HR2 binding site-grafted Alphabodies.

The aim of the present example is to demonstrate a practically feasible method to generate an Alphabody that mimics the HR2 surface that makes contact with an N-trimer groove in a 6-helix bundle of HIV-1 gp41 (HR2 binding site-grafted or HR2-mimicking Alphabody). Essentially the same strategy was followed as in EXAMPLE 1, with specific modifications as discussed hereinafter.

Figure 7, panel A, lower helical wheel, shows a schematic representation of an HR2 helix of HIV-1 gp41.

According to the authors, the HR2 residues that constitute the interface with an N-trimer are located at heptad a- and d-positions. However, according to our own structural analysis, at least the e-residues should be taken into account as well. Therefore, when grafting gp41 groove residues onto structurally equivalent positions in an Alphabody, the set amino acid residues located at a-, d- and e-positions are to be considered, unlike what is suggested in figure 4 of Root et al. (ibid), where only the a- and d-residues are depicted as interface residues.

In the legend to Figure 7, some structural aspects relating to the grafting of specific amino acid residues from the HR2 helix onto an Alphabody are explained.

In Figure 8B, sequence alignments of Alphabody scAB013 (SEQ ID No: 64) with HR2 sequence denoted 'C-38' are provided in two different frames. Special in this case is that heptad positions a, d and e in HR2 are to be grafted on maximally exposed positions in an Alphabody so as to be fully accessible for gp41 N-trimer binding. The Alphabody positions chosen for this purpose are b-, c- and f-positions, with the mapping as explained in the legend to Figure 7. This mapping was used in the alignments shown in Figure 8B.

With respect to the type of Alphabody, it does not make an essential difference whether the latter is parallel or antiparallel, because the only aim is to make the Alphabody bind to a gp41 N-trimer groove through a single alpha-helix. Which helix (A, B or C) is chosen is in principle also not relevant, but in view of one of the embodiments of the present invention, i.e., to develop bifunctional Alphabodies, the most optimal choice is the B-helix.

The alignments in Figure 8B form the basis of the grafting procedure. There, all HR2 a-residues are transferred to Alphabody f-positions, and HR2 d- and e-residues are transferred to Alphabody b- and c-positions, respectively. This gives rise to the initial (non-optimized) Alphabody sequences with grafted HR2 residues, as depicted in Figure 11.

As in EXAMPLE 1, all amino acid residues that were grafted on a sequence basis as shown in Figure 11 were effectively placed on a 3-D model of the scAB013 Alphabody by mutating the latter with standard torsion angles. Next, each mutated residue was examined in its structural context. In case this analysis casted doubt on the structural compatibility, then alternative substitutions were considered. The latter are shown in Figure 12 as double-underlined residues. Unlike in EXAMPLE 1, most uncertain residues were this time not mutated into alanines, but into isosteric or slightly larger residue types to compensate for the helical bending which is towards the center of an Alphabody, whereas the bending should be opposite for ideal binding to a gp41 N-trimer.

The final, structurally optimized Alphabody constructs with a grafted HR2-like surface are shown in Figure 14. The linker sequences selected to connect the Alphabody helices were again chosen to have the 6-residue amino acid sequence 'glycine-glycine-serine-serine-glycine-glycine'. The combined sequences are provided as SEQ ID No: 68 (denoted 'scAB013_C1', Table 1) and SEQ ID No: 69 ('scAB013_C2', Table 1). The glycine-glycine-serine-serine-glycine-glycine sequence is provided as SEQ ID NO: 23.

### EXAMPLE 3. Soluble expression, binding and antiviral activity of scAB013 C2.

The aim of the present example is to demonstrate that a gp41 HR2-mimicking Alphabody can be solubly expressed and purified from E. coli, that it has a high in vitro binding affinity for its cognate target region, and that it is active in a standard antiviral assay.

A synthetic gene for scAB013_C2 (SEQ ID No: 69) was purchased (GeneArt). This coding sequence was subcloned into the pET22b vector (Novagen). Using this vector, a (His)6 tag, preceded by leucine and glutamic acid, is added at the C-terminus of the scAB013_C2 sequence. The resulting construct was transformed into the host E. coli strain BL21(DE3) harboring a chromosomal copy of the T7 polymerase gene under control of the lacUV5 promoter (DE3 lysogen). Transformed cells were grown in medium supplemented with ampicillin and protein expression was induced by the addition of IPTG to exponentially growing cultures. Cells containing the expressed Alphabodies were collected by centrifugation and the pellets were resuspended in 50 mM Tris, 500 mM NaCl, pH 7.8. Cells were then disrupted by sonication and spun down for cell debris removal. The cleared supernatants were applied onto a HITrap IMAC HP column (GE Healthcare) loaded with Ni²⁺ ions. Bound proteins were eluted by applying an imidazole gradient from 5 to 1000 mM. Alphabody-containing fractions were pooled, concentrated and loaded on a Superdex 75 size exclusion chromatography (SEC) column (GE Healthcare). During this final purification step, the buffer was changed to 50 mM Tris, 150 mM NaCl, pH 7.8. A CD thermal denaturation scan at 222 nm of 11 microM scAB013_C2 in 5 M GuHCl showed a melting curve from which a transition temperature Tm of 72 °C was derived, indicating that scAB013_C2 is extremely stable (i.e., resistant to chemical and thermal denaturation).

The binding affinity of scAB013_C2 for biotinylated N51 was analyzed by surface plasmon resonance (SPR). HIV-1 N51 corresponds to residues 540 to 590 of the HIV-1 HXB2 sequence. The biotinylated N51 sequence (provided herein as SEQ ID No: 70, Table 1) was N-terminally preceded by a biotin moiety and a glycine-glycine-serine-glycine spacer fragment. Biotinylated N51 was immobilized at 5 microM for 20 min at a flow rate of 5 microliter per min on a streptavidin-coated biosensor chip in a running buffer [0.01 M HEPES pH 7.4, 0.15 M NaCl, 3 mM EDTA, 0.005% (v/v) surfactant P20 (HBS-EP)]. Each sensorgram (binding-dissociation curve) was recorded at a flow rate of 30 microliter per min, with the following steps: 3 min association time, followed by 20 min dissociation time, followed by two 18 sec regeneration pulses using 0.05% SDS, followed by 2 min stabilization time. Two-fold dilutions of scAB013_C2, starting at 4000 nM and with 7.8 nM as lowest concentration, were tested by SPR in duplicate. The resulting sensorgrams, corrected on the basis of an irrelevant control flow cell and also by subtracting the 0 nM curves, are shown in Figure 15. The results were kinetically analyzed in accordance with a 1:1 Langmuir binding model using BIAcore software (BIAEVALUATION 4.1). The kinetic parameters derived from this experiment are: on-rate constant kOn = 1.01 × 10⁵ M⁻¹s⁻¹ and dissociation rate constant kOff = 2.09 × 10⁻⁴ s⁻¹; hence, the overall affinity constant KD = kOff/kOn = 2.07 × 10⁻⁹ M or about 2 nM.

The HIV inhibitory capacity of wild type scAB013_C2 was analyzed in a 5 day infection assay using a cell line (MT4-X4) displaying CD4 and CXCR4 receptors. The results are shown in Figure 16. The virus used in this assay was the laboratory adapted reference strain HXB2 virus using CXCR4 as co-receptor. Cells were infected with 100 TCID50/ml of virus in the presence of three-fold dilutions of Alphabody starting at 2.5 microM. Inhibition of HIV infection by scAB013_C2 was evaluated by monitoring the cell survival using MTT (3-(4,5-Dimethylthiazol-2-Yl)-2,5-Diphenyltetrazolium Bromide). MTT is reduced to formazan by living cells. Solubilization of the formazan crystals results in a colored product that can be measured by spectrophotometry at 540 nm. The cellular toxicity of the Alphabodies was monitored in the same assay using the same read-out, i.e. cell survival. As controls, the clinically approved T20 peptide (Fuzeon®, Roche) was used, as well as the CXCR4 antagonist, AMD3100 (MozobileTM, Genzyme). The 50% inhibition concentration (IC50) derived from this experiment was 62 nM, compared to 54 nM for AMD3100 and 6 nM for T-20.

The HIV inhibitory capacity of wild type scAB013_C2 was also analyzed in a 7 day infection assay using IL-2 and phycohemagglutinin activated peripheral blood mononuclear cells (PBMC) isolated from a healthy donor and a laboratory adapted HIV strain (HXB2). The results are shown in Figure 17. PBMC were infected with 4000 TCID50/ml of virus and added to serial dilutions of scAB_C2 starting at 2 microM. After 7 days, the amplification of the virus in absence and presence of scAB_C2 was monitored by measuring the viral p24 concentration. The maximal p24 concentration was measured when PBMC and virus were incubated without inhibitory molecules and was set at 100% viral amplification. As controls, the clinically approved T20 (Fuzeon®, Roche) was used, as well as the CXCR4 antagonist, AMD3100 (MozobileTM, Genzyme). The toxicity of scAB013_C2 on PBMC was also measured by monitoring the cell viability using the MTT read-out. The 50% inhibition concentration (IC50) derived from this experiment was 218 nM, compared to 97 nM for AMD3100 and 60 nM for T-20.

EXAMPLE 4. Alphabodies engrafted with HRSV-F HR1 and HR2 binding site residues.

The aim of the present example is to demonstrate a practically feasible method to generate Alphabodies forming structural mimics of membrane fusion-driving subregions HR1 and HR2 from human respiratory syncytial virus (HRSV) fusion protein (HRSV-F).

Applicants have analyzed the crystallographically determined structure of the HRSV postfusion 6-helix bundle (HRSV F1 heptad repeat structure, PDB entry code 1 G2C). The HRSV HR1 and HR2 sequences were used as input sequences in a BLAST search in order to retrieve additional homologous sequences. Figure 18 shows a sequence alignment of 6-helix bundle fragments from 11 different paramyxoviridae. HRSV, TRT and PVM belong to the subfamily of pneumovirinae and the 8 others are paramyxovirinae. It was found that the HR1 sequences all show regular heptad repeats, although they are not all of the same length (Henipaviruses Nipah and Hendra are shorter). A proline near the end of most HR1 sequences causes an irregularity with the effect of an insertion, which is reflected by three consecutive d-positions (Figure 18A). A strong conservation of small residues (glycine, alanine, serine) at HR1 g-positions was observed (Figure 18A, underlined residues). These positions are directly covered in the structure by HR2 d-residues which point in lateral direction. The most conserved HR2 residues are at the e-positions: there is an extreme conservation of aliphatic residues isoleucine and leucine (Figure 18B), suggesting that these positions are very important for the interaction HR2-HR1. The a-positions near the middle of HR2 are often small amino acids such as alanine or serine (anchors 3 and 4 in Figure 18B). In the PDB structure 1G2C, they point straight to the center of the HR1 N-trimer (i.e., they pack in a true knobs-into-holes fashion). In contrast, anchor residues at a-positions near the ends of HR2 are more bulky (phenylalanine at anchor 1 in pneumovirinae and mostly isoleucine in paramyxovirinae; mostly valine at anchor 5 in all paramyxoviridae). Taken together, the sequence comparison suggests that HR2 positions a and e (and to a much lesser extent also d) are mandatory for strong HR1-HR2 interaction. The structure further showed that the pockets in the HR1 N-trimer are primarily formed by e- and g-residues, with occasional contributions from b- and c-residues, and with the highest degree of conservation observed near the termini. It is also remarked that, in contrast to Alphabodies where at least 50% of the core residues are isoleucines, only very few of the coiled coil (N-trimer) core a- and d-residues are isoleucines; since these residues form the bottom of each pocket, this observation renders the following grafting procedure not obvious.

In the legend to Figure 19, some structural aspects relating to the grafting of specific amino acid residues from the HRSV N-trimer groove onto an Alphabody groove, and of HRSV HR2 residues onto an Alphabody B-helix are explained.

In Figure 20, sequence alignments of Alphabody denoted 'scAB013' (SEQ ID No: 64) with HR1 sequence denoted 'rN51' (SEQ ID No: 24) are provided in three different frames. These alignments form the basis of the grafting procedure of HR1 groove residues onto an Alphabody. Concretely, N-trimer positions c and g were transferred (grafted) onto an Alphabody A-helix and positions b and e were grafted onto the C-helix. This gives rise to the initial (non-optimized) Alphabody sequences with grafted groove residues, as depicted in the figure.

Next, all amino acid residues that were grafted on a sequence basis as shown in Figure 20 were effectively placed on a 3-D model of the scAB013 Alphabody by mutating the latter with standard torsion angles. Each mutated residue was examined in its structural context. In case this analysis casted doubt on the structural compatibility, then alternative substitutions were considered. The latter are shown in Figure 21 as double-underlined residues.

The final, structurally optimized Alphabody constructs with grafted HRSV N-trimer-like binding grooves are shown in Figure 22. The linker fragment sequences selected to connect helices A to B (L1) and helices B to C (L2) were chosen to have the 8-residue amino acid sequences 'glycine-glycine-serine-glycine-glycine-serine-glycine-glycine' and 'glycine-serine-glycine-glycine-glycine-glycine-serine-glycine', respectively. The combined sequences are provided as SEQ ID No: 71 (denoted 'scAB_RsvN1', Table 1), SEQ ID No: 72 ('scAB_RsvN2', Table 1) and SEQ ID No: 73 ('scAB_RsvN3', Table 1).

In Figure 23, sequence alignments of Alphabody denoted 'scAB013' (SEQ ID No: 64; Table 1) with HR2 sequence denoted 'rC39' (SEQ ID No: 35) are provided in two different frames. These alignments form the basis of the grafting procedure of HR2 groove-binding residues onto an Alphabody. Concretely, HR2 positions a, d and e were transferred (grafted) onto positions f, b and c, respectively, in an Alphabody helix. This gives rise to the initial (non-optimized) Alphabody sequences with grafted HR2 residues, as depicted in the figure.

Next, all amino acid residues that were grafted on a sequence basis as shown in Figure 23 were effectively placed on a 3-D model of the scAB013 Alphabody by mutating the corresponding Alphabody B-helix residues with standard torsion angles. Each mutated residue was examined in its structural context. In case this analysis casted doubt on the structural compatibility, then alternative substitutions were considered. The latter are shown in Figure 24 as double-underlined residues.

The final, structurally optimized Alphabody constructs with grafted HRSV HR2-like B-helix are shown in Figure 25. The linker fragment sequences selected to connect helices A to B (L1) and helices B to C (L2) were chosen to have the 8-residue amino acid sequences 'glycine-glycine-serine-glycine-glycine-serine-glycine-glycine' and 'glycine-serine-glycine-glycine-glycine-glycine-serine-glycine', respectively. The combined sequences are provided as SEQ ID No: 74 (denoted 'scAB_RsvC1', Table 1) and SEQ ID No: 75 ('scAB_RsvC2', Table 1).

**Table 1. Amino acid sequences of Single-chain Alphabody sequences**

| Alphabody 'scAB013' sequence |
|---|
| MSIEEIQKQIAAIQKQIAAIQKQIYRMTGGSGGGSGGGSGGGSGMSIEEIQKQIAAIQKQ IAAIQKQIYRMTGGSGGGSGGGSGGGSGMSIEEIQKQIAAIQKQIAAIQKQIYRMTP (SEQ ID NO:64) |

| Alphabody 'scAB013_N1' sequence |
|---|
| ALISGIQQQIANLQKAIAAQQHLIYLMTGGSSGGMSIEEIQKQIAAIQKQIAAIQKQIYR MTGGSSGGMSLSAIVKQQNAILKQIQAIQKQLQRMVA (SEQ ID NO: 65) |

| Alphabody 'scAB013_N2' sequence |
|---|
| QQIANLQKAIAAQQHLIALIQWGIYRMTGGSSGGMSIEEIQKQIAAIQKQIAAIQKQIYR MTGGSSGGMSQNAILKQIQAIQKQLQAIVKQIKAMQA (SEQ ID NO: 66) |

| Alphabody 'scAB013_N3' sequence |
|---|
| AAIAAQQHLIALIQWGIAQLQARIYAMTGGSSGGMSIEEIQKQIAAIQKQIAAIQKQIYR MTGGSSGGMSIQAIQKQLQAIVKQIKAIQKQILAMEA (SEQ ID NO: 67) |

| Alphabody 'scAB013_C1' sequence |
|---|
| MSIEEIQKQIAAIQKQIAAIQKQIYRMTGGSSGGTWQMWEIQIQIYTIQIQILIIQAQIQ QWKQGGSSGGMSIEEIQKQIAAIQKQIAAIQKQIYRMTP (SEQ ID No: 68) |

| Alphabody 'scAB013_C2' sequence |
|---|
| MSIEEIQKQIAAIQKQIAAIQKQIYRMTGGSSGGSMQMYTIQIQILIIQAQIQQIQNQIE LMTLGGSSGGMSIEEIQKQIAAIQKQIAAIQKQIYRMTP (SEQ ID No: 69) |

| Biotinylated N51 sequence |
|---|
| biotin-GGSGQARQLLSGIVQQQNNLLRAIEAQQHLLQLTVWGIKQLQARILAVERYLKDQ **(SEQ ID No: 70)** |

| Alphabody 'scAB_RsvN1' sequence |
|---|
| SAEIQKIQSAIASTQQAIASLQNAIYVFTGGSGGSGGMSIEEIQKQIAAIQKQIAAIQKQ IYRMTGSGGGGSGMSINEIKKQLLAINKQVVAIAQQVSAMSPSR (SEQ ID No: 71) |

| Alphabody 'scAB_RsvN2' sequence |
|---|
| SAAIESTQQAIASIQNAIAVIQQKIYDLTGGSGGSGGMSIEEIQKQIAAIQKQIAAIQKQ IYRMTGSGGGGSGMSLLEINKQVVAISQQISAITKQILRMKNTP (SEQ ID No: 72) |

| Alphabody'scAB_RsvN3' sequence |
|---|
| SAAIESIQNAIAVIQQKIADIQQAIYNMTGGSGGSGGMSIEEIQKQIAAIQKQIAAIQKQ IYRMTGSGGGGSGMSVVEISQQISAITKQILAIKNQIQAMTP (SEQ ID No: 73) |

| Alphabody 'scAB_RsvC1' sequence |
|---|
| MSIEEIQKQIAAIQKQIAAIQKQIYRMTGGSGGSGGEFDISIIQVQIKIIQSQIYIIQSD ILLMTVGSGGGGSGMSIEEIQKQIAAIQKQIAAIQKQIYRMTP (SEQ ID No: 74) |

| Alphabody 'scAB_RsvC2' sequence |
|---|
| MSIEEIQKQIAAIQKQIAAIQKQIYRMTGGSGGSGGDVQIKIIQSQIYIIQSDILLIQAQ ISRMTGSGGGGSGMSIEEIQKQIAAIQKQIAAIQKQIYRMTP (SEQ ID No: 75) |

### EXAMPLE 5. Soluble expression and binding of Alphabodies with grafted HRSV N-trimer-like binding grooves

All five constructs scAB_RsvN1, -N2, -N3 and scAB_RsvC1 and -C2 were produced in soluble form in E. coli according to the same protocol as in EXAMPLE 3. However, for these constructs, subcloning of synthetic genes was performed in the pET16b vector (Novagen), instead of in pET22b, in order to have an N-terminal 10-histidine tag. The expression levels of constructs scAB_RsvN3 and scAB_Rsv_C1 were too low to obtain sufficient amounts of purified product to continue with. The remaining three constructs scAB_RsvN1, scAB_RsvN2 and scAB_RsvC2 were at least 95% pure after final size exclusion chromatography, as estimated from SDS PAGE, shown in Figure 26. None of the three Alphabodies showed tendency to aggregate (based on visual inspection, OD measurements at 340 nm, size exclusion chromatography profiles and SDS-PAGE) at the concentration ranges tested (typically, up to at least 1 µM).

Figure 27 shows the results of ELISA experiments on scAB_RsvN1 and scAB_RsvN2, wherein binding was analyzed to a HRSV-F HR2-derived target peptide. This target sequence (target peptide) was a derivative of the rC39 sequence (SEQ ID No:35), which was N-terminally biotinylated and C-terminally amidated and wherein the N-terminal biotin group was attached to the sequence through a 4-residue Gly/Ser linker. The full target sequence has the amino acid sequence of SEQ ID No: 76, written in single-letter notation as 'biotin-GSGS-VFPSDEFDASISQVNEKINQSLAFIRKSDELLHNVNAGK-NH2', and herein also referred to as 'bL4_rC39' and to the HIV-1 gp41 HR2-derived biotinylated control peptide C36. Neutravidin (10 ug/mol, 100 ul/well) was immobilized on Maxisorp (Nunc) microtiterplates overnight at 4 °C. After the incubation, plates were washed 3 x with PBS containing 0.05% Tween 20 (PBST) and blocked with 1% BSA in PBS (200 ul/well) for 1 hr at R.T. Then, biotinylated target peptides (100 nM, 100 ul/well) were immobilized for 1 hr at R.T. Plates were washed 3 x with PBST and skimmed milk-blocked (200 ul of a 2 % skimmed milk in PBS solution incubated for 1 hr at 37 °C). After washing the plates 4 x with PBST, two-fold dilution series of Alphabodies in PBS containing 0.1% skimmed milk were added and incubated for 2 hrs at R.T. Plates were washed 4 x with PBST and the detection of the Alphabody binding was performed using 100 ul of 1/2000 anti-His antibody conjugated to HRP (Sigma) in PBS with 2% skimmed milk, incubated for 1 hr at R.T. After incubation, plates were washed 5 x with PBST and developed with ortho-phenylenediamine (OPD, Sigma) and stopped with 4 M H₂SO₄**.** Plates were read at 492 nm and 630 nm. The obtained optical density (OD) data were plotted as a function of Alphabody concentration (Figure 27). A sigmoid curve (dashed lines) was fitted through the data points to obtain apparent binding constants.

The data shown in Figure 27 prove that both of the Alphabodies scAB_RsvN1 and -N2 bind with high affinity (apparent K_{D} < 10 nM) to immobilized rC39 target peptide. The apparent binding constant of scAB_RsvN1 was determined at 1.1 nM. The apparent binding constant of scAB_RsvN2 was determined at 2.3 nM. The affinities for the HIV-1 gp41-derived control peptide C36 were found to be in the micromolar range (> 5000 nM for both constructs), indicating that the recognition of RSV-F HR2-derived target peptide is relatively specific.

### EXAMPLE 6. Construction of a bifunctional Alphabody

The aim of the present example is to demonstrate the introduction through rational design of a second binding site into an Alphabody already comprising a first binding site. Thus the present example describes the provision of a bifunctional (bispecific) single-chain Alphabody i.e., an Alphabody that comprises two functional binding regions within the same molecule (such as illustrated in Alphabody structures shown in Figures 7D and 19), wherein these two regions have a distinct target binding specificity.

The two functional binding regions of the Alphabody of the present example are both alpha-helical binding regions, i.e., the binding surfaces are predominantly constituted of alpha-helical parts of the Alphabody. The binding surfaces in the present example are also non-overlapping (segregated in space), so that under appropriate conditions two target molecules can be simultaneously bound by one Alphabody. Further, a first binding region mimics part of an HIV-1 gp41 HR2 surface and is able to bind a gp41 N-trimer groove, and a second binding region mimics part of an HIV-1 gp41 N-trimer groove and is able to bind a gp41 HR2 surface.

In the present example it was opted to introduce an additional binding site into Alphabody scAB_Env03 displaying an N-trimer groove-like binding site. The sequence of scAB_Env03 is shown in Figure 28 and herein provided as SEQ ID No: 77. This Alphabody was derived from a generic phage-displayed library of Alphabodies comprising random sequence variegation at heptad c- and g-positions in the A-helix and at heptad b- and e-positions in the C-helix. This Alphabody is of the type 'groove binder', meaning that the binding residues of scAB_Env03 together form a binding groove in between two parallel alpha-helices A and C. Although there is virtually no sequence identity between the residues of the scAB013_Env03 binding groove and those of the HIV-1 gp41 N-trimer binding groove, the Alphabody forms a structural mimic of the gp41 N-trimer binding region because both entities are alpha-helical coiled coils with highly similar alpha-helix orientation and 'knobs' and 'holes' positions. At the same time, the almost complete absence of sequence correspondence reduces the risk of self association mediated by the engrafted groove-binding region.

Having selected scAB_Env03 as the starting point for a bifunctional Alphabody, the earlier selected binding residues from scAB013_C2 (EXAMPLE 2) were grafted onto its B-helix. Figure 28 illustrates the procedure followed. The A-, B- and C-helices of the starting templates scAB_Env03 and scAB013_C2 were first aligned separately. Then, a combination sequence named 'scAB_Combi', composed of the A- and C-helices of scAB_Env03 and the B-helix of scAB013_C2, was considered. Next, all amino acid residues that were specific to scAB_Env03 were effectively placed on the 3-D model of scAB013_C2 of EXAMPLE 2. This allowed to evaluate the structural features of the scAB_Combi sequence of Figure 28. The latter evaluation showed several opportunities for further optimization (underlined residues in the sequences of Figure 28). Most optimizations were based on electrostatic considerations, improved N-terminal alpha-helix capping, or mutations to enhance the helical propensity of the B-helix without affecting binding. Concretely, the following optimizations were introduced in the designed construct named 'scAB_Bis': (i) an N-terminal 'GSA' motif (glycine-serine-alanine) was introduced instead of 'MS' (methionine-serine) to avoid that the capping methionine blocks the binding groove, (ii) the N-terminal glutamates of the A- and C-helices were substituted into glutamines to avoid electrostatic repulsion with the acidic HR2 sequence, (iii) proline at position g of the first heptad of the A-helix was mutated into alanine to increase the helical stability of the A-helix, (iv) the glutamates at the f-position in the second heptad of the A- and C-helices were mutated into lysine for electrostatic reasons similar to the mutation of N-terminal glutamates, (v) isoleucines were placed at the d-position of the fourth heptad of the A- and C-helices instead of methionines to improve core residue packing stability, (vi) aspartic acid was chosen at the N-terminal position of the B-helix to strengthen the capping as well as electrostatic interaction with the gp41 N-trimer, (vii) the first and third glutamines and the first threonine of the B-helix were mutated into alanine to enhance the helical propensity with minimal risk to affect binding, (viii) the last glutamine of the B-helix was mutated into glutamate for electrostatic reasons, and (ix) the N-terminus of the C-helix was mutated into GD (glycine-aspartic acid) instead of MS (methionine-serine) to strengthen the capping of the C-helix and to compensate for the absence of N-terminal glutamates. Next, the single-chain scAB_Bis sequence was completed by two additional features: (i) flexible linker sequences L1 and L2 were chosen: here it was opted to use 8-residue glycine/serine linkers, and (ii) an N-terminal His-tag was chosen. The full amino acid sequence of scAB_Bis is also shown in Figure 28 and is herein referred to as SEQ ID No: 78.

In addition to the scAB_Bis construct, two control constructs were designed in order to be able to assess the effect of the multiple substitutions compared to the parental constructs scAB_Env03 and scAB013_C2. These control constructs are basically the scAB_Bis construct wherein the binding residues from either the A/C-groove or from the B-helix were 'reset' to those in the parental Alphabodies. These constructs can therefore also be seen as the monofunctional variants of scAB_Bis. The first control construct named 'scAB_Env03mut' (SEQ ID No: 79) comprised all A- and C-helix mutations shown in Figure 28 but not those of the B-helix (except the methionine to glycine substitution at the N-terminus and methionine to isoleucine substitution at the last core position, both being selected for reasons of consistency between the three helices, and both lying away from the HR2-binding site). Analogously, the construct named 'scAB013_C2mut' (SEQ ID No: 80) comprised all B-helix mutations but not those of the A- and C-helices.

Constructs scAB_Bis, scAb_Env03mut and scAB013_C2mut were produced in soluble form in E. coli according to the same protocol as in EXAMPLE 3. However, for these constructs, subcloning of synthetic genes was performed in the pET16b vector (Novagen), instead of in pET22b, in order to have an N-terminal 10-histidine tag. All three constructs were at least 95% pure after final size exclusion chromatography, as estimated from SDS PAGE (data not shown). None of the three Alphabodies showed tendency to aggregate (based on visual inspection, OD measurements at 340 nm, size exclusion chromatography profiles and SDS-PAGE) at the concentration ranges tested (typically, up to 1 µM).

Figure 29 shows the results of ELISA experiments on bispecific scAB_Bis and on monospecific control constructs scAb_Env03mut and scAB013_C2mut, wherein binding was analyzed to HIV-1 gp41-derived target peptides.

The target sequence (target peptide) of the gp41 HR2 described in the present example was a derivative of a peptide corresponding to residues 628 to 661 of the HIV-1 HXB2 Env that was N-terminally biotinylated and C-terminally amidated and wherein the N-terminal biotin group was attached to the sequence through a 4-residue Gly/Ser linker. The full target sequence has the amino acid sequence of SEQ ID No: 93, written in single-letter notation as 'biotin GSGSWMEWDREINNYTSLIHSLIEESQNQQEKNEQELLEL-NH2', and herein also referred to as 'bL4_C36'. The target peptide of the gp41 HR1 region used was N51 (provided herein as SEQ ID No: 70, Table 1)

Figure 29A shows the binding profiles to HR2-derived biotinylated peptide C36 and Figure 29B shows the binding profiles to HR1-derived biotinylated N51. Streptavidin (10 ug/mol, 100 ul/well) was immobilized on Maxisorp (Nunc) microtiterplates overnight at 4 °C. After the incubation, plates were washed 3 x with PBS containing 0.05% Tween 20 (PBST) and blocked with 1% BSA in PBS (200 ul/well) for 1 hr at 37 °C. Then, biotinylated target peptides (250 nM, 100 ul/well) were immobilized for 1 hr at R.T. The N51 peptide was pre-treated by dissolving 0.2 mg in 400 µl PBS, followed by 10 min. heating at 70 °C and dilution to final concentration. Plates were washed 3 x with PBST and skimmed milk-blocked (200 ul of a 2 % skimmed milk in PBS solution incubated for 1 hr at 37 °C). After washing the plates 4 x with PBST, two-fold dilution series of Alphabodies in PBS containing 0.1% skimmed milk were added and incubated for 2 hrs at R.T. Plates were washed 4 x with PBST and the detection of the Alphabody binding was performed using 100 ul of 1/2000 anti-His antibody conjugated to HRP (Sigma) in PBS with 2% skimmed milk, incubated for 1 hr at R.T. After incubation, plates were washed 5 x with PBST and developed with ortho-phenylenediamine (OPD, Sigma) and stopped with 4 M H₂SO₄. Plates were read at 492 nm and 630 nm. The obtained optical density (OD) data were plotted as a function of Alphabody concentration (Figure 29). A sigmoid curve (dashed lines) was fitted through the data points to obtain apparent binding constants (K_{D} values in Table 2).

The data in Figure 29 and Table 2 provided the following results. It was found that the monofunctional Alphabody scAB_Env03mut binds with high affinity (apparent K_{D} < 10 nM) to the gp41 C36 target peptide. Unexpectedly, the apparent K_{D} of 4.6 nM was more than an order of magnitude lower (better) than that of the parental scAB_Env03 Alphabody. The apparent K_{D} of 10.4 nM for scAB013_C2mut binding to N51 also showed an unexpected significant improvement over that of the parental Alphabody scAB013_C2 (K_{D} = 18 nM by ELISA). For both of these Alphabodies, the control experiment on the C36 peptide showed complete absence of binding (K_{D} > 10000 nM), proving their high specificity for an HIV-1 gp41 N-trimer-derived target region.

The scAB_Bis Alphabody was found to recognize both C36 and N51 with comparable affinities in the single-digit nanomolar range (Figure 29 and Table 2), thereby clearly demonstrating its bifunctional (in the present case, bispecific) character. The apparent binding constant for the C36 peptide was 8.7 nM, a value less than a factor 2 higher than that of the monofunctional scAB_Env03mut Alphabody. Interestingly, the apparent binding constant of scAB_Bis for N51 was found to be 4.9 nM, which is about a factor 2 better than that of the monofunctional scAB013_C2mut.

In conclusion, the results indicate that a) different binding sites with distinct binding specificities can be combined in the same single-chain Alphabody, resulting in a single-domain bispecific construct and b) that rational based design can be combined with random-based screening to obtain optimal bi-functional antibodies. The results also show that such construct (scAB_Bis) can be made which stably folds and which does not aggregate through association of the engineered groove surface (formed by the A- and C-helices) and engineered helix surface (displayed at the B-helix). This result is to be considered non-trivial because the two functional binding sites form a structural mimic of, on the one hand, the coiled coil structure of the HIV-1 gp41 N-trimer groove and, on the other hand, the alpha-helical structure of the HIV-1 gp41 HR2 binding region, both of which are known to be tightly associated in the post-fusion 6-helix bundle state. Finally, the results indicate that the said two distinct binding sites can be combined with preservation of affinity compared to monofunctional variants.

**Table 2**

| Construct | Reference | K_{D} C36 binding (nM) | K_{D} N51 binding (nM) |
|---|---|---|---|
| scAB_Bis | SEQ ID No: 78 | 8.7 | 4.9 |
| scAB_Env03mut | SEQ ID No: 79 | 4.6 | >10000 |
| scAB013_C2mut | SEQ ID No: 80 | >10000 | 10.4 |
| scAB_Env03 | SEQ ID No: 77 | 211 | (N.D.) |
| scAB013_C2 | SEQ ID No: 69 | >10000 | 18.0 |

## Claims

1. A single-chain Alphabody, having the general formula HRS1-L1-HRS2-L2-HRS3, wherein each of HRS1, HRS2 and HRS3 is independently a heptad repeat sequence (HRS) consisting of 2 to 7 consecutive heptad repeat units, at least 50% of all heptad a- and d-positions are occupied by isoleucine residues, each HRS starts and ends with an aliphatic or aromatic amino acid residue located at either a heptad a- or d-position, and HRS1, HRS2 and HRS3 together form a triple-stranded, alpha-helical, coiled coil structure; and
each of L1 and L2 is independently a linker fragment, covalently connecting HRS1 to HRS2 and HRS2 to HRS3, respectively, and consisting of at least 4 amino acid residues, preferably at least 50% of which are selected from the group proline, glycine, serine,
**characterized in that** said Alphabody comprises an alpha-helical binding region which mediates binding to a first fusion-driving region of a class-I viral fusion protein and which structurally mimics a second fusion-driving region of said class-I viral fusion protein, wherein said first and second fusion-driving regions of said class-I viral fusion protein are regions which interact to drive the fusion between a virus displaying said class-I viral fusion protein and a target cell, wherein said class-I viral fusion protein is not the gp41 subunit of HIV-1 envelope glycoprotein.

2. The single-chain Alphabody according to claim 1, wherein said fusion-driving regions are chosen from the group consisting of 'heptad repeat 1' ('HR1'), 'N-terminal heptad repeat' ('HRN' or 'HR-N'), 'N-trimer region' ('N-trimer'), 'N-peptide region', 'coiled coil region', 'heptad repeat 2' ('HR2'), 'C-terminal heptad repeat' ('HRC' or 'HR-C') and 'C-peptide region'.

3. The single-chain Alphabody according to claim 1 or 2, wherein said alpha-helical binding region forms a structural mimic of the secondary structure of said second fusion-driving region.

4. The single-chain Alphabody according to claim 3, wherein said alpha-helical binding region is located at a solvent-oriented surface of one of the Alphabody alpha-helices, and wherein said alpha-helical binding region includes at least 9 amino acid residues located at heptad b-, c- and f-positions.

5. The single-chain Alphabody according to claim 4, wherein at least 5 of said 9 amino acid residues located at said heptad b-, c- and f-positions are identical to amino acid residues appearing at structurally equivalent positions in said second fusion-driving region.

6. The single-chain Alphabody according to claim 3, wherein said alpha-helical binding region is located at the groove formed by two adjacent alpha-helices of the Alphabody, and wherein said alpha-helical binding region includes at least 10 amino acid residues located at heptad b- and e-positions in one of said adjacent alpha-helices and heptad c- and g-positions in the other of said adjacent alpha-helices.

7. The single-chain Alphabody according to claim 6, wherein at least 5 of said 10 amino acid residues located at said heptad positions are identical to amino acid residues appearing at structurally equivalent positions in said second fusion-driving region.

8. The single-chain Alphabody according to any of claims 1 to 3, wherein said Alphabody is bispecific, in that, it comprises two alpha-helical binding regions, the first being defined as in claim 4 or 5 and the second being defined as in claim 6 or 7.

9. The single-chain Alphabody according to any of claims 1 to 8, wherein the class-I viral fusion protein is selected from the group consisting of influenza virus haemagglutinin (HA), simian virus 5 F1 protein, ebola virus Gp2 protein, SARS corona virus S1/S2 protein, F protein of respiratory syncytial virus, the HEF protein of influenza C virus, the F protein of Simian parainfluenza virus, the F protein of Human parainfluenza virus, the F protein of Newcastle disease virus, the F2 protein of measles, the F2 protein of Sendai virus, the TM protein of Moloney murine leukemia virus, the gp41 protein of Simian immunodeficiency virus, the gp21 protein of Human T-cell leukemia virus 1, the TM protein of Human syncytin-2, the TM protein of Visna virus, the S2 protein of Mouse hepatitis virus, the E2 protein of SARS corona virus, the E protein of Tick-borne encephalitis virus, the E2 protein of Dengue 2 and 3 virus, the E protein of Yellow Fever virus, the E protein of West Nile virus, the E1 protein of Semliki forest virus, the E1 protein of Sindbis virus, the G protein of Rabies virus, the G protein of Vesicular stomatitis virus and the gB protein of Herpes simplex virus.

10. A method for producing a single-chain Alphabody according to any of claims 1 to 9 capable of inhibiting the fusion of a class I viral fusion protein, at least comprising the step of grafting amino acid residues that are selected from a membrane fusion-driving region of said class-I viral fusion protein onto an alpha-helical region of a single-chain Alphabody.

11. A method for producing a single-chain Alphabody according to any of claims 1 to 9 capable of inhibiting the fusion of a class I viral fusion protein, at least comprising the steps of
a) selecting a fusion-driving region of a class-I viral fusion protein, said selected region being chosen from the group consisting of 'heptad repeat 2' ('HR2'), 'C-terminal heptad repeat' ('HRC' or 'HR-C') or 'C-peptide region',
b) identifying in said selected fusion-driving region the amino acid residues interacting with a complementary fusion-driving region,
c) selecting an alpha-helical region located at a solvent-oriented surface of one of the Alphabody alpha-helices, this alpha-helical region forming a structural mimic of the secondary structure of said selected fusion-driving region, and identifying in this alpha-helical region the heptad b-, c- and f-positions,
d) matching the amino acid residues identified in step b) with the heptad b-, c- and f-positions identified in step c),
e) selecting at least 5 amino acid residues identified in step b) and transferring them to heptad b-, c- and f-positions of the alpha-helical region selected in step c) in accordance with the matching operation of step d),
f) producing the Alphabody comprising the amino acid residues that are transferred in step e).

12. A method for producing a single-chain Alphabody according to any of claims 1 to 9, capable of inhibiting the fusion of a class I viral fusion protein, at least comprising the steps of
a) selecting a fusion-driving region of a class-I viral fusion protein, said selected region being chosen from the group consisting of 'heptad repeat 1' ('HR1'), 'N-terminal heptad repeat' ('HRN' or 'HR-N'), 'N-trimer region' ('N-trimer'), 'N-peptide region' or 'coiled coil region',
b) identifying in said selected fusion-driving region the amino acid residues interacting with a complementary fusion-driving region,
c) selecting an alpha-helical region located at a groove formed by two adjacent alpha-helices of the Alphabody, this alpha-helical region forming a structural mimic of the secondary structure of said selected fusion-driving region, and identifying in this alpha-helical region the heptad b- and e-positions in one of said adjacent alpha-helices and heptad c- and g-positions in the other of said adjacent alpha-helices,
d) matching the amino acid residues identified in step b) with the heptad b-, c-, e-and g-positions identified in step c),
e) selecting at least 5 amino acid residues identified in step b) and transferring them to heptad b-, c-, e- and g-positions of the alpha-helical region selected in step c) in accordance with the matching operation of step d),
f) producing the Alphabody comprising the amino acid residues that are transferred in step e).

13. A single-chain Alphabody obtainable by the method of any one of claims 10-12.

14. The single-chain Alphabody of any one of claims 1 to 9 or 13, for use in the treatment of a viral infection.

15. The single chain Alphabody of claim 14, for use in the treatment of a disease caused by a virus **characterized by** a class-I viral protein.

## Patentansprüche

1. Einzelkette-Alphabody, mit der allgemeinen Formel HRS1-L1-HRS2-L2-HRS3, wobei es sich bei HRS1, HRS2 und HRS3 jeweils unabhängig um eine HRS (Heptad Repeat Sequence), die aus 2 bis 7 aufeinander folgenden Heptad-Repeat-Einheiten besteht, handelt, wenigstens 50% aller Heptad-Positionen a und d von Isoleucin-Resten besetzt sind, die HRS jeweils mit einem entweder an einer a- oder d-Heptad-Position lokalisierten aliphatischen oder aromatischen Aminosäurerest beginnt und endet und HRS1, HRS2 und HRS3 zusammen eine tripelsträngige, alpha-helikale Coiled-Coil-Struktur bilden; und
es sich bei L1 und L2 jeweils unabhängig um ein Linker-Fragment handelt, das HRS1 mit HRS2 bzw. HRS2 mit HRS3 kovalent verbindet und aus wenigstens 4 Aminosäureresten besteht, von denen vorzugsweise wenigstens 50% aus der Gruppe Prolin, Glycin, Serin ausgewählt sind,
**dadurch gekennzeichnet, dass** der Alphabody eine alpha-helikale Bindungsregion umfasst, die die Bindung an eine erste fusionstreibende Region eines Klasse-I-Virusfusionsproteins vermittelt und die strukturell eine zweite fusionstreibende Region des Klasse-I-Virusfusionsproteins imitiert, wobei es sich bei der ersten und der zweiten fusionstreibenden Region des Klasse-I-Virus-fusionsproteins um Regionen handelt, die wechselwirken, um die Fusion zwischen einem das Klasse-I-Virusfusionsprotein präsentierenden Virus und einer Zielzelle zu treiben, wobei es sich bei dem Klasse-I-Virusfusionsprotein nicht um die gp41-Untereinheit von HIV-1-Hülle-Glykoprotein handelt.

2. Einzelkette-Alphabody nach Anspruch 1, wobei die fusionstreibenden Regionen aus der aus 'heptad repeat 1' ('HR1'), 'N-terminal heptad repeat' ('HRN' oder 'HR-N'), 'N-trimer region' ('N-trimer'), 'N-peptide region', 'coiled coil region', 'heptad repeat 2' ('HR2'), 'C-terminal heptad repeat' ('HRC' oder 'HR-C) und 'C-peptide region' bestehenden Gruppe gewählt werden.

3. Einzelkette-Alphabody nach Anspruch 1 oder 2, wobei die alpha-helikale Bindungsregion ein strukturelles Imitat der Sekundärstruktur der zweiten fusionstreibenden Region bildet.

4. Einzelkette-Alphabody nach Anspruch 3, wobei die alpha-helikale Bindungsregion an einer lösungsmittel-orientierten Oberfläche einer der Alphabody-alpha-Helices lokalisiert ist und wobei die alpha-helikale Bindungsregion wenigstens 9 an Heptad-Positionen b, c und f lokalisierte Aminosäurereste enthält.

5. Einzelkette-Alphabody nach Anspruch 4, wobei wenigstens 5 der 9 an den b-, c- und f-Heptad-Positionen lokalisierten Aminosäurereste mit Aminosäureresten identisch sind, die an strukturäquivalenten Positionen in der zweiten fusionstreibenden Region auftreten.

6. Einzelkette-Alphabody nach Anspruch 3, wobei die alpha-helikale Bindungsregion an der von zwei benachbarten Alpha-Helices des Alphabody gebildeten Furche lokalisiert ist und wobei die alpha-helikale Bindungsregion wenigstens 10 an Heptad-Positionen b und e in einer der benachbarten Alpha-Helices und Heptad-Positionen c und g in der anderen der benachbarten Alpha-Helices lokalisierte Aminosäurereste enthält.

7. Einzelkette-Alphabody nach Anspruch 6, wobei wenigstens 5 der 10 an den Heptad-Positionen lokalisierten Aminosäurereste mit Aminosäureresten identisch sind, die an strukturäquivalenten Positionen in der zweiten fusionstreibenden Region auftreten.

8. Einzelkette-Alphabody nach einem der Ansprüche 1 bis 3, wobei der Alphabody dahingehend bispezifisch ist, dass er zwei alpha-helikale Bindungsregionen umfasst, von denen die erste die in Anspruch 4 oder 5 angegebene Bedeutung und die zweite die in Anspruch 6 oder 7 angegebene Bedeutung hat.

9. Einzelkette-Alphabody nach einem der Ansprüche 1 bis 8, wobei das Klasse-I-Virusfusionsprotein aus der aus Influenzavirus-Hämagglutinin (HA), Affenvirus-5-F1-Protein, Ebola-Virus-Gp2-Protein, SARS-Coronavirus-S1/S2-Protein, F-Protein von RSV (Respiratory Syncytial Virus), dem HEF-Protein von Influenza-C-Virus, dem F-Protein von SPIV (Simian Parainfluenza Virus), dem F-Protein von HPIV (Human Parainfluenza Virus), dem F-Protein von Newcastle-Krankheit-Virus, dem Masern-F2-Protein, dem F2-Protein von Sendai-Virus, dem TM-Protein von MMLV (Moloney Murine Leukemia Virus), dem gp41-Protein von SIV (Simian Immunodeficiency Virus), dem gp21-Protein von HTLV-1 (Human T-cell Leukemia Virus 1), dem TM-Protein von Human-Syncytin-2, dem TM-Protein von Visna-Virus, dem S2-Protein von Maus-Hepatitis-Virus, dem E2-Protein von SARS-Coronavirus, dem E-Protein von FSME-Virus (Tick-borne Encephalitis Virus), dem E2-Protein von Dengue-2- und -3-Virus, dem E-Protein von Gelbfieber-Virus, dem E-Protein von West-Nil-Virus, dem E1-Protein von SFV (Semliki Forest Virus), dem E1-Protein von Sindbis-Virus, dem G-Protein des Tollwutvirus, dem G-Protein von VSV (Vesicular Stomatitis Virus) und dem gB-Protein von Herpes-simplex-Virus bestehenden Gruppe ausgewählt ist.

10. Verfahren zur Herstellung eines Einzelkette-Alphabody nach einem der Ansprüche 1 bis 9 mit der Fähigkeit zur Hemmung der Fusion eines Klasse-I-Virusfusionsproteins, wenigstens umfassend den Schritt des Pfropfens von Aminosäureresten, die aus einer membranfusionstreibenden Region des Klasse-I-Virusfusionsproteins ausgewählt sind, auf eine alpha-helikale Region eines Einzelkette-Alphabody.

11. Verfahren zur Herstellung eines Einzelkette-Alphabody nach einem der Ansprüche 1 bis 9 mit der Fähigkeit zur Hemmung der Fusion eines Klasse-I-Virusfusionsproteins, wenigstens umfassend die Schritte
a) Auswählen einer fusionstreibenden Region eines Klasse-I-Virusfusionsproteins, wobei die ausgewählte Region aus der aus 'heptad repeat 2' ('HR2'), 'C-terminal heptad repeat' ('HRC' oder 'HR-C') oder 'C-peptide region' bestehenden Gruppe gewählt wird,
b) Identifizieren der Aminosäurereste in der ausgewählten fusionstreibenden Region, die mit einer komplementären fusionstreibenden Region wechselwirken,
c) Auswählen einer an einer lösungsmittelorientierten Oberfläche einer der Alphabody-alpha-Helices lokalisierten alpha-helikalen Region, wobei diese alpha-helikale Region ein strukturelles Imitat der Sekundärstruktur der ausgewählten fusionstreibenden Region bildet, und Identifizieren der b-, c- und f-Heptad-Positionen in dieser alpha-helikalen Region,
d) Abgleichen der in Schritt b) identifizierten Aminosäurereste mit den in Schritt c) identifizierten b-, c- und f-Heptad-Positionen,
e) Auswählen von wenigstens 5 in Schritt b) identifizierten Aminosäureresten und Übertragen davon in Heptad-Positionen b, c und f der in Schritt c) ausgewählten alpha-helikalen Region gemäß dem Abgleichvorgang von Schritt d),
f) Herstellen des die Aminosäurereste, die in Schritt e) übertragen werden, umfassenden Alphabody.

12. Verfahren zur Herstellung eines Einzelkette-Alphabody nach einem der Ansprüche 1 bis 9 mit der Fähigkeit zur Hemmung der Fusion eines Klasse-I-Virusfusionsproteins, wenigstens umfassend die Schritte
a) Auswählen einer fusionstreibenden Region eines Klasse-I-Virusfusionsproteins, wobei die ausgewählte Region aus der aus 'heptad repeat 1' ('HR1'), 'N-terminal heptad repeat' ('HRN' oder 'HR-N'), 'N-trimer region' ('N-trimer'), 'N-peptide region' oder 'coiled coil region' bestehenden Gruppe gewählt wird,
b) Identifizieren der Aminosäurereste in der ausgewählten fusionstreibenden Region, die mit einer komplementären fusionstreibenden Region wechselwirken,
c) Auswählen einer an einer von zwei benachbarten Alpha-Helices des Alphabody gebildeten Furche lokalisierten alpha-helikalen Region, wobei diese alpha-helikale Region ein strukturelles Imitat der Sekundärstruktur der ausgewählten fusionstreibenden Region bildet, und Identifizieren der b- und e-Heptad-Positionen in einer der benachbarten Alpha-Helices und der c- und g-Heptad-Positionen in der anderen der benachbarten Alpha-Helices in dieser alpha-helikalen Region,
d) Abgleichen der in Schritt b) identifizierten Aminosäurereste mit den in Schritt c) identifizierten b-, c-, e- und g-Heptad-Positionen,
e) Auswählen von wenigstens 5 in Schritt b) identifizierten Aminosäureresten und Übertragen davon in Heptad-Positionen b, c, e und g der in Schritt c) ausgewählten alpha-helikalen Region gemäß dem Abgleichvorgang von Schritt d),
f) Herstellen des die Aminosäurereste, die in Schritt e) übertragen werden, umfassenden Alphabody.

13. Einzelkette-Alphabody, erhältlich mit dem Verfahren gemäß einem der Ansprüche 10-12.

14. Einzelkette-Alphabody gemäß einem der Ansprüche 1 bis 9 oder 13, zur Verwendung bei der Behandlung einer Virusinfektion.

15. Einzelkette-Alphabody gemäß Anspruch 14, zur Verwendung bei der Behandlung einer von einem durch ein Klasse-I-Virusprotein gekennzeichneten Virus verursachten Krankheit.

## Revendications

1. Alphabody monocaténaire, ayant la formule générale HRS1-L1-HRS2-L2-HRS3, dans laquelle chacun de HRS1, HRS2 et HRS3 est indépendamment une séquence de répétition d'heptade (HRS) constituée de 2 à 7 motifs de répétition d'heptade consécutifs, au moins 50 % de toutes les positions d'heptade a et d sont occupées par des résidus isoleucine, chaque HRS commence et termine par un résidu d'acide aminé aliphatique ou aromatique situé à une position d'heptade a ou d, et HRS1, HRS2 et HRS3 forment conjointement une structure de superhélice, de type hélice alpha, tricaténaire ; et
chacun de L1 et L2 est indépendamment un fragment de lieur, reliant de façon covalente HRS1 à HRS2 et HRS2 à HRS3, respectivement, et constitué d'au moins 4 résidus d'acide aminé, de préférence dont au moins 50 % sont choisis dans le groupe de la proline, la glycine, sérine,
**caractérisé en ce que** ledit Alphabody comprend une région de liaison de type hélice alpha qui médie la liaison à une première région de contrôle de fusion d'une protéine de fusion virale de classe I et qui mime structurellement une deuxième région de contrôle de fusion de ladite protéine de fusion virale de classe I, où lesdites première et deuxième régions de contrôle de fusion de ladite protéine de fusion virale de classe I sont des régions qui interagissent pour contrôler la fusion entre un virus présentant ladite protéine de fusion virale de classe I et une cellule cible, où ladite protéine de fusion virale de classe I n'est pas la sous-unité gp41 de glycoprotéine d'enveloppe de VIH-1.

2. Alphabody monocaténaire selon la revendication 1, dans lequel lesdites régions de contrôle de fusion sont choisies dans le groupe constitué des « répétition d'heptade 1 » (« HR1 »), « répétition d'heptade N-terminale » (« HRN » ou « HR-N »), « région de N-trimère » (« N-trimère »), « région de N-peptide », « région de superhélice », « répétition d'heptade 2 » (« HR2 »), « répétition d'heptade C-terminale » (« HRC » ou « HR-C ») et « région de C-peptide ».

3. Alphabody monocaténaire selon la revendication 1 ou 2, dans lequel ladite région de liaison de type hélice alpha forme un mimétique structural de la structure secondaire de ladite deuxième région de contrôle de fusion.

4. Alphabody monocaténaire selon la revendication 3, dans lequel ladite région de liaison de type hélice alpha est située au niveau d'une surface orientée solvant d'une des hélices alpha de l'Alphabody, et dans lequel ladite région de liaison de type hélice alpha comprend au moins 9 résidus d'acide aminé situés aux positions d'heptade b, c et f.

5. Alphabody monocaténaire selon la revendication 4, dans lequel au moins 5 desdits 9 résidus d'acide aminé situés auxdites positions d'heptade b, c et f sont identiques aux résidus d'acide aminé apparaissant à des positions structurellement équivalentes dans ladite deuxième région de contrôle de fusion.

6. Alphabody monocaténaire selon la revendication 3, dans lequel ladite région de liaison de type hélice alpha est située au niveau de la fente formée par les deux hélices alpha adjacentes de l'Alphabody, et dans lequel ladite région de liaison de type hélice alpha comprend au moins 10 résidus d'acide aminé situés aux positions d'heptade b et e dans l'une desdites hélices alpha adjacentes et aux positions d'heptade c et g dans l'autre desdites hélices alpha adjacentes.

7. Alphabody monocaténaire selon la revendication 6, dans lequel au moins 5 desdits 10 résidus d'acide aminé situés auxdites positions d'heptade sont identiques aux résidus d'acide aminé apparaissant à des positions structurellement équivalentes dans ladite deuxième région de contrôle de fusion.

8. Alphabody monocaténaire selon l'une quelconque des revendications 1 à 3, **caractérisé en ce que** ledit Alphabody est bispécifique, **en ce qu'**il comprend deux régions de liaison de type hélice alpha, la première étant définie comme décrit dans la revendication 4 ou 5 et la deuxième étant définie comme décrit dans la revendication 6 ou 7.

9. Alphabody monocaténaire selon l'une quelconque des revendications 1 à 8, dans lequel la protéine de fusion virale de classe I est choisie dans le groupe constitué de l'hémagglutinine du virus de la grippe (HA), la protéine F1 du virus simien 5, la protéine Gp2 du virus Ebola, la S1/S2 du SARS coronavirus, la protéine F du virus syncytial respiratoire, la protéine HEF protéine du virus de la grippe C, la protéine F du virus parainfluenza simien, la protéine F du virus parainfluenza humain, la protéine F du virus de la maladie de Newcastle, la protéine F2 de la rougeole, la protéine F2 du virus Sendai, la protéine TM du virus de la leucémie murine de Moloney, la protéine gp41 du virus d'immunodéficience simien, la protéine gp21 du virus de la leucémie à lymphocytes T humaine 1, la protéine TM de syncytine 2 humaine, la protéine TM du virus Visna, la protéine S2 du virus de l'hépatite de la souris, la protéine E2 du SARS coronavirus, la protéine E du virus de l'encéphalite à tiques, la protéine E2 du virus de la dengue 2 et 3, la protéine E du virus de la fièvre jaune, la protéine E du virus du Nil occidental, la protéine E1 du virus de la forêt de Semliki, la protéine E1 du virus Sindbis, la protéine G du virus de la rage, la protéine G du virus de la stomatite vésiculaire et la protéine gB du virus de l'herpès simplex.

10. Procédé de production d'un Alphabody monocaténaire selon l'une quelconque des revendications 1 à 9 capable d'inhiber la fusion d'une protéine de fusion virale de classe I, comprenant au moins l'étape de greffage de résidus d'acide aminé qui sont choisis parmi une région de contrôle de fusion membranaire de ladite protéine de fusion virale de classe I sur une région de type hélice alpha d'un Alphabody monocaténaire.

11. Procédé de production d'un Alphabody monocaténaire selon l'une quelconque des revendications 1 à 9 capable d'inhiber la fusion d'une protéine de fusion virale de classe I, comprenant au moins les étapes de
a) sélection d'une région de contrôle de fusion d'une protéine de fusion virale de classe I, ladite région sélectionnée étant choisie dans le groupe constitué des « répétition d'heptade 2 » (« HR2 »), « répétition d'heptade C-terminale » (« HRC » ou « HR-C ») ou « région de C-peptide »,
b) identification dans ladite région de contrôle de fusion sélectionnée des résidus d'acide aminé interagissant avec une région de contrôle de fusion complémentaire,
c) sélection d'une région de type hélice alpha située au niveau d'une surface orientée solvant d'une des hélices alpha de l'Alphabody, cette région de type hélice alpha formant un mimétique structural de la structure secondaire de ladite région de contrôle de fusion sélectionnée, et identification dans cette région de type hélice alpha des positions d'heptade b, c et f,
d) mise en correspondance des résidus d'acide aminé identifiés dans l'étape b) avec les positions d'heptade b, c et f identifiées dans l'étape c),
e) sélection d'au moins 5 résidus d'acide aminé identifiés dans l'étape b) et transfert de ceux-ci aux positions d'heptade b, c et f de la région de type hélice alpha sélectionnée dans l'étape c) conformément à l'opération de mise en correspondance de l'étape d),
f) production de l'Alphabody comprenant les résidus d'acide aminé qui sont transférés dans l'étape e).

12. Procédé de production d'un Alphabody monocaténaire selon l'une quelconque des revendications 1 à 9, capable d'inhiber la fusion d'une protéine de fusion virale de classe I, comprenant au moins les étapes de
a) sélection d'une région de contrôle de fusion d'une protéine de fusion virale de classe I, ladite région sélectionnée étant choisie dans le groupe constitué des « répétition d'heptade 1 » (« HR1 »), « répétition d'heptade N-terminale » (« HRN » ou « HR-N »), « région de N-trimère » (« N-trimère »), « région de N-peptide » ou « région de superhélice »,
b) identification dans ladite région de contrôle de fusion sélectionnée des résidus d'acide aminé interagissant avec une région de contrôle de fusion complémentaire,
c) sélection d'une région de type hélice alpha située au niveau d'une fente formée par deux hélices alpha adjacentes de l'Alphabody, cette région de type hélice alpha formant un mimétique structural de la structure secondaire de ladite région de contrôle de fusion sélectionnée, et identification dans cette région de type hélice alpha des positions d'heptade b et e dans une desdites hélices alpha adjacentes et des positions heptade c et g dans l'autre desdites hélices alpha adjacentes,
d) mise en correspondance des résidus d'acide aminé identifiés dans l'étape b) avec les positions d'heptade b, c, e et g identifiées dans l'étape c),
e) sélection d'au moins 5 résidus d'acide aminé identifiés dans l'étape b) et transfert de ceux-ci aux positions d'heptade b, c, e et g de la région de type hélice alpha sélectionnée dans l'étape c) conformément à l'opération de mise en correspondance de l'étape d),
f) production de l'Alphabody comprenant les résidus d'acide aminé qui sont transférés dans l'étape e).

13. Alphabody monocaténaire pouvant être obtenu par le procédé de l'une quelconque des revendications 10 à 12.

14. Alphabody monocaténaire de l'une quelconque des revendications 1 à 9 ou 13, pour utilisation dans le traitement d'une infection virale.

15. Alphabody monocaténaire de la revendication 14, pour utilisation dans le traitement d'une maladie causée par un virus **caractérisé par** une protéine virale de classe I.
